# EUROPEAN PATENT APPLICATION

(11) **EP 4 851 288 A1**
(43) Date of publication of application: **09.12.2026**
(21) Application number: 25747839.6
(22) Date of filing: 24.01.2025
(51) Int. Cl.: C12N 15/113, A61K 31/713, A61P 1/16, A61P 35/00

(54) **SIRNA USED FOR INHIBITING HSD17B13 EXPRESSION, CONJUGATE THEREOF, PHARMACEUTICAL COMPOSITION AND USE THEREOF**

(30) Priority: 03.02.2024 CN 202410154875; 30.09.2024 CN 202411388014
(71) Applicant: BEIJING FOYOU PHARMA CO., LTD, Beijing 101113 (CN)
(72) Inventor: WANG, Shucheng, Beijing 101113 (CN); WANG, Yan, Beijing 101113 (CN); HUANG, He, Beijing 101113 (CN); LIN, Guoliang, Beijing 101113 (CN); WANG, Xiaojun, Beijing 101113 (CN); NIU, Lijiao, Beijing 101113 (CN); CHAN, Yunxia, Beijing 101113 (CN); GENG, Yuxian, Beijing 101113 (CN)
(74) Representative: Weickmann & Weickmann PartmbB
(86) International application number: PCT/CN2025/074744
(87) International publication number: WO 2025/162271

(57) **Abstract**

Provided are a siRNA capable of inhibiting HSD17B13 gene expression, a siRNA conjugate, a prodrug, a pharmaceutical composition comprising same, and the use thereof. Each nucleotide in the siRNA is independently a modified or unmodified nucleotide, and the siRNA contains a sense strand and an antisense strand. The siRNA, the conjugate thereof and the pharmaceutical composition can effectively treat and/or prevent diseases related to the overexpression of the HSD17B13 gene.

## Description

### TECHNICAL FIELD

The present disclosure relates to a siRNA capable of inhibiting HSD17B13 gene expression, a siRNA conjugate, a prodrug, a pharmaceutical composition comprising the same, a preparation method therefor, and use thereof.

### BACKGROUND

The 17β-hydroxysteroid dehydrogenase (17β-HSD) family consists of 15 enzymes. Most of them are involved in the activation or inactivation of sex hormones (e.g., HSD17B1, HSD17B2, HSD17B3, HSD17B5, and HSD17B6), while other members participate in fatty acid metabolism, cholesterol biosynthesis, bile acid production, and the like. Members of the HSD17B family differ in tissue distribution, subcellular localization, and catalytic preference, and possess different substrate specificities (Marchais Oberwinkler, et al. (2011) J Steroid Biochem Mol Biol 125(1-2):66-82).

HSD17B13, a member of the 17β-hydroxysteroid dehydrogenase family, is primarily localized in hepatocytes. It is known to exhibit the highest expression level in the hepatocytes of the liver, while only lower levels can be detected in the ovary, bone marrow, kidney, brain, lung, skeletal muscle, bladder, and testis. It is a hepatocyte-specific lipid droplet (LD)-associated protein, and increasing evidences suggest that it plays a key role in liver lipid metabolism. The function of HSD17B13 is not yet fully understood; however, some 17β-HSD family members, including 17β-HSD-4, 17β-HSD-7, 17β-HSD-10, and 17β-HSD-12, have been shown to be involved in carbohydrate and fatty acid metabolism. This suggests that HSD17B13 may also play a role in lipid metabolism pathways. It has been reported that hepatic upregulation of HSD17B13 has been observed in patients with fatty liver, which supports the role of this enzyme in the pathogenesis of non-alcoholic fatty liver disease (NAFLD).

Non-alcoholic fatty liver disease (NAFLD), also known as metabolic (dysfunction)-associated fatty liver disease (MAFLD), refers to excessive fat accumulation in the liver in the absence of other clear causes (such as alcohol consumption). NAFLD is the most common liver disease in the world, affecting about 25% of the world's population. The prevalence rate of NAFLD still shows an upward trend, which will undoubtedly lead to an increase in economic burden and a sharp increase in the number of patients with end-stage liver disease requiring liver transplantation, as well as the number of individuals suffering from hepatocellular carcinoma. There are currently no specific treatments for NAFLD; it is primarily managed through dietary changes and exercise for weight loss, with preliminary studies suggesting the potential of pioglitazone and vitamin E for treatment.

Wen Su et al. previously identified HSD17B13 as a lipid droplet (LD)-associated protein in NAFLD patients and reported that HSD17B13 is one of the most abundantly expressed LD proteins specifically localized on the surface of LDs (Wen Su et al., Comparative proteomic study reveals 17β-HSD13 as a pathogenic protein in nonalcoholic fatty liver disease, 111 PNAS 11437-11442 (2014)). Further, the levels of HSD17B13 were found to be upregulated in the livers of patients and mice with NAFLD. Overexpression leads to an increase in the number and size of LDs, while gene silencing of HSD17B13 attenuates oleic acid-induced LD formation in cultured hepatocytes. Hepatic overexpression of the HSD17B13 protein in C57BL/6 mice has also been shown to significantly increase lipogenesis and triglyceride (TG) content in the liver, leading to a fatty liver phenotype. N. S. Abul-Husn et al. provided additional evidence implying HSD17B13 gene expression in the pathogenesis of NAFLD and non-alcoholic steatohepatitis (NASH) (N. S. Abul-Husn et al., A Protein-Truncating HSD17B13 Variant and Protection from Chronic Liver Disease, 378 N. Eng. J. Med. 1096-1106 (2018)). The team conducted a genome-wide association study, which revealed that an HSD17B13 splice variant (rs72613567:TA) associated with reduced levels of alanine aminotransferase (ALT) and aspartate aminotransferase (AST) indicated less liver damage and inflammation in fatty liver patients. The splice variant results in a truncated deletion of the functional protein, suggesting that HSD17B13 normally generates products leading to hepatocyte damage. ARO-HSD, an RNAi therapy targeting HSD17B13 jointly developed by GlaxoSmithKline and Arrowhead for the treatment of non-alcoholic steatohepatitis, has entered Phase I clinical studies. Currently, there are no marketed nucleic acid drugs targeting HSD17B13.

The present disclosure aims to provide a siRNA, a siRNA conjugate, a prodrug, and a pharmaceutical composition thereof, which can affect the RNA-induced silencing complex (RISC)-mediated cleavage of RNA transcripts of the HSD17B13 gene, thereby inhibiting the expression of the HSD17B13 gene in the liver and achieving the purpose of disease treatment.

### SUMMARY OF THE INVENTION

The present disclosure provides a siRNA capable of inhibiting HSD17B13 gene expression, and the siRNA comprises a sense strand and an antisense strand, wherein each nucleotide in the siRNA is independently a modified or unmodified nucleotide; the sense strand comprises a nucleotide sequence I, the antisense strand comprisesa nucleotide sequence II, and the nucleotide sequence I is at least partially reversely complementary to the nucleotide sequence II to form a double-stranded region, wherein the nucleotide sequence I and the nucleotide sequence II are selected from the following sequences:
(1) the nucleotide sequence I comprising the nucleotide sequence as shown in SEQ ID NO: 25, and the nucleotide sequence II comprising the nucleotide sequence as shown in SEQ ID NO: 26;
(2) the nucleotide sequence I comprising the nucleotide sequence as shown in SEQ ID NO: 40, and the nucleotide sequence II comprising the nucleotide sequence as shown in SEQ ID NO: 41;
(3) the nucleotide sequence I comprising the nucleotide sequence as shown in SEQ ID NO: 53, and the nucleotide sequence II comprising the nucleotide sequence as shown in SEQ ID NO: 54;
(4) the nucleotide sequence I comprising the nucleotide sequence as shown in SEQ ID NO: 140, and the nucleotide sequence II comprising the nucleotide sequence as shown in SEQ ID NO: 141:
   5'-GUAUGCAGAAUAUU-3' (SEQ ID NO: 140)
   5'-AAUAUUCUGCAUAC-3' (SEQ ID NO: 141);
(5) the nucleotide sequence I comprising the nucleotide sequence as shown in SEQ ID NO: 142, and the nucleotide sequence II comprising the nucleotide sequence as shown in SEQ ID NO: 143:
   5'-GAAUAUUCAAUUUGAA-3' (SEQ ID NO: 142)
   5'-UUCAAAUUGAAUAUUC-3' (SEQ ID NO: 143);
(6) the nucleotide sequence I comprising the nucleotide sequence as shown in SEQ ID NO: 1, and the nucleotide sequence II comprising the nucleotide sequence as shown in SEQ ID NO: 2; and
(7) the nucleotide sequence I comprising the nucleotide sequence as shown in SEQ ID NO: 27, and the nucleotide sequence II comprising the nucleotide sequence as shown in SEQ ID NO: 28.

In one embodiment, the nucleotide sequence I is substantially reversely complementary, virtually reversely complementary, or completely reversely complementary to the nucleotide sequence II; the substantially reversely complementary means that there are no more than 3 base mismatches between the two nucleotide sequences; the virtually reversely complementary means that there is no more than 1 base mismatch between the two nucleotide sequences; the completely reversely complementary means that there is no mismatch between the two nucleotide sequences.

In one embodiment, the sense strand further comprises a nucleotide sequence III, and the antisense strand further comprises a nucleotide sequence IV, wherein the nucleotide sequence III and the nucleotide sequence IV each independently have a length of 0-7 nucleotides; the nucleotide sequence III is linked to the 5' end of the nucleotide sequence I, the nucleotide sequence IV is linked to the 3' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences; and/or
the nucleotide sequence III is linked to the 3' end of the nucleotide sequence I, the nucleotide sequence IV is linked to the 5' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences.

In one embodiment, the siRNA comprises a sense strand and an antisense strand, wherein each nucleotide in the siRNA is independently a modified or unmodified nucleotide; the sense strand comprises nucleotide sequences I and III, the antisense strand comprises nucleotide sequences II and IV, and the nucleotide sequences I and III are at least partially reversely complementary to the nucleotide sequences II and IV to form a double-stranded region, wherein the nucleotide sequences I and III and the nucleotide sequences II and IV are selected from the following sequences:
(1) the nucleotide sequences I and III comprising or consisting of the nucleotide sequence as shown in SEQ ID NO: 112, and the nucleotide sequences II and IV comprising or consisting of the nucleotide sequence as shown in SEQ ID NO: 113; and
(2) the nucleotide sequences I and III comprising or consisting of the nucleotide sequence as shown in SEQ ID NO: 208, and the nucleotide sequences II and IV comprising or consisting of the nucleotide sequence as shown in SEQ ID NO: 209.

In one embodiment, the sense strand further comprises a nucleotide sequence V, and/or the antisense strand further comprises a nucleotide sequence VI, wherein the nucleotide sequences V and VI have a length of 0 to 3 nucleotides; the nucleotide sequence V is linked to the 3' end of the sense strand to form the 3' overhang of the sense strand, and/or the nucleotide sequence VI is linked to the 3' end of the antisense strand to form the 3' overhang of the antisense strand. In one preferred embodiment, the nucleotide sequence V or VI has a length of 2 nucleotides. In one preferred embodiment, the nucleotide sequence V or VI is two consecutive thymine deoxyribonucleotides or two consecutive uracil ribonucleotides. In one preferred embodiment, the nucleotide sequence V is identical to or different from a nucleotide at a corresponding position of the target mRNA, or the nucleotide sequence VI is mismatched or complementary to a nucleotide at a corresponding position of the target mRNA.

In one embodiment, the double-stranded region has a length of 15-30 nucleotide pairs; preferably, the double-stranded region has a length of 17-23 nucleotide pairs; more preferably, the double-stranded region has a length of 19-21 nucleotide pairs.

In another embodiment, the sense strand or the antisense strand has 15-30 nucleotides; preferably, the sense strand or the antisense strand has 19-25 nucleotides; more preferably, the sense strand or the antisense strand has 19-23 nucleotides.

In one embodiment, at least one nucleotide in the sense strand or the antisense strand is a modified nucleotide, and/or at least one phosphoester group is a phosphoester group with a modification group; preferably, the phosphoester group with a modification group is a phosphorothioate group formed by substituting one oxygen atom in a phosphodiester bond of the phosphoester group with a sulfur atom.

In one embodiment, the siRNA comprises a sense strand without a 3' overhang nucleotide.

In one embodiment, the 5' end nucleotide of the antisense strand is linked to a 5' phosphate group or a 5' phosphate-derived group, or the 5' end nucleotide of the antisense strand is not linked to a 5' phosphate group or a 5' phosphate-derived group.

In one embodiment, the 3' end nucleotide of the sense strand is linked to an inverted abasic deoxyribose residue, and/or the 5' end nucleotide of the sense strand is linked to an inverted abasic deoxyribose residue.

In one embodiment, the inverted abasic deoxyribose residue is linked to the 3' end nucleotide and/or 5' end nucleotide of the sense strand via a phosphodiester bond, a phosphorothioate group, or other internucleoside bonds.

In one embodiment, the modified nucleotide is selected from a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxyribonucleotide, a 2'-amino-modified nucleotide, a 2'-substituted amino-modified nucleotide, a nucleotide analog, and a combination of any two or more thereof.

In one embodiment, the modified nucleotide is selected from a 2'-fluoro-modified nucleotide, a 2'-methoxy-modified nucleotide, a 2'-O-CH₂-CH₂-O-CH₃-modified nucleotide, a 2'-O-CH₂-CH=CH₂-modified nucleotide, a 2'-CH₂-CH₂-CH=CH₂-modified nucleotide, a 2'-deoxyribonucleotide, a nucleotide analog, and a combination of any two or more thereof.

In one embodiment, each nucleotide in the sense strand and the antisense strand is independently a 2'-fluoro-modified nucleotide or a non-fluoro-modified nucleotide.

In one preferred embodiment, in the sense strand, in the 5' to 3' direction, the 2'-fluoro-modified nucleotides are located at positions 7, 9, 10, and 11, and the remaining positions are the non-fluoro-modified nucleotides; or, the 2'-fluoro-modified nucleotides are located at positions 9, 10, and 11, and the remaining positions are the non-fluoro-modified nucleotides; or, the 2'-fluoro-modified nucleotides are located at positions 8, 9, and 10, and the remaining positions are the non-fluoro-modified nucleotides; or, the 2'-fluoro-modified nucleotides are located at positions 8, 9, 10, and 11, and the remaining positions are the non-fluoro-modified nucleotides; or, the 2'-fluoro-modified nucleotides are located at positions 7 and 9, and the remaining positions are the non-fluoro-modified nucleotides; or, the 2'-fluoro-modified nucleotides are located at positions 7, 9, and 11, and the remaining positions are the non-fluoro-modified nucleotides; or, the 2'-fluoro-modified nucleotides are located at positions 7, 9, 11, 13, and 15, and the remaining positions are the non-fluoro-modified nucleotides; or, the 2'-fluoro-modified nucleotides are located at positions 7, 9, 11, and 13, and the remaining positions are the non-fluoro-modified nucleotides; or, the 2'-fluoro-modified nucleotides are located at positions 3, 7, 8, and 9, and the remaining positions are the non-fluoro-modified nucleotides; or, the 2'-fluoro-modified nucleotides are located at positions 3, 9, 11, and 13, and the remaining positions are the non-fluoro-modified nucleotides; or, the 2'-fluoro-modified nucleotides are located at positions 3, 7, 9, and 11, and the remaining positions are the non-fluoro-modified nucleotides; or, the 2'-fluoro-modified nucleotides are located at positions 7, 11, and 13, and the remaining positions are the non-fluoro-modified nucleotides; or, the 2'-fluoro-modified nucleotides are located at positions 7, 11, 12, and 13, and the remaining positions are the non-fluoro-modified nucleotides; or, the 2'-fluoro-modified nucleotides are located at positions 3, 7, 11, and 16, and the remaining positions are the non-fluoro-modified nucleotides; or, the 2'-fluoro-modified nucleotides are located at positions 3, 7, 11, 16, and 17, and the remaining positions are the non-fluoro-modified nucleotides; or, the 2'-fluoro-modified nucleotides are located at positions 3, 7, and 9, and the remaining positions are the non-fluoro-modified nucleotides; or, the 2'-fluoro-modified nucleotides are located at positions 7, 9, and 14, and the remaining positions are the non-fluoro-modified nucleotides; or, the 2'-fluoro-modified nucleotides are located at positions 5, 7, 8, and 9, and the remaining positions are the non-fluoro-modified nucleotides; and/or
in the antisense strand, in the 5' to 3' direction, the 2'-fluoro-modified nucleotides are located at positions 2, 6, 14, and 16, and the remaining positions are the non-fluoro-modified nucleotides; or, the 2'-fluoro-modified nucleotides are located at positions 2, 6, 8, 9, 14, and 16, and the remaining positions are the non-fluoro-modified nucleotides; or, the 2'-fluoro-modified nucleotides are located at positions 2, 14, and 16, and the remaining positions are the non-fluoro-modified nucleotides; or, the 2'-fluoro-modified nucleotides are located at positions 2, 6, and 14, and the remaining positions are the non-fluoro-modified nucleotides; or, the 2'-fluoro-modified nucleotides are located at positions 2, 6, 8, 9, and 14, and the remaining positions are the non-fluoro-modified nucleotides; or, the 2'-fluoro-modified nucleotides are located at positions 2, 3, 5, 8, 10, 14, 16, and 18, and the remaining positions are the non-fluoro-modified nucleotides; or, the 2'-fluoro-modified nucleotides are located at positions 2, 3, 4, 5, 7, 10, and 14, and the remaining positions are the non-fluoro-modified nucleotides; or, the 2'-fluoro-modified nucleotides are located at positions 2, 4, 6, 12, 14, 16, and 18, and the remaining positions are the non-fluoro-modified nucleotides; or, the 2'-fluoro-modified nucleotides are located at positions 2 and 14, and the remaining positions are the non-fluoro-modified nucleotides; or, the 2'-fluoro-modified nucleotides are located at positions 2, 4, 5, 6, 8, 10, 12, 14, 16, and 18, and the remaining positions are the non-fluoro-modified nucleotides; or, the 2'-fluoro-modified nucleotides are located at positions 2, 7, 10, and 14, and the remaining positions are the non-fluoro-modified nucleotides; or, the 2'-fluoro-modified nucleotides are located at positions 2, 5, 7, and 14, and the remaining positions are the non-fluoro-modified nucleotides; or, the 2'-fluoro-modified nucleotides are located at positions 2, 7, 12, and 14, and the remaining positions are the non-fluoro-modified nucleotides; or, the 2'-fluoro-modified nucleotides are located at positions 2, 6, 12, and 14, and the remaining positions are the non-fluoro-modified nucleotides; or, the 2'-fluoro-modified nucleotides are located at positions 2, 3, 6, and 14, and the remaining positions are the non-fluoro-modified nucleotides; or, the 2'-fluoro-modified nucleotides are located at positions 2, 6, 8, and 14, and the remaining positions are the non-fluoro-modified nucleotides; or, the 2'-fluoro-modified nucleotides are located at positions 2, 10, and 14, and the remaining positions are the non-fluoro-modified nucleotides; or, the 2'-fluoro-modified nucleotides are located at positions 2, 7, and 14, and the remaining positions are the non-fluoro-modified nucleotides; or, the 2'-fluoro-modified nucleotides are located at positions 2, 3, 5, 7, 10, 12, and 14, and the remaining positions are the non-fluoro-modified nucleotides.

In one embodiment, each of the non-fluoro-modified nucleotides is independently selected from one of a nucleotide formed by substituting hydroxy at the 2' position of a ribosyl group of the nucleotide with a non-fluorine group and a nucleotide analog, and the nucleotide analog is selected from one of pseudouracil, isonucleotide, LNA, ENA, cET BNA, UNA, and GNA.

In one embodiment, each nucleotide in the sense strand and the antisense strand is independently a 2'-fluoro-modified nucleotide, a 2'-methoxy-modified nucleotide, a 2'-deoxyribonucleotide, a GNA-modified nucleotide, or a combination of any two or more thereof.

In one preferred embodiment, in the sense strand, in the 5' to 3' direction, the 2'-fluoro-modified nucleotides are located at positions 7, 9, 10, and 11, and the remaining positions are the 2'-methoxy-modified nucleotides; or, the 2'-fluoro-modified nucleotides are located at positions 9, 10, and 11, and the remaining positions are the 2'-methoxy-modified nucleotides; or, the 2'-fluoro-modified nucleotides are located at positions 8, 9, and 10, and the remaining positions are the 2'-methoxy-modified nucleotides; or, the 2'-fluoro-modified nucleotides are located at positions 8, 9, 10, and 11, and the remaining positions are the 2'-methoxy-modified nucleotides; or, the 2'-fluoro-modified nucleotides are located at positions 7 and 9, the 2'-deoxyribonucleotide is located at position 11 of the sense strand, and the remaining positions are the 2'-methoxy-modified nucleotides; or, the 2'-fluoro-modified nucleotides are located at positions 7, 9, and 11, and the remaining positions are the 2'-methoxy-modified nucleotides; or, the 2'-fluoro-modified nucleotides are located at positions 7, 9, 11, 13, and 15, and the remaining positions are the 2'-methoxy-modified nucleotides; or, the 2'-fluoro-modified nucleotides are located at positions 7, 9, 11, and 13, and the remaining positions are the 2'-methoxy-modified nucleotides; or, the 2'-fluoro-modified nucleotides are located at positions 3, 7, 8, and 9, and the remaining positions are the 2'-methoxy-modified nucleotides; or, the 2'-fluoro-modified nucleotides are located at positions 3, 9, 11, and 13, and the remaining positions are the 2'-methoxy-modified nucleotides; or, the 2'-fluoro-modified nucleotides are located at positions 3, 7, 9, and 11, and the remaining positions are the 2'-methoxy-modified nucleotides; or, the 2'-fluoro-modified nucleotides are located at positions 7, 11, and 13, and the remaining positions are the 2'-methoxy-modified nucleotides; or, the 2'-fluoro-modified nucleotides are located at positions 7, 11, 12, and 13, and the remaining positions are the 2'-methoxy-modified nucleotides; or, the 2'-fluoro-modified nucleotides are located at positions 3, 7, 11, and 16, and the remaining positions are the 2'-methoxy-modified nucleotides; or, the 2'-fluoro-modified nucleotides are located at positions 3, 7, 11, 16, and 17, and the remaining positions are the 2'-methoxy-modified nucleotides; or, the 2'-fluoro-modified nucleotides are located at positions 3, 7, and 9, and the remaining positions are the 2'-methoxy-modified nucleotides; or, the 2'-fluoro-modified nucleotides are located at positions 7, 9, and 14, and the remaining positions are the 2'-methoxy-modified nucleotides; or, the 2'-fluoro-modified nucleotides are located at positions 5, 7, 8, and 9, and the remaining positions are the 2'-methoxy-modified nucleotides; and/or
in the antisense strand, in the 5' to 3' direction, the 2'-fluoro-modified nucleotides are located at positions 2, 6, 14, and 16, and the remaining positions are the 2'-methoxy-modified nucleotides; or, the 2'-fluoro-modified nucleotides are located at positions 2, 6, 8, 9, 14, and 16, and the remaining positions are the 2'-methoxy-modified nucleotides; or, the 2'-fluoro-modified nucleotides are located at positions 2, 14, and 16, the GNA-modified nucleotide is located at position 6 of the antisense strand, and the remaining positions are the 2'-methoxy-modified nucleotides; or, the 2'-fluoro-modified nucleotides are located at positions 2, 6, 14, and 16, the GNA-modified nucleotide is located at position 7 of the antisense strand, and the remaining positions are the 2'-methoxy-modified nucleotides; or, the 2'-fluoro-modified nucleotides are located at positions 2, 6, and 14, and the remaining positions are the 2'-methoxy-modified nucleotides; or, the 2'-fluoro-modified nucleotides are located at positions 2, 6, 8, 9, and 14, and the remaining positions are the 2'-methoxy-modified nucleotides; or, the 2'-fluoro-modified nucleotides are located at positions 2, 3, 5, 8, 10, 14, 16, and 18, and the remaining positions are the 2'-methoxy-modified nucleotides; or, the 2'-fluoro-modified nucleotides are located at positions 2, 3, 4, 5, 7, 10, and 14, and the remaining positions are the 2'-methoxy-modified nucleotides; or, the 2'-fluoro-modified nucleotides are located at positions 2, 4, 6, 12, 14, 16, and 18, and the remaining positions are the 2'-methoxy-modified nucleotides; or, the 2'-fluoro-modified nucleotides are located at positions 2 and 14, and the remaining positions are the 2'-methoxy-modified nucleotides; or, the 2'-fluoro-modified nucleotides are located at positions 2, 4, 5, 6, 8, 10, 12, 14, 16, and 18, and the remaining positions are the 2'-methoxy-modified nucleotides; or, the 2'-fluoro-modified nucleotides are located at positions 2, 3, 4, 5, 7, 10, and 14, the GNA-modified nucleotide is located at position 6 of the antisense strand, and the remaining positions are the 2'-methoxy-modified nucleotides; or, the 2'-fluoro-modified nucleotides are located at positions 2, 7, 10, and 14, and the remaining positions are the 2'-methoxy-modified nucleotides; or, the 2'-fluoro-modified nucleotides are located at positions 2, 5, 7, and 14, and the remaining positions are the 2'-methoxy-modified nucleotides; or, the 2'-fluoro-modified nucleotides are located at positions 2, 7, 12, and 14, and the remaining positions are the 2'-methoxy-modified nucleotides; or, the 2'-fluoro-modified nucleotides are located at positions 2, 6, 12, and 14, and the remaining positions are the 2'-methoxy-modified nucleotides; or, the 2'-fluoro-modified nucleotides are located at positions 2, 3, 6, and 14, and the remaining positions are the 2'-methoxy-modified nucleotides; or, the 2'-fluoro-modified nucleotides are located at positions 2, 6, 8, and 14, and the remaining positions are the 2'-methoxy-modified nucleotides; or, the 2'-fluoro-modified nucleotides are located at positions 2, 10, and 14, the nucleotide at position 7 is replaced with a deoxyribonucleotide, the 2'-deoxyribonucleotide is located at position 5 of the antisense strand, and the remaining positions are the 2'-methoxy-modified nucleotides; or, the 2'-fluoro-modified nucleotides are located at positions 2, 7, and 14, the nucleotide at position 12 is replaced with a deoxyribonucleotide, the 2'-deoxyribonucleotide is located at position 5 of the antisense strand, and the remaining positions are the 2'-methoxy-modified nucleotides; or, the 2'-fluoro-modified nucleotides are located at positions 2 and 14, the nucleotides at positions 7 and 12 are replaced with deoxyribonucleotides, and the remaining positions are the 2'-methoxy-modified nucleotides; or, the 2'-fluoro-modified nucleotides are located at positions 2, 3, 5, 7, 10, 12, and 14, the GNA-modified nucleotide is located at position 6 of the antisense strand, and the remaining positions are the 2'-methoxy-modified nucleotides.

In some embodiments, in the direction from the 5' end to the 3' end of the siRNA,
(1) the sense strand comprises phosphorothioate groups located at the positions shown below:
   a position between the first nucleotide and the second nucleotide from the 5' end of the sense strand; and
   a position between the second nucleotide and the third nucleotide from the 5' end of the sense strand; and
   a position between the first nucleotide and the second nucleotide from the 3' end of the sense strand; and
   a position between the second nucleotide and the third nucleotide from the 3' end of the sense strand;
   or,
(2) the sense strand comprises phosphorothioate groups located at the positions shown below:
   a position between the first nucleotide and the second nucleotide from the 5' end of the sense strand; and
   a position between the second nucleotide and the third nucleotide from the 5' end of the sense strand;
   or,
(3) the sense strand comprises phosphorothioate groups located at the positions shown below:
   a position between the first nucleotide and the second nucleotide from the 5' end of the sense strand;
   or,
(4) the sense strand comprises phosphorothioate groups located at the positions shown below:
   a position between the first nucleotide and the second nucleotide from the 5' end of the sense strand; and
   a position between the second nucleotide and the third nucleotide from the 5' end of the sense strand; and
   a position between the inverted abasic deoxyribose residue and the first nucleotide from the 3' end of the sense strand;
   or,
(5) the sense strand comprises phosphorothioate groups located at the positions shown below:
   a position between the inverted abasic deoxyribose residue and the first nucleotide from the 5' end of the sense strand; and
   a position between the first nucleotide and the second nucleotide from the 5' end of the sense strand; and
   a position between the inverted abasic deoxyribose residue and the first nucleotide from the 3' end of the sense strand.

In some embodiments, in the direction from the 5' end to the 3' end of the siRNA, the antisense strand comprises phosphorothioate groups located at the positions shown below:
a position between the first nucleotide and the second nucleotide from the 5' end of the antisense strand; and
a position between the second nucleotide and the third nucleotide from the 5' end of the antisense strand; and
a position between the first nucleotide and the second nucleotide from the 3' end of the antisense strand; and
a position between the second nucleotide and the third nucleotide from the 3' end of the antisense strand.

In one embodiment, each nucleotide in the sense strand and the antisense strand is independently a 2'-fluoro-modified nucleotide, a 2'-methoxy-modified nucleotide, a 2'-deoxyribonucleotide, a GNA-modified nucleotide, or a combination of any two or more thereof.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, and the remaining positions are the 2'-methoxy-modified nucleotides; the 5' end nucleotide of the antisense strand is linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 14, and 16 of the antisense strand, the GNA-modified nucleotide is located at position 6 of the antisense strand, and the remaining positions are the 2'-methoxy-modified nucleotides; the 5' end nucleotide of the antisense strand is linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the GNA-modified nucleotide is located at position 7 of the antisense strand, and the remaining positions are the 2'-methoxy-modified nucleotides; the 5' end nucleotide of the antisense strand is linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, and the remaining positions are the 2'-methoxy-modified nucleotides; the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, and 14 of the antisense strand, and the remaining positions are the 2'-methoxy-modified nucleotides; the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, and 14 of the antisense strand, and the remaining positions are the 2'-methoxy-modified nucleotides; the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 8, 9, and 10 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 5, 8, 10, 14, 16, and 18 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 8, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 4, 6, 12, 14, 16, and 18 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2 and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7 and 9 of the sense strand, the 2'-deoxyribonucleotide is located at position 11 of the sense strand, and the remaining positions are the 2'-methoxy-modified nucleotides; in the 5' to 3' direction, the 2'-fluoro-modified nucleotides are located at positions 2, 4, 5, 6, 8, 10, 12, 14, 16, and 18 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 11, 13, and 15 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 8, and 9 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 11, 12, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 11, and 16 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 11, 16, and 17 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, position 6 is the GNA-modified nucleotide, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 5, 7, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 8, and 9 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 5, 7, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, and 9 of the sense strand, and the remaining positions are the 2'-methoxy-modified nucleotides; in the 5' to 3' direction, the 2'-fluoro-modified nucleotides are located at positions 2, 5, 7, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, and 9 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 7, 12, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 6, 12, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 3, 6, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 6, 8, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 10, and 14 of the antisense strand, the nucleotide at position 7 is replaced with a thymine nucleotide, the 2'-deoxyribonucleotide is located at position 5 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 7, and 14 of the antisense strand, the nucleotide at position 12 is replaced with a thymine nucleotide, the 2'-deoxyribonucleotide is located at position 5 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2 and 14 of the antisense strand, the nucleotides at positions 7 and 12 are replaced with thymine nucleotides, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, and 14 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 8, and 9 of the sense strand, and the remaining positions are the 2'-methoxy-modified nucleotides; in the 5' to 3' direction, the 2'-fluoro-modified nucleotides are located at positions 2, 3, 5, 7, 10, 12, and 14 of the antisense strand, position 6 is the GNA-modified nucleotide, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 3, 5, 7, 10, 12, and 14 of the antisense strand, position 6 is the GNA-modified nucleotide, and the remaining positions are the 2'-methoxy-modified nucleotides; the 5' end nucleotide of the antisense strand is linked to a 5'-(E)-vinylphosphonate group.

In one preferred embodiment, in the 5' to 3' direction, the 2'-fluoro-modified nucleotides are located at positions 5, 7, 8, and 9 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides.

In one specific embodiment, the present disclosure provides a siRNA selected from Table 1; preferably, the siRNA is selected from N-ER-FY007180, N-ER-FY007180M8, N-ER-FY007180M44, N-ER-FY007180M56, N-ER-FY007180M58, N-ER-FY007180M62, N-ER-FY007180M2, N-ER-FY007180M3, N-ER-FY007180M4, N-ER-FY007180M5, N-ER-FY007180M6, N-ER-FY007180M7, N-ER-FY007180M9, N-ER-FY007180M10, N-ER-FY007180M11, N-ER-FY007180M12, N-ER-FY007180M13, N-ER-FY007180M14, N-ER-FY007180M15, N-ER-FY007180M16, N-ER-FY007180M17, N-ER-FY007180M18, N-ER-FY007180M19, N-ER-FY007180M21, N-ER-FY007180M24, N-ER-FY007180M26, N-ER-FY007180M27, N-ER-FY007180M28, N-ER-FY007180M29, N-ER-FY007180M30, N-ER-FY007180M31, N-ER-FY007180M32, N-ER-FY007180M34, N-ER-FY007180M35, N-ER-FY007180M37, N-ER-FY007180M40, N-ER-FY007180M45, N-ER-FY007180M46, N-ER-FY007180M47, N-ER-FY007180M48, N-ER-FY007180M49, N-ER-FY007180M50, N-ER-FY007180M51, N-ER-FY007180M52, N-ER-FY007180M53, N-ER-FY007180M54, N-ER-FY007180M55, N-ER-FY007180M57, N-ER-FY007180M59, N-ER-FY007180M60, N-ER-FY007180M61, N-ER-FY007180M63, and N-ER-FY007180M64.

The present disclosure also provides a siRNA conjugate or a prodrug thereof, and the siRNA conjugate comprises the siRNA of the present disclosure and a conjugated group conjugated to the siRNA (as shown in the following formula, the double helix structure represents the siRNA, and the conjugated group is linked to the 3' end of the sense strand of the siRNA): 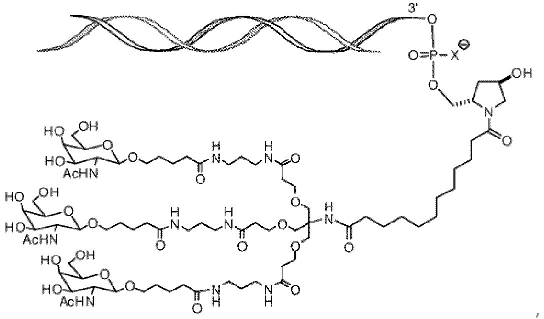 in the conjugate structure described above, X may optionally be O or S; in one embodiment, X is O.

In one embodiment, the conjugated group comprises a pharmaceutically acceptable targeting group and a linker, and the siRNA, the linker, and the targeting group are sequentially linked covalently or non-covalently.

Preferably, in the siRNA conjugate, the sense strand and the antisense strand of the siRNA are complementary to form a double-stranded region of the siRNA conjugate, a blunt end is formed at the 3' end of the sense strand, and the 3' end of the antisense strand has 1-3 protruding nucleotides extending out of the double-stranded region;
or,
in the siRNA conjugate, the sense strand and the antisense strand of the siRNA are complementary to form a double-stranded region of the siRNA conjugate, a blunt end is formed at the 3' end of the sense strand, and a blunt end is formed at the 3' end of the antisense strand.

In one embodiment, the conjugated group is selected from:

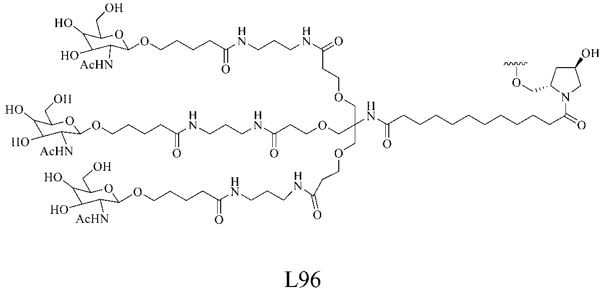

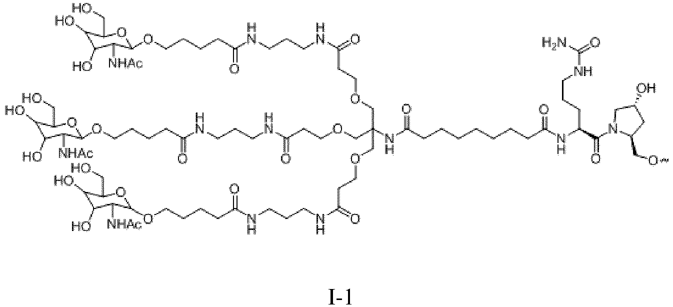

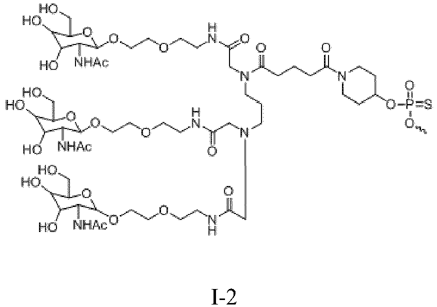

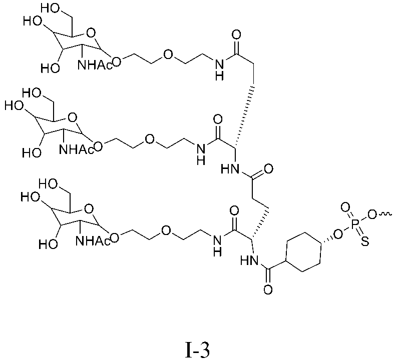

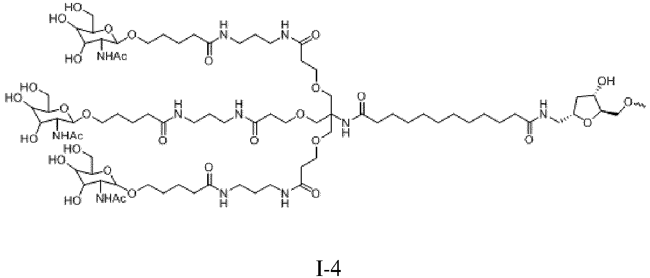

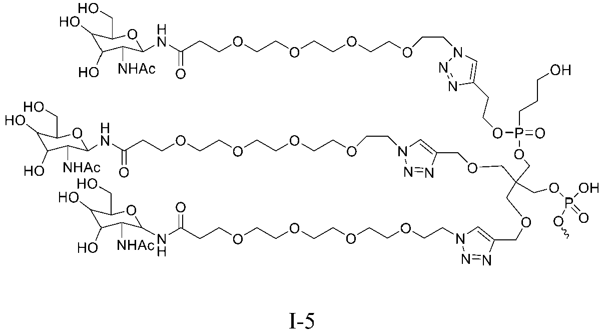

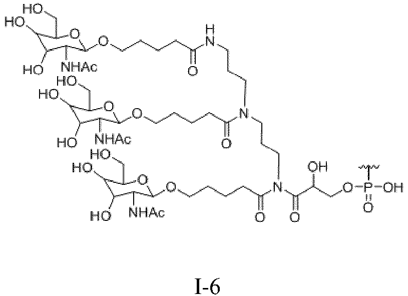

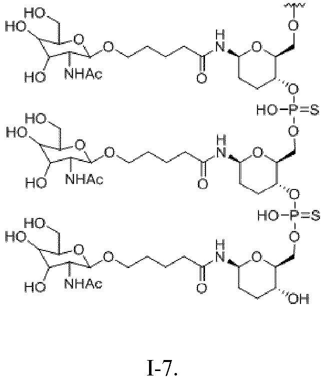

In one specific embodiment, the siRNA conjugate is a siRNA conjugate selected from Table 2; preferably, the siRNA conjugate is selected from N-ER-FY007180M8L96, N-ER-FY007180M44L96, N-ER-FY007180M56L96, N-ER-FY007180M58L96, N-ER-FY007180M62L96, N-ER-FY007180M2L96, N-ER-FY007180M3L96, N-ER-FY007180M6L96, N-ER-FY007180M7L96, N-ER-FY007180M9L96, N-ER-FY007180M10L96, N-ER-FY007180M11L96, N-ER-FY007180M13L96, N-ER-FY007180M15L96, N-ER-FY007180M21L96, N-ER-FY007180M24L96, N-ER-FY007180M26L96, N-ER-FY007180M27L96, N-ER-FY007180M29L96, N-ER-FY007180M31L96, N-ER-FY007180M32L96, N-ER-FY007180M34L96, N-ER-FY007180M35L96, N-ER-FY007180M37L96, N-ER-FY007180M40L96, N-ER-FY007180M45L96, N-ER-FY007180M46L96, N-ER-FY007180M47L96, N-ER-FY007180M48L96, N-ER-FY007180M49L96, N-ER-FY007180M50L96, N-ER-FY007180M51L96, N-ER-FY007180M52L96, N-ER-FY007180M53L96, N-ER-FY007180M54L96, N-ER-FY007180M55L96, N-ER-FY007180M57L96, N-ER-FY007180M59L96, N-ER-FY007180M60L96, N-ER-FY007180M61L96, N-ER-FY007180M63L96, and N-ER-FY007180M64L96.

The present disclosure also provides a pharmaceutical composition, which comprises the siRNA of the present disclosure, or the siRNA conjugate or the prodrug thereof of the present disclosure, and a pharmaceutically acceptable carrier.

The present disclosure also provides a kit, which comprises the siRNA of the present disclosure, or the siRNA conjugate or the prodrug thereof of the present disclosure, or the pharmaceutical composition of the present disclosure.

The present disclosure also provides use of the siRNA of the present disclosure, or the siRNA conjugate or the prodrug thereof of the present disclosure, or the pharmaceutical composition of the present disclosure in preparing a medicament for inhibiting HSD17B13 gene expression.

The present disclosure also provides use of the siRNA of the present disclosure, or the siRNA conjugate or the prodrug thereof of the present disclosure, or the pharmaceutical composition of the present disclosure in preparing a medicament for preventing and/or treating a disease associated with HSD17B13 gene overexpression.

In one embodiment, the disease is selected from non-alcoholic fatty liver disease, non-alcoholic steatohepatitis, cirrhosis, alcoholic hepatitis, hepatic fibrosis, and liver cancer.

The present disclosure also provides a method for inhibiting HSD17B13 gene expression, which comprises contacting a therapeutically effective amount of the siRNA of the present disclosure, or the siRNA conjugate or the prodrug thereof of the present disclosure, or the pharmaceutical composition of the present disclosure with a cell expressing HSD17B13, or administering to a subject in need a therapeutically effective amount of the siRNA of the present disclosure, or the siRNA conjugate or the prodrug thereof of the present disclosure, or the pharmaceutical composition of the present disclosure.

The present disclosure also provides a method for treating and/or preventing a disease associated with HSD17B13 gene overexpression, which comprises administering to a subject in need a therapeutically effective amount of the siRNA of the present disclosure, or the siRNA conjugate or the prodrug thereof of the present disclosure, or the pharmaceutical composition of the present disclosure.

The present disclosure also provides the siRNA of the present disclosure, or the siRNA conjugate or the prodrug thereof of the present disclosure, or the pharmaceutical composition of the present disclosure for use as a medicament.

### Beneficial Effects

The siRNA, the pharmaceutical composition, and the siRNA conjugates provided in the present disclosure exhibit excellent inhibition activity against HSD17B13 gene expression in *in vitro* cell experiments and have good potential for treating diseases associated with HSD17B13 gene overexpression. For example, the siRNA and the conjugates thereof provided in the present disclosure can reduce the expression of HSD17B13 mRNA in the liver, with low toxic and side effects, good plasma stability, long-lasting efficacy, and promising prospects for clinical application.

The siRNA and the siRNA conjugates provided in the present disclosure exhibit a good inhibition effect on the HSD17B13 gene in human liver cancer cells (Huh7 cells).

The siRNA conjugates provided in the present disclosure exhibits a good inhibition effect on the HSD17B13 gene in primary human hepatocytes (PHH cells). In some specific embodiments, in a case where the siRNA conjugates provided in the present disclosure enters PHH through free uptake, the inhibition rate is about 70% or more at 100 nM and about 65% at 10 nM.

In some specific embodiments, the siRNA conjugates of the present disclosure exhibit relatively high inhibition activity against the hHSD17B13 gene *in vivo* and are able to reduce the hHSD17B13 protein level for a long duration.

In some specific embodiments, the siRNA conjugates of the present disclosure are able to significantly reduce the expression level of HSD17B13 mRNA in the liver of cynomolgus monkeys *in vivo*, and are superior to ARO-HSD, a drug under clinical development.

### DETAILED DESCRIPTION OF THE INVENTION

### Definitions

Throughout the specification, unless otherwise specified, in the art, "G", "C", "A", "T", and "U" generally represent the bases of guanine, cytosine, adenine, thymine, and uracil, respectively, but it is also generally known in the art that "G", "C", "A", "T", and "U" each also generally represent nucleotides that contain guanine, cytosine, adenine, thymine, and uracil, respectively, as bases, which is a common way of representing deoxyribonucleic acid sequences and/or ribonucleic acid sequences. Therefore, in the context of the present disclosure, the meanings represented by "G", "C", "A", "T", and "U" include the various possible cases described above. "Nucleotide" and "ribonucleotide" are used interchangeably in the present disclosure, and "deoxyribonucleotide" and "2'-deoxyribonucleotide" are used interchangeably in the present disclosure. The lowercase letters a, u, c, and g represent 2'-methoxy-modified nucleotides; Af, Gf, Cf, and Uf represent 2'-fluoro-modified nucleotides; "dG", "dC", and "dA" represent 2'-deoxyribonucleotides; when "T" is at an overhang position, it represents thymine ribonucleotide (T), and when "T" is in a double-stranded region of a motif, it represents that a uracil ribonucleotide (U) at a corresponding position in the motif sequence is replaced with a thymine ribonucleotide (T); (invAb) is an inverted abasic deoxyribose residue; the lowercase letter s represents that a phosphorothioate linkage is present between two nucleotides immediately adjacent to the left and right of the letter s; P1 represents that one nucleotide immediately adjacent to the right of the P1 is a 5'-phosphate nucleotide; EVP represents that one nucleotide immediately adjacent to the right of the EVP is a 5'-(E)-vinylphosphonate nucleotide; ***A***, ***U***, ***C***, and ***G*** (underlined + bold + italic) represent GNA-modified nucleotides; Base represents a base, such as A, U, G, C, or T.

In the text above and below, the "2'-fluoro-modified nucleotide" refers to a nucleotide formed by substituting hydroxy at the 2' position of a ribosyl group of the nucleotide with fluorine. "Non-fluoro-modified nucleotide" refers to a nucleotide formed by substituting hydroxy at the 2' position of a ribosyl group of the nucleotide with a non-fluorine group or a nucleotide analog. In the present disclosure, "non-fluoro modification" also includes nucleotide replacement, e.g., replacement of uracil (U) with thymine (T); correspondingly, "non-fluoro-modified nucleotide" also includes nucleotides that have been subjected to base replacement described above. In some embodiments, each of the non-fluoro-modified nucleotides is independently selected from one of a nucleotide formed by substituting hydroxy at the 2' position of a ribosyl group of the nucleotide with a non-fluorine group and a nucleotide analog. Nucleotides in which hydroxy at the 2' position of a ribosyl group is substituted with a non-fluorine group are well known to those skilled in the art. These nucleotides may be selected from one of 2'-alkoxy-modified nucleotides, 2'-substituted alkoxy-modified nucleotides, 2'-alkyl-modified nucleotides, 2'-substituted alkyl-modified nucleotides, 2'-amino-modified nucleotides, 2'-substituted amino-modified nucleotides, and 2'-deoxyribonucleotides.

As used in this specification, "the replacement of a nucleotide with a deoxyribonucleotide" refers to the replacement of a ribonucleotide A, U, C, or G with a corresponding deoxyribonucleotide A, T, C, or G during the modification. For example, in the 5' to 3' direction, the nucleotide at position 7 of the antisense strand of N-ER-FY007180 is replaced with a deoxyribonucleotide, which means that the ribonucleotide "U" at position 7 is replaced with "T". Similarly, the ribonucleotide A is replaced with the corresponding deoxyribonucleotide A; the ribonucleotide C is replaced with the corresponding deoxyribonucleotide C; the ribonucleotide G is replaced with the corresponding deoxyribonucleotide G.

"Alkyl" includes linear, branched, or cyclic saturated alkyl groups. For example, the alkyl includes, but is not limited to, methyl, ethyl, propyl, cyclopropyl, n-butyl, isobutyl, sec-butyl, tert-butyl, cyclobutyl, n-pentyl, cyclohexyl, and similar groups. Illustratively, "C₁₋₆" in "C₁₋₆ alkyl" refers to a group containing 1, 2, 3, 4, 5, or 6 carbon atoms arranged in a linear, branched, or cyclic form.

"Alkoxy" as used herein refers to an alkyl group attached to the rest of the molecule via an oxygen atom (-O-alkyl), wherein the alkyl is as defined herein. Non-limiting examples of the alkoxy include methoxy, ethoxy, trifluoromethoxy, difluoromethoxy, n-propoxy, isopropoxy, n-butoxy, tert-butoxy, n-pentyloxy, and the like.

"Nucleotide analog" refers to a group that can replace a nucleotide in a nucleic acid but has a structure different from adenine ribonucleotide, guanine ribonucleotide, cytosine ribonucleotide, uracil ribonucleotide, or thymine deoxyribonucleotide, e.g., a pseudouracil (Ψ), an isonucleotide, a bridged nucleic acid (BNA for short), or an acyclic nucleotide.

Pseudouracil (Ψ) refers to a natural structural analog of uridine, in which the ribose is linked to C5 of a pyrimidine ring instead of N1 of uracil; the structural formula of pseudouracil is shown below:

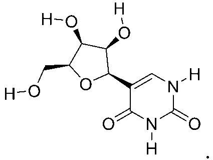

A BNA refers to a constrained or inaccessible nucleotide. A BNA may contain a bridged structure with a "locked" C3'-endo sugar pucker, with forms a five-membered, six-membered, or seven-membered ring. The bridge is typically incorporated at the 2'- and 4'-positions of the ribose to provide a 2',4'-BNA nucleotide, such as LNA, ENA, and cET BNA, wherein LNA is as shown in formula (1), ENA is as shown in formula (2), and cET BNA is as shown in formula (3):

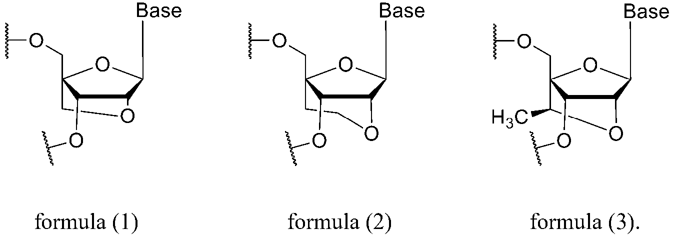

Acyclic nucleotides are a class of nucleotides formed by opening the sugar ring of nucleotides, such as unlocked nucleic acid (UNA) or glycerol nucleic acid (GNA), wherein UNA is as shown in formula (4), and GNA is as shown in formula (5):

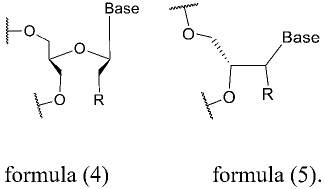

In formula (4) and formula (5), R is selected from H, OH, and alkoxy (O-alkyl).

The isonucleotide refers to a compound formed by changing the position of a base on a ribose ring in a nucleotide, such as a compound formed by moving the base from the 1'-position to the 2'-position or 3'-position of the ribose ring, as shown in formula (6) or (7):

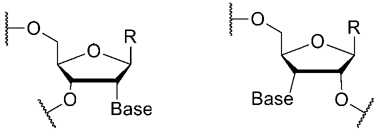

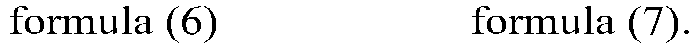

In the compounds of formulas (6)-(7), Base represents a base, such as A, U, G, C, or T; R is selected from H, OH, F, and non-fluorine groups as described above.

In some embodiments, the nucleotide analog is selected from one of pseudouracil, isonucleotide, LNA, ENA, cET BNA, UNA, and GNA. In some embodiments, each of the non-fluoro-modified nucleotides is a 2'-methoxy-modified nucleotide, a GNA-modified nucleotide, or a combination of any two or more thereof. In some preferred embodiments, each of the non-fluoro-modified nucleotides is a 2'-methoxy-modified nucleotide, and in the text above and below, the 2'-methoxy-modified nucleotide refers to a nucleotide formed by substituting the 2'-hydroxy of a ribosyl group with methoxy.

The "2'-methoxy-modified nucleotide" refers to a nucleotide formed by substituting the 2'-hydroxy of a ribosyl group with methoxy. The "phosphorothioate group" refers to a phosphorothioate group formed by substituting one oxygen atom in a phosphodiester bond of the phosphoester group with a sulfur atom.

The "phosphorothioate group" refers to the following formula:

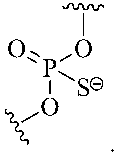

The "5'-phosphate nucleotide" refers to a structure of the following formula:

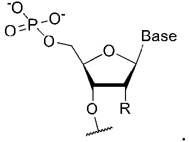

In the context of the specification, the expressions "complementary" and "reversely complementary" are used interchangeably and have the meaning well known to those skilled in the art, that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (G) is always paired with the pyrimidine base cytosine (C). Each base pair comprises a purine and a pyrimidine. When adenines of one strand are always paired with thymines (or uracils) of the other strand and guanines are always paired with cytosines, the two strands are considered complementary to each other, and the sequence of one strand can be deduced from the sequence of its complementary strand. Accordingly, "mismatch" in the art means that in a double-stranded nucleic acid, the bases in the corresponding positions are not paired in a complementary manner.

In the text above and below, unless otherwise specified, "substantially reversely complementary" means that there are no more than 3 base mismatches between two nucleotide sequence segments involved; "virtually reversely complementary" means that there is no more than 1 base mismatch between two nucleotide sequence segments; "completely reversely complementary" means that there is no base mismatch between two nucleotide sequence segments.

In the text above and below, a "nucleotide difference" between one nucleotide sequence and another nucleotide sequence refers to a change in the type of nucleotide base at the same position when comparing the former with the latter. For example, if one nucleotide base in the latter is A, while the corresponding nucleotide base at the same position in the former is U, C, G, or T, it is considered that a nucleotide difference exists between the two nucleotide sequences at that position. In some embodiments, the replacement of a nucleotide at its original position with a base-free nucleotide or an equivalent thereof can also be considered to create a nucleotide difference at that position.

In the context, "double-stranded region" refers to a double-stranded region of a siRNA formed by complementary pairing of a sense strand and an antisense strand of the siRNA; "overhang" refers to one or more unpaired nucleotides protruding from a duplex structure of the siRNA when the 3' end of one strand of the siRNA extends beyond the 5' end of the other strand, or vice versa. "Blunt end" means that there are no unpaired nucleotides at that end of the siRNA, i.e., no nucleotide overhang. A "blunt-ended" siRNA is a siRNA that is fully double-stranded along its entire length, meaning that there are no nucleotide overhangs at either end of the molecule.

In the text above and below of the specification of the present disclosure, especially when describing methods for preparing the siRNA, the pharmaceutical composition, or the siRNA conjugate disclosed herein, unless otherwise specified, the nucleoside monomer refers to a modified or unmodified nucleoside phosphoramidite monomer used in solid-phase phosphoramidite synthesis according to the type and sequence of nucleotides in the siRNA or siRNA conjugate to be prepared. Solid-phase phosphoramidite synthesis is a method used in RNA synthesis, well known to those skilled in the art. The nucleoside monomers used in the present disclosure are all commercially available.

In the context of the present disclosure, unless otherwise stated, "conjugation" means that two or more chemical moieties, each having a specific function, are covalently linked to each other; accordingly, "conjugate" refers to a compound formed by covalent linkage between the various chemical moieties. Further, "siRNA conjugate" refers to a compound formed by covalently linking one or more chemical moieties with specific functions to a siRNA. The siRNA conjugate should be understood as a general term of multiple siRNA conjugates or a siRNA conjugate represented by a certain chemical formula according to the context. In the context of the specification of the present disclosure, "conjugated molecule" should be understood as a specific compound that may be conjugated to a siRNA via a reaction, ultimately forming the siRNA conjugate of the present disclosure.

A variety of hydroxy protecting groups may be used in the present disclosure. In general, protecting groups render a chemical functional group insensitive to specific reaction conditions and may be added to and removed from that functional group in a molecule without substantially damaging the rest of the molecule. In some embodiments, the protecting groups are stable under basic conditions and can be removed under acidic conditions. In some embodiments, non-exclusive examples of hydroxy protecting groups that can be used in the present disclosure include monomethoxytrityl, 9-phenylxanthine-9-yl (Pixyl), and 9-(p-methoxyphenyl)xanthine-9-yl (Mox). In some embodiments, non-exclusive examples of hydroxy protecting groups that can be used in the present disclosure include Tr (trityl), MMTr (4-methoxytrityl), DMTr (4,4'-dimethoxytrityl), and TMTr (4,4',4''-trimethoxytrityl).

As used in the specification, "optional" or "optionally" means that the subsequently described event or circumstance may or may not occur, and that the description includes instances where the event or circumstance occurs and instances where it does not.

The term "subject", as used in the specification, refers to any animal, such as a mammal or a marsupial. Subjects of the present disclosure include, but are not limited to, humans, non-human primates (e.g., rhesus or other types of macaques), mice, pigs, horses, donkeys, cattle, sheep, rats, rabbits, or any species of poultry.

As used in the specification, "treatment" refers to means of obtaining a beneficial or desired result, including but not limited to a treatment benefit. The "treatment benefit" means eradicating or ameliorating the underlying disorder being treated. Furthermore, treatment benefit is obtained by eradicating or ameliorating one or more physiological symptoms associated with the underlying disorder, whereby improvement is observed in the subject, although the subject may still be afflicted by the underlying disorder.

As used in the specification, "prevention" refers to means of obtaining a beneficial or desired result, including but not limited to prophylactic benefit. In order to obtain "prophylactic benefit", a siRNA, a siRNA conjugate, or a pharmaceutical composition may be administered to a subject at risk for a particular disease, or a subject with one or more physiological symptoms of a reported disease, even though a diagnosis of the disease may not have been made.

"Prodrug" as described in the specification refers to a compound that can be converted into an active compound through certain chemical or physiological processes (e.g., enzymatic processes and metabolic hydrolysis). Thus, the term "prodrug" also refers to a precursor of a pharmaceutically acceptable biologically active compound.

In the context, taking into account the case where a 5' end nucleotide of an antisense strand is linked to a 5' hydroxy group (i.e., without a phosphate group), such an antisense strand will first undergo phosphorylation *in vivo* to convert the 5' end nucleotide into a nucleotide carrying a 5' phosphate group, and then exert an effect *in vivo*. Therefore, in the present disclosure, such a siRNA, modified siRNA, and siRNA conjugate are also referred to as a prodrug. For example, the modified siRNA or conjugate having an M6 pattern modification in the present disclosure is a prodrug of the modified siRNA or conjugate having an M2 pattern modification, because the difference between the M2 pattern modification and the M6 pattern modification is whether the 5' end of the antisense strand has P1. Similarly, the relationship between an M7 pattern modification and an M3 pattern modification is also the same. Therefore, in the context, the siRNA includes a corresponding prodrug thereof.

### siRNA

The present disclosure relates to a siRNA capable of inhibiting HSD17B13 gene expression. The siRNA of the present disclosure contains a nucleotide group as a basic structural unit. It is well known to those skilled in the art that the nucleotide group contains a phosphate group, a ribosyl group, and a base. siRNAs that are generally active, i.e., functional, have a length of about 12-40 nucleotides, and in some embodiments, have a length of about 15-30 nucleotides.

The siRNA of the present disclosure comprises a sense strand and an antisense strand, and each nucleotide in the siRNA is independently a modified or unmodified nucleotide, wherein the sense strand comprises a nucleotide sequence I, the antisense strand comprises a nucleotide sequence II, and the nucleotide sequence I is at least partially reversely complementary to the nucleotide sequence II to form a double-stranded region. In some embodiments, the double-stranded region has a length of 15-30 nucleotide pairs. In other embodiments, the double-stranded region has a length of 17-23 nucleotide pairs. In other embodiments, the double-stranded region has a length of 19-21 nucleotide pairs. In yet other embodiments, the double-stranded region has a length of 19 or 21 nucleotide pairs.

In some embodiments, the sense strand further comprises a nucleotide sequence III, and the antisense strand further comprises a nucleotide sequence IV, wherein the nucleotide sequence III and the nucleotide sequence IV each independently have a length of 0-7 nucleotides; the nucleotide sequence III is linked to the 5' end of the nucleotide sequence I, the nucleotide sequence IV is linked to the 3' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences. In some embodiments, the sense strand further comprises a nucleotide sequence III, and the antisense strand further comprises a nucleotide sequence IV, wherein the nucleotide sequence III and the nucleotide sequence IV each independently have a length of 0-7 nucleotides; the nucleotide sequence III is linked to the 3' end of the nucleotide sequence I, the nucleotide sequence IV is linked to the 5' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences. In some embodiments, the sense strand further comprises a nucleotide sequence III, and the antisense strand further comprises a nucleotide sequence IV, wherein the nucleotide sequence III and the nucleotide sequence IV each independently have a length of 0-7 nucleotides; the nucleotide sequence III is linked to the 5' end of the nucleotide sequence I, the nucleotide sequence IV is linked to the 3' end of the nucleotide sequence II, and the nucleotide sequence III and the nucleotide sequence IV are equal in length and virtually reversely complementary or completely reversely complementary; and the nucleotide sequence III is linked to the 3' end of the nucleotide sequence I, the nucleotide sequence IV is linked to the 5' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences.

In some embodiments, the sense strand further comprises a nucleotide sequence V, and/or the antisense strand further comprises a nucleotide sequence VI, wherein the nucleotide sequences V and VI have a length of 0 to 3 nucleotides; the nucleotide sequence V is linked to the 3' end of the sense strand to form the 3' overhang of the sense strand, and/or the nucleotide sequence VI is linked to the 3' end of the antisense strand to form the 3' overhang of the antisense strand. In some embodiments, the nucleotide sequence V or VI has a length of 2 nucleotides. In other embodiments, the nucleotide sequence V or VI is two consecutive thymine deoxyribonucleotides or two consecutive uracil ribonucleotides. In other embodiments, the nucleotide sequence V is identical to or different from a nucleotide at a corresponding position of the target mRNA, or the nucleotide sequence VI is mismatched or complementary to a nucleotide at a corresponding position of the target mRNA.

The sense strand and the antisense strand provided in the present disclosure are identical or different in length, and in some embodiments, the sense strand or the antisense strand has 15-30 nucleotides. In other embodiments, the sense strand or the antisense strand has 19-25 nucleotides. In other embodiments, the sense strand or the antisense strand has 19-23 nucleotides. The length ratio of the sense strand to the antisense strand of the siRNA provided in the present disclosure may be 15/15, 16/16, 17/17, 18/18, 19/19, 19/20, 19/21, 19/22, 19/23, 20/19, 20/20, 20/21, 20/22, 20/23, 21/19, 21/20, 21/21, 21/22, 21/23, 22/19, 22/20, 22/21, 22/22, 22/23, 23/19, 23/20, 23/21, 23/22, 23/23, 24/24, 25/25, 26/26, 27/27, 28/28, 29/29, 30/30, 22/24, 22/25, 22/26, 23/24, 23/25, 23/26, etc. In some embodiments, the length ratio of the sense strand to the antisense strand of the siRNA is 19/19, 21/21, 19/21, 21/23, or 23/23, and at this time, the siRNA of the present disclosure has better cellular mRNA silencing activity.

It is found that different modification strategies can have distinct effects on indicators such as stability, biological activity, and cytotoxicity of siRNA. For example, CN102140458B investigated various chemical modification strategies for siRNAs and validated seven effective modification approaches. Compared with unmodified siRNAs, siRNAs obtained by one of the modification approaches exhibit enhanced blood stability while maintaining inhibitory activity largely comparable to that of unmodified siRNAs.

The nucleotides in the siRNA of the present disclosure are each independently a modified or unmodified nucleotide. In some embodiments, each nucleotide in the siRNA of the present disclosure is an unmodified nucleotide; in some embodiments, some or all of the nucleotides in the siRNA of the present disclosure are modified nucleotides, and these modifications on the nucleotide groups do not cause obvious weakening or loss of the function of the siRNA of the present disclosure in inhibiting the HSD17B13 gene expression.

In some embodiments, the siRNA of the present disclosure comprises at least one modified nucleotide. In the context of the present disclosure, the term "modified nucleotide" as used refers to a nucleotide formed by substituting hydroxy at the 2' position of a ribosyl group of the nucleotide with other groups or a nucleotide analog, or a nucleotide with a modified base. The modified nucleotides do not cause obvious weakening or loss of the function of the siRNA in inhibiting gene expression. For example, the modified nucleotides disclosed in J.K. Watts, G.F. Deleavey, and M.J. Damha, Chemically modified siRNA: tools and applications. Drug Discov Today, 2008, 13(19-20): 842-55 may be selected.

In some embodiments, at least one nucleotide in the sense strand or the antisense strand of the siRNA provided in the present disclosure is a modified nucleotide, and/or at least one phosphoester group is a phosphoester group with a modification group; in other words, at least a portion of a phosphoester group and/or a ribosyl group in a phosphate-sugar backbone of at least one single strand of the sense strand and the antisense strand is a phosphoester group with a modification group and/or a ribosyl group with a modification group. In some embodiments, the phosphoester group with a modification group is a phosphorothioate group formed by substituting one oxygen atom in a phosphodiester bond of the phosphoester group with a sulfur atom.

In some embodiments, the siRNA comprises a sense strand without a 3' overhang nucleotide; that is, while the sense strand of the siRNA may have a 3' overhang nucleotide, a blunt end may be formed by removing the 3' overhang nucleotide from the sense strand.

In some embodiments, when the nucleotide sequence of the sense strand and the nucleotide sequence of the antisense strand are complementary to form a double-stranded region, and there is no protruding nucleotide at the 3' end of the sense strand, nucleotide sequence V is added to the 3' end of the sense strand as the protruding nucleotide. Subsequently, after chemical modification of the nucleotide sequence formed by linking nucleotide sequence V to the 3' end of the sense strand is completed, nucleotide sequence V is removed, and correspondingly, a blunt end is formed in the sense strand of the siRNA.

In some embodiments, when the nucleotide sequence of the sense strand and the nucleotide sequence of the antisense strand are complementary to form a double-stranded region, and the 3' end of the sense strand has a protruding nucleotide extending out of the double-stranded region, the protruding nucleotide located at the 3' end of the sense strand is removed to serve as the nucleotide sequence of the sense strand, and correspondingly, a blunt end is formed in the sense strand of the siRNA.

In some embodiments, the 5' end nucleotide of the antisense strand is linked to a 5' phosphate group or a 5' phosphate-derived group.

An exemplary structure of the 5' phosphate group is: 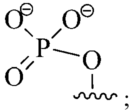 the structure of the 5' phosphate-derived group includes, but is not limited to: 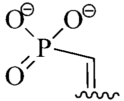 (EVP), 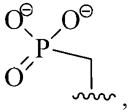 and the like.

After the 5' end nucleotide located on the antisense strand is linked to a 5' phosphate group or a 5' phosphate-derived group, the following structures are formed: 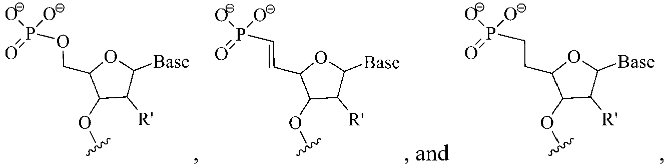 wherein Base represents a base, e.g., A, U, G, C, or T. R' is hydroxy or is substituted with various groups known to those skilled in the art; for example, the modified nucleotide after substitution may be a 2'-fluoro (2'-F)-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, or a 2'-deoxyribonucleotide.

In some embodiments, the 5'-end nucleotide of the sense strand or the antisense strand is not linked to a 5' phosphate group or a 5' phosphate-derived group (i.e., the ribosyl group of the 5' end nucleotide of the sense strand or the antisense strand is a 5' hydroxy group), and its structure is shown below: 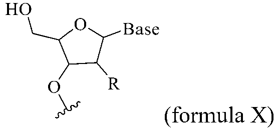 wherein Base represents a base, e.g., A, U, G, C, or T. R is hydroxy or hydrogen or is substituted with various groups known to those skilled in the art; for example, R may be 2'-fluoro (2'-F), 2'-alkoxy, 2'-substituted alkoxy, 2'-alkyl, 2'-substituted alkyl, 2'-amino, or 2'-substituted amino.

An exemplary modified nucleotide has the structure shown below: 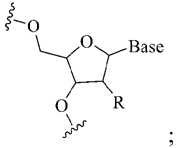 wherein Base represents a base, e.g., A, U, G, C, or T. The hydroxy at the 2' position of the ribosyl group is substituted with R. The hydroxy at the 2' position of these ribosyl groups may be substituted with various groups known to those skilled in the art; for example, the modified nucleotide after substitution may be a 2'-fluoro (2'-F)-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, or a 2'-deoxyribonucleotide.

In some embodiments, the sense strand may comprise one or more capping residues or moieties, sometimes referred to in the art as a "cap", "terminal cap", or "capping residue". As used herein, "capping residue" is a non-nucleotide compound or other moiety that can be incorporated at one or more ends of a nucleotide sequence of the siRNA disclosed herein. In some cases, the capping residue can provide the siRNA with certain beneficial properties, such as protection against exonuclease degradation. In some embodiments, an inverted abasic deoxyribose residue (invAb) is added as the capping residue. In some embodiments, the capping residue is present at the 5' end, the 3' end, or both the 5' and 3' ends of the sense strand.

In some embodiments, one or more inverted abasic deoxyribose residues (invAb) are added to the 3' end of the sense strand. In some embodiments, one or more inverted abasic deoxyribose residues (invAb) are added to the 5' end of the sense strand. In some embodiments, one or more inverted abasic deoxyribose residues (invAb) are added to the 5' end of the sense strand and the 3' end of the sense strand. The inverted abasic deoxyribose residue and the end nucleotide may be linked via a phosphodiester bond, a phosphorothioate group, or other internucleoside bonds. The chemical structure of the inverted abasic deoxyribose residue is shown below:
formula A when (invAb) is located inside the siRNA; formula B when (invAb) is located at the 3' end of the siRNA; and formula C when (invAb) is located at the 5' end of the siRNA: 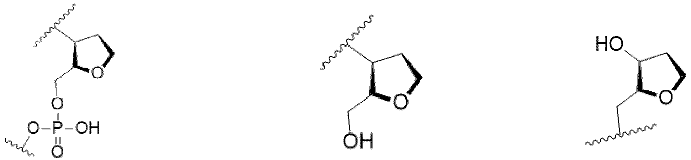 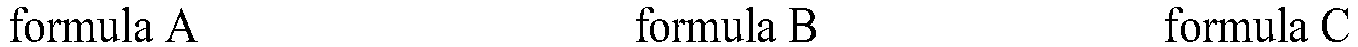
wherein a bond of a phosphodiester bond part of formula A is a bond toward the 3' end of the siRNA.

### siRNA Conjugate

The present disclosure relates to a siRNA conjugate, and the siRNA conjugate comprises the siRNA described above and a conjugated group conjugated to the siRNA.

In the present disclosure, the sense strand and the antisense strand of the siRNA conjugate form a double-stranded region of the siRNA conjugate, and a blunt end is formed at the 3' end of the sense strand of the siRNA conjugate. In some embodiments, a blunt end is formed at the 3' end of the sense strand of the siRNA conjugate, and the 3' end of the antisense strand of the siRNA conjugate has 1-3 protruding nucleotides extending out of the double-stranded region. In other embodiments, a blunt end is formed at the 3' end of the sense strand of the siRNA conjugate, and a blunt end is formed at the 3' end of the antisense strand of the siRNA conjugate.

In some preferred embodiments, the siRNA conjugate is obtained by conjugating the siRNA to a conjugated group. The sense strand and the antisense strand of the siRNA are complementary to form a double-stranded region of the siRNA, a blunt end is formed at the 3' end of the sense strand of the siRNA, and the conjugated group is conjugated to the 3' end of the sense strand with the blunt end to form the siRNA conjugate.

In some preferred embodiments, the 3' end of the sense strand of the siRNA has a protruding nucleotide extending out of the double-stranded region, the sequence with a 3' blunt end formed by removing the protruding nucleotide located at the 3' end of the sense strand is used as a nucleotide sequence for linking to a conjugated group, and the conjugated group is linked to the 3' blunt end of the sense strand to form the siRNA conjugate.

In some more preferred embodiments, when the nucleotide sequence of the sense strand and the nucleotide sequence of the antisense strand are complementary to form a double-stranded region, and there is no protruding nucleotide at the 3' end of the sense strand, a nucleotide sequence V is added to the 3' end of the sense strand as the protruding nucleotide. The sequence with a 3' blunt end formed by removing the protruding nucleotide located at the 3' end of the sense strand is used as a nucleotide sequence for linking to a conjugated group, and the conjugated group is linked to the 3' blunt end of the sense strand to form the siRNA conjugate.

In some more preferred embodiments, when the nucleotide sequence of the sense strand and the nucleotide sequence of the antisense strand are complementary to form a double-stranded region, and the 3' end of the sense strand has a protruding nucleotide extending out of the double-stranded region, the sequence with a 3' blunt end formed by removing the protruding nucleotide located at the 3' end of the sense strand is used as a nucleotide sequence for linking to a conjugated group, and the conjugated group is linked to the 3' blunt end of the sense strand to form the siRNA conjugate.

Illustratively, for a siRNA having a sequence as shown in N-ER-FY007166M2, the 3' end of the sense strand of the siRNA has a protruding nucleotide extending out of the double-stranded region, and the blunt-ended sequence gsusaagaAfgUfCfUfgauagaua formed by removing the protruding -sTsT nucleotide located at the 3' end of the sense strand is used as a nucleotide sequence for linking to an L96 conjugated group. Therefore, the sequences forming the siRNA conjugate is as follows: the sense strand is gsusaagaAfgUfCfUfgauagauaL96, and the antisense strand is P1usAfsucuAfucagacuUfcUfuacsTsT.

Generally, the conjugated group comprises at least one pharmaceutically acceptable targeting group or optionally further comprises a linker, and the siRNA, the linker, and the targeting group are linked sequentially. In some embodiments, the number of the targeting groups is 1-6. In some embodiments, the number of the targeting groups is 2-4. The siRNA molecule may be non-covalently or covalently conjugated to the conjugated group, for example, covalently conjugated to the conjugated group. The conjugation site between the siRNA and the conjugated group may be at the 3' end or 5' end of the sense strand of the siRNA, or at the 5' end of the antisense strand, or in the internal sequence of the siRNA. In some embodiments, the conjugation site between the siRNA and the conjugated group is at the 3' end of the sense strand of the siRNA.

In some embodiments, the conjugated group may be linked to the phosphate group, the 2'-position hydroxy, or the base of the nucleotide. In some embodiments, the conjugated group may also be linked to the 3'-position hydroxy, and at this time, the nucleotides are linked via a 2'-5' phosphodiester bond. When the conjugated group is linked to the end of the siRNA chain, the conjugated group is usually linked to the phosphate group of the nucleotide; when the conjugated group is linked to the internal sequence of the siRNA, the conjugated group is usually linked to the ribose sugar ring or the base. For various linkage methods, please refer to the literature: Muthiah Manoharan et.al., siRNA conjugates carrying sequentially assembled trivalent N-acetylgalactosamine linked through nucleosides elicit robust gene silencing in vivo in hepatocytes. ACS Chemical biology, 2015, 10(5): 1181-7.

In some embodiments, the siRNA and the conjugated group may be linked via acid-labile or reducible chemical bonds, which are degradable in the acidic environment of the cell endosome, thereby enabling the siRNA to be in a free state. For non-degradable conjugation methods, the conjugated group can be linked to the sense strand of the siRNA to minimize the effect of conjugation on the activity of the siRNA.

In some embodiments, the pharmaceutically acceptable targeting group may be a ligand conventionally used in the field of siRNA administration, such as the various ligands described in WO2009082607A2, which is incorporated herein by reference in its entirety.

In some embodiments, the pharmaceutically acceptable targeting group may be selected from one or more of ligands formed by the following targeting molecules or derivatives thereof: lipophilic molecules such as cholesterol, bile acids, vitamins (e.g., vitamin E), and lipid molecules of different chain lengths; polymers such as polyethylene glycol; polypeptides such as transmembrane peptides; aptamers; antibodies; quantum dots; sugars such as lactose, polylactose, mannose, galactose, and N-acetylgalactosamine (GalNAc); folate; ligands for receptors expressed by hepatocytes such as asialoglycoprotein, asialosaccharide residues, lipoproteins (e.g., high-density lipoprotein, low-density lipoprotein, etc.), glucagon, neurotransmitters (e.g., epinephrine), growth factors, transferrin, and the like.

In some embodiments, each of the ligands is independently selected from a ligand capable of binding to a cell surface receptor. In some embodiments, at least one ligand is a ligand capable of binding to a hepatocyte surface receptor. In some embodiments, at least one ligand is a ligand capable of binding to a mammalian cell surface receptor. In some embodiments, at least one ligand is a ligand capable of binding to a human hepatocyte surface receptor. In some embodiments, at least one ligand is a ligand capable of binding to the liver surface asialoglycoprotein receptor (ASGPR). The types of these ligands are well known to those skilled in the art, and their functions are generally to bind to specific receptors on the surface of target cells and mediate the delivery of a siRNA linked to a ligand to the target cells.

In some embodiments, the pharmaceutically acceptable targeting group may be any ligand that binds to the asialoglycoprotein receptor (ASGPR) on the surface of mammalian hepatocytes. In some embodiments, each ligand is independently an asialoglycoprotein, such as asialoorosomucoid (ASOR) or asialofetuin (ASF). In some embodiments, the ligands are sugars or sugar derivatives.

In some embodiments, at least one ligand is a sugar. In some embodiments, each ligand is a sugar. In some embodiments, at least one ligand is a monosaccharide, a polysaccharide, a modified monosaccharide, a modified polysaccharide, or a sugar derivative. In some embodiments, at least one of the ligands may be a monosaccharide, a disaccharide, or a trisaccharide. In some embodiments, at least one ligand is a modified sugar. In some embodiments, each ligand is a modified sugar. In some embodiments, each ligand is independently selected from a polysaccharide, a modified polysaccharide, a monosaccharide, a modified monosaccharide, a polysaccharide derivative, and a monosaccharide derivative. In some embodiments, each or at least one ligand is selected from the group consisting of the following sugars: glucose and derivatives thereof, mannan and derivatives thereof, galactose and derivatives thereof, xylose and derivatives thereof, ribose and derivatives thereof, fucose and derivatives thereof, lactose and derivatives thereof, maltose and derivatives thereof, arabinose and derivatives thereof, fructose and derivatives thereof, and sialic acid.

In some embodiments, each of the ligands can be independently selected from D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, 2,3,4-tri-O-acetyl-1-thio-6-O-trityl-α-D-glucopyranoside methyl ester, 4-thio-β-D-galactopyranose, 3,4,6,7-tetra-O-acetyl-2-deoxy-1,5-dithio-α-D-glucopyranoside ethyl ester, 2,5-anhydro-D-allose nitrile, ribose, D-ribose, D-4-thioribose, L-ribose, and L-4-thioribose. Other options for the ligands can be found, for example, in CN105378082A, which is incorporated herein by reference in its entirety.

In some embodiments, the pharmaceutically acceptable targeting group in the siRNA conjugate may be galactose or N-acetylgalactosamine, wherein the galactose or N-acetylgalactosamine molecule may be monovalent, divalent, trivalent, or tetravalent. It should be understood that the monovalent, divalent, trivalent, or tetravalent described herein refers to that after a siRNA molecule forms a siRNA conjugate with a conjugated group containing a galactose or N-acetylgalactosamine molecule as a targeting group, the molar ratio of the siRNA molecule to the galactose or N-acetylgalactosamine molecule in the siRNA conjugate is 1:1, 1:2, 1:3, or 1:4. In some embodiments, the pharmaceutically acceptable targeting group is N-acetylgalactosamine. In some embodiments, when the siRNA described in the present disclosure is conjugated to a conjugated group comprising N-acetylgalactosamine, the N-acetylgalactosamine molecule is trivalent or tetravalent. In some embodiments, when the siRNA described in the present disclosure is conjugated to a conjugated group containing N-acetylgalactosamine, the N-acetylgalactosamine molecule is trivalent.

The targeting group may be linked to the siRNA molecule via a suitable linker, and those skilled in the art may select a suitable linker according to the specific type of the targeting group. The types of these linkers and targeting groups and the linkage methods with the siRNA can be found in the disclosure of WO2015006740A2, which is incorporated herein by reference in its entirety.

### Synthesis Method for siRNAs

Nucleoside monomers are linked one by one in the 3' to 5' direction according to a nucleotide arrangement sequence by a conventional solid-phase phosphoramidite method in the art. The linkage of each nucleoside monomer involves four reactions: deprotection, coupling, oxidation or sulfurization, and capping. When a phosphoester linkage is present between two nucleotides, the steps for linking the subsequent nucleoside monomer include deprotection, coupling, oxidation, and capping. When a phosphorothioate linkage is present between two nucleotides, the steps for linking the subsequent nucleoside monomer include deprotection, coupling, sulfurization, and capping. In the present disclosure, nucleotide monomers are selected according to the target sequence to be synthesized, and the selected nucleotide monomers are nucleotide monomers commonly used by those skilled in the art. For example, the nucleotide monomer for synthesizing A may be, but is not limited to, adenosine-3-phosphate. It should be understood that these monomers, when present in the oligonucleotide, are linked to each other via a 5'-3' phosphodiester bond or a 5'-3' phosphorothioate group, and in cases where, for example, the last nucleotide in the 5' to 3' direction has a hydroxyl group at its 3' position, this is achieved according to conventional techniques in the art.

For example, the synthesis conditions of the siRNA of the present disclosure may be as follows:
Deprotection conditions include: a reaction temperature of 25°C, a reaction time of 70 s, a deprotection reagent selected from a solution of dichloroacetic acid in dichloromethane (3% v/v), and a molar ratio of the deprotection reagent to the protecting group 4,4'-dimethoxytrityl on the solid-phase support of 5:1.

Coupling reaction conditions include: a reaction temperature of 25°C, a reaction time of 600 s, a coupling reagent selected from a 0.25 M solution of 5-ethylthio-1H-tetrazole (ETT) in acetonitrile, a molar ratio of the nucleic acid sequence linked to the solid-phase support to the nucleoside monomer of 1:10, and a molar ratio of the nucleic acid sequence linked to the solid-phase support to the coupling reagent of 1:65.

Oxidation reaction conditions include: a reaction temperature of 25°C, a reaction time of 15 s, an oxidation reagent selected from a 0.05 M solution of iodine in tetrahydrofuran, and a molar ratio of the oxidation reagent to the nucleic acid sequence linked to the solid-phase support in the coupling step of 30:1. The reaction is performed in a mixed solvent of tetrahydrofuran:water:pyridine = 3:1:1.

Sulfurization reaction conditions include: a reaction temperature of 25°C, a reaction time of 300 s, a sulfurization reagent selected from xanthane hydride, and a molar ratio of the sulfurization reagent to the nucleic acid sequence linked to the solid-phase support in the coupling step of 120:1. The reaction is performed in a mixed solvent of acetonitrile:pyridine = 1:1.

Capping reaction conditions include: a reaction temperature of 25°C, a reaction time of 15 s, a capping reagent selected from a mixed solution of CapA (a 10% solution of acetic anhydride in acetonitrile) and CapB (a 10% solution of N-methylimidazole in pyridine/acetonitrile) in a molar ratio of 1:1, and a molar ratio of the capping reagent to the nucleic acid sequence linked to the solid-phase support, the molar ratio being acetic anhydride:N-methylimidazole:nucleic acid sequence linked to the solid-phase support = 1:1:1.

After all nucleoside monomers are linked, ammonolysis, purification, and desalting are sequentially performed on the nucleic acid sequence linked to the solid-phase support to obtain a siRNA sense strand and a siRNA antisense strand, and finally, the two strands are subjected to heating and annealing to obtain a product.

Methods of ammonolysis, purification, desalting, and annealing are well known in the art. For example, ammonolysis is performed by contacting the nucleotide sequence linked to the solid-phase support with concentrated ammonia water; purification is performed by chromatography; desalting is performed by reversed-phase chromatography; annealing is performed by mixing the sense strand and the antisense strand in an equimolar ratio under different stringency conditions, followed by gradual cooling.

The synthesized siRNAs are shown in Table 1 and Table 1-1.

### Synthesis Method for siRNA Conjugates

Taking the synthesis of L96 as an example:

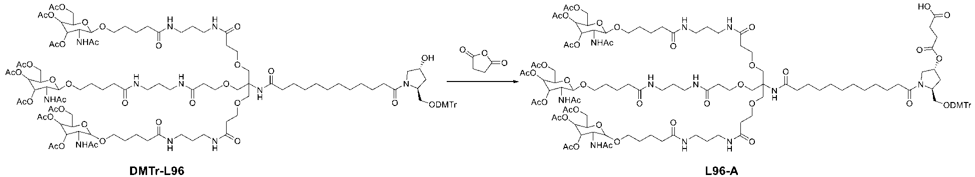

Step 1: Reaction of DMTr-L96 with succinic anhydride to obtain compound L96-A:
Preparation process: DMTr-L96, succinic anhydride, 4-dimethylaminopyridine, and diisopropylethylamine are added to dichloromethane, and the mixture is reacted under stirring at 25°C for 24 h. The reaction liquid is then washed with 0.5 M triethylammonium phosphate. The aqueous phase is washed three times with dichloromethane, and the organic phases are combined and evaporated to dryness under reduced pressure to obtain a crude product; purification is then performed by column chromatography to obtain pure L96-A.

Step 2: Reaction of L96-A with NH₂-SPS to obtain L96-B:

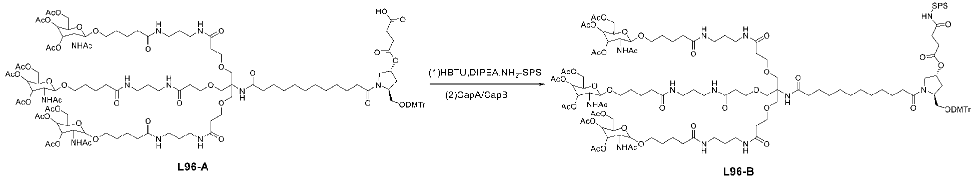

Preparation process: L96-A, O-(benzotriazol-1-yl)-N,N,N',N'-tetramethyluronium hexafluorophosphate (HBTU), and diisopropylethylamine (DIPEA) are mixed and dissolved in acetonitrile. The mixture is stirred at room temperature for 5 min to obtain a homogeneous solution. An aminomethyl resin (NH₂-SPS, 100-200 mesh) is added to the reaction liquid. The mixture is reacted on a shaker at 25°C for 18 h and then filtered, and the filter cake is washed sequentially with dichloromethane and acetonitrile to obtain a filter cake. Capping reaction is performed on the resulting filter cake with a CapA/CapB mixed solution to obtain L96-B, i.e., a solid-phase support comprising a conjugated molecule.

Step 3: Synthesis of siRNA conjugate:
The sense strand of a siRNA conjugate is synthesized by using L96-B as a solid-phase support according to the siRNA synthesis method described above, and the antisense strand of the siRNA conjugate is synthesized using the siRNA synthesis method described above. Annealing is performed to generate the siRNA conjugate of the present disclosure.

The synthesized siRNA conjugates are shown in Table 2.

### Pharmaceutical Composition

The present disclosure provides a pharmaceutical composition, and the pharmaceutical composition comprises the siRNA described above as an active ingredient and a pharmaceutically acceptable carrier.

The pharmaceutically acceptable carrier may be a carrier commonly used in the field of siRNA administration, for example, but not limited to one or more of lipid nanoparticles (LNPs), magnetic nanoparticles (such as nanoparticles based on Fe₃O₄ or Fe₂O₃), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid)copolymer (PLGA), poly(2-aminoethyl ethylene phosphate) (PPEEA), and poly(2-dimethylaminoethyl methacrylate) (PDMAEMA), as well as derivatives thereof.

In the pharmaceutical composition, there is no special requirement on the contents of the siRNA and the pharmaceutically acceptable carrier, and the content may be a conventional content of each component.

In some embodiments, the pharmaceutical composition may further comprise other pharmaceutically acceptable excipients, which may be one or more of various formulations or compounds conventionally used in the art. For example, the other pharmaceutically acceptable excipients may include at least one of a pH buffer, a protective agent, and an osmotic pressure regulator.

The pH buffer may be a tris(hydroxymethyl)aminomethane hydrochloride buffer with a pH value of 7.5-8.5 and/or a phosphate buffer with a pH value of 5.5-8.5, for example, a phosphate buffer with a pH value of 5.5-8.5.

The protective agent may be at least one of inositol, sorbitol, sucrose, trehalose, mannose, maltose, lactose, and glucose. Based on the total weight of the pharmaceutical composition, the content of the protective agent may be 0.01-30 wt%.

The osmotic pressure regulator may be sodium chloride and/or potassium chloride. The content of the osmotic pressure regulator makes the osmotic pressure of the pharmaceutical composition 200-700 milliosmol/kg (mOsm/kg). According to the desired osmotic pressure, those skilled in the art may easily determine the content of the osmotic pressure regulator.

In some embodiments, the pharmaceutical composition may be a liquid formulation, such as an injection; it may also be a freeze-dried powder injection, which is mixed with a liquid excipient to prepare a liquid formulation during administration. The liquid formulation may be used for, but is not limited to, subcutaneous, intramuscular, or intravenous injection administration, and may also be administered via, but not limited to, spray to the lungs or spray to other organ tissues (e.g., the liver) through the lungs. In some embodiments, the pharmaceutical composition is used for intravenous injection administration.

In some embodiments, the pharmaceutical composition may be in the form of a liposomal formulation. In some embodiments, the pharmaceutically acceptable carrier used in the liposomal formulation comprises an amine-containing transfection compound (hereinafter also referred to as an organic amine), a helper lipid, and/or a PEGylated lipid.

The following examples are intended to further illustrate the present disclosure, but not to limit the present disclosure in any way.

### EXAMPLES

Other objects, features, and advantages of the present disclosure will become apparent from the following detailed description. However, it should be understood that the detailed description and the specific examples, while indicating specific examples of the present disclosure, are provided for explanatory purposes only. After reading this detailed description, various changes and modifications made within the spirit and scope of the present disclosure will become apparent to those skilled in the art.

The experimental techniques and methods used in the examples, unless otherwise specified, are all conventional techniques and methods. For example, in the following examples, if no specific conditions are indicated, the experimental methods are generally performed under conventional conditions, such as those described in Sambrook et al., Molecular Cloning: A Laboratory Manual (New York: Cold Spring Harbor Laboratory Press, 1989) or those as recommended by the manufacturer. All materials, reagents, and the like used in the examples are commercially available unless otherwise specified.

### Example 1. Preparation of siRNAs

siRNA molecules having the following sequences were synthesized by Shanghai Talen-bio Scientific Co.,Ltd.

**Table 1. siRNAs and sequences thereof**

| siRNA ID | Ss: 5'- 3' | SEQ ID NO | As: 5'-3' | SEQ ID NO |
|---|---|---|---|---|
| N-ER-FY007104 | GGUUCUGUGGAUAUUA AUAAGC | 1 | UUAUUAAUAUCCCAC AGAACCAA | 2 |
| N-ER-FY007104M2 | gsgsuucuGfuGfGfGfauauuaau aasgsc | 3 | P1usUfsauuAfauaucccAfc Afgaaccsasa | 4 |
| N-ER-FY007104M3 | gsgsuucuGfuGfGfGfauauuaau aasgsc | 3 | P1usUfsauuAfaUfAfucccA fcAfgaaccsasa | 5 |
| N-ER-FY007104M4 | gsgsuucuGfuGfGfGfauauuaau aasgsc | 3 | P1usUfsauuAauaucccAfcA fgaaccsasa | 6 |
| N-ER-FY007104M5 | gsgsuucuGfuGfGfGfauauuaau aasgsc | 3 | P1usUfsauuAfAuaucccAfc Afgaaccsasa | 7 |
| N-ER-FY007104M6 | gsgsuucuGfuGfGfGfauauuaau aasgsc | 3 | usUfsauuAfauaucccAfcAfg aaccsasa | 8 |
| N-ER-FY007104M7 | gsgsuucuGfuGfGfGfauauuaau aasgsc | 3 | usUfsauuAfaUfAfucccAfc Afgaaccsasa | 9 |
| N-ER-FY007104M8 | gsgsuucuGfuGfGfGfauauuaau aasgsc | 3 | EVPusUfsauuAfauaucccAf cAfgaaccsasa | 10 |
| N-ER-FY007104M9 | gsgsuucuGfuGfGfGfauauuaau aasgsc | 3 | EVPusUfsauuAfaUfAfuccc AfcAfgaaccsasa | 11 |
| N-ER-FY007127 | CACCAAGGAUGAAGAGA UUAACA | 12 | UUAAUCUCUUCAUCC UUGGUGCU | 13 |
| N-ER-FY007127M2 | csasccaaGfgAfUfGfaagagauu aascsa | 14 | P1usUfsaauCfucuucauCfc Ufuggugscsu | 15 |
| N-ER-FY007127M3 | csasccaaGfgAfUfGfaagagauu aascsa | 14 | P1usUfsaauCfuCfUfucauCf cUfuggugscsu | 16 |
| N-ER-FY007127M4 | csasccaaGfgAfUfGfaagagauu aascsa | 14 | P1usUfsaauCucuucauCfcU fuggugscsu | 17 |
| N-ER-FY007127M5 | csasccaaGfgAfUfGfaagagauu aascsa | 14 | P1usUfsaauCfUcuucauCfc Ufuggugscsu | 18 |
| N-ER-FY007127M6 | csasccaaGfgAfUfGfaagagauu aascsa | 14 | usUfsaauCfucuucauCfcUfu ggugscsu | 19 |
| N-ER-FY007127M7 | csasccaaGfgAfUfGfaagagauu aascsa | 14 | usUfsaauCfuCfUfucauCfc Ufuggugscsu | 20 |
| N-ER-FY007127M8 | csasccaaGfgAfUfGfaagagauu aascsa | 14 | EVPusUfsaauCfucuucauCf cUfuggugscsu | 21 |
| N-ER-FY007127M9 | csasccaaGfgAfUfGfaagagauu aascsa | 14 | EVPusUfsaauCfuCfUfucau CfcUfuggugscsu | 22 |
| N-ER-FY007128 | CCAAGGAUGAAGAGAUU AACA | 23 | UUAAUCUCUUCAUCC UUGGUG | 24 |
| N-ER-FY007129 | CCAAGGAUGAAGAGAUU AA | 25 | UUAAUCUCUUCAUCC UUGG | 26 |
| N-ER-FY007159 | GUUCACCAAAAAUCCAA GCAAAA | 27 | UUGCUUGGAUUUUG GUGAACCC | 28 |
| N-ER-FY007159M2 | gsusucacCfaAfAfAfauccaagc aasasa | 29 | P1usUfsgcuUfggauuuuUfg Gfugaacscsc | 30 |
| N-ER-FY007159M3 | gsusucacCfaAfAfAfauccaagc aasasa | 29 | P1usUfsgcuUfgGfAfuuuuU fgGfugaacscsc | 31 |
| N-ER-FY007159M4 | gsusucacCfaAfAfAfauccaagc aasasa | 29 | P1usUfsgcuUggauuuuUfg Gfugaacscsc | 32 |
| N-ER-FY007159M5 | gsusucacCfaAfAfAfauccaagc aasasa | 29 | P1usUfsgcuUfGgauuuuUfg Gfugaacscsc | 33 |
| N-ER-FY007159M6 | gsusucacCfaAfAfAfauccaagc aasasa | 29 | usUfsgcuUfggauuuuUfgGf ugaacscsc | 34 |
| N-ER-FY007159M7 | gsusucacCfaAfAfAfauccaagc aasasa | 29 | usUfsgcuUfgGfAfuuuuUfg Gfugaacscsc | 35 |
| N-ER-FY007159M8 | gsusucacCfaAfAfAfauccaagc aasasa | 29 | EVPusUfsgcuUfggauuuuUf gGfugaacscsc | 36 |
| N-ER-FY007159M9 | gsusucacCfaAfAfAfauccaagc aasasa | 29 | EVPusUfsgcuUfgGfAfuuu uUfgGfugaacscsc | 37 |
| N-ER-FY007165 | GUAAGAAGUCUGAUAGA UAGA | 38 | UAUCUAUCAGACUUC UUACGA | 39 |
| N-ER-FY007166 | GUAAGAAGUCUGAUAGA UA | 40 | UAUCUAUCAGACUUC UUAC | 41 |
| N-ER-FY007166M2 | gsusaagaAfgUfCfUfgauagaua sTsT | 42 | P1usAfsucuAfucagacuUfc UfuacsTsT | 43 |
| N-ER-FY007166M3 | gsusaagaAfgUfCfUfgauagaua sTsT | 42 | P1usAfsucuAfuCfAfgacuU fcUfuacsTsT | 44 |
| N-ER-FY007166M4 | gsusaagaAfgUfCfUfgauagaua sTsT | 42 | P1usAfsucuAucagacuUfcU fuacsTsT | 45 |
| N-ER-FY007166M5 | gsusaagaAfgUfCfUfgauagaua sTsT | 42 | P1usAfsucuAfUcagacuUfc UfuacsTsT | 46 |
| N-ER-FY007166M6 | gsusaagaAfgUfCfUfgauagaua sTsT | 42 | usAfsucuAfucagacuUfcUfu acsTsT | 47 |
| N-ER-FY007166M7 | gsusaagaAfgUfCfUfgauagaua sTsT | 42 | usAfsucuAfuCfAfgacuUfc UfuacsTsT | 48 |
| N-ER-FY007166M8 | gsusaagaAfgUfCfUfgauagaua sTsT | 42 | EVPusAfsucuAfucagacuUf cUfuacsTsT | 49 |
| N-ER-FY007166M9 | gsusaagaAfgUfCfUfgauagaua sTsT | 42 | EVPusAfsucuAfuCfAfgacu UfcUfuacsTsT | 50 |
| N-ER-FY007166M10 | gsusaagaAfgUfCfUfgauagaua sTsT | 42 | usAfsucuAfucagacuUfcuua csTsT | 51 |
| N-ER-FY007166M11 | gsusaagaAfgUfCfUfgauagaua sTsT | 42 | usAfsucuAfuCfAfgacuUfc uuacsTsT | 52 |
| N-ER-FY007168 | GAUAGAUGGAAUACUUA CA | 53 | UGUAAGUAUUCCAUC UAUC | 54 |
| N-ER-FY007168M2 | gsasuagaUfgGfAfAfuacuuaca sTsT | 55 | P1usGfsuaaGfuauuccaUfc UfaucsTsT | 56 |
| N-ER-FY007168M3 | gsasuagaUfgGfAfAfuacuuaca sTsT | 55 | P1usGfsuaaGfuAfUfuccaU fcUfaucsTsT | 57 |
| N-ER-FY007168M4 | gsasuagaUfgGfAfAfuacuuaca sTsT | 55 | P1usGfsuaaGuauuccaUfcU faucsTsT | 58 |
| N-ER-FY007168M5 | gsasuagaUfgGfAfAfuacuuaca sTsT | 55 | P1usGfsuaaGfUauuccaUfc UfaucsTsT | 59 |
| N-ER-FY007168M6 | gsasuagaUfgGfAfAfuacuuaca sTsT | 55 | usGfsuaaGfuauuccaUfcUfa ucsTsT | 60 |
| N-ER-FY007168M7 | gsasuagaUfgGfAfAfuacuuaca sTsT | 55 | usGfsuaaGfuAfUfuccaUfc UfaucsTsT | 61 |
| N-ER-FY007168M8 | gsasuagaUfgGfAfAfuacuuaca sTsT | 55 | EVPusGfsuaaGfuauuccaUf cUfaucsTsT | 62 |
| N-ER-FY007168M9 | gsasuagaUfgGfAfAfuacuuaca sTsT | 55 | EVPusGfsuaaGfuAfUfucca UfcUfaucsTsT | 63 |
| N-ER-FY007193 | GAUAGAUGGAAUACUUA CAAA | 64 | UGUAAGUAUUCCAUC UAUCAG | 65 |
| N-ER-FY007193M2 | gsasuagaUfgGfAfAfuacuuaca sasa | 66 | P1usGfsuaaGfuauuccaUfc Ufaucsasg | 67 |
| N-ER-FY007193M3 | gsasuagaUfgGfAfAfuacuuaca sasa | 66 | P1usGfsuaaGfuAfUfuccaU fcUfaucsasg | 68 |
| N-ER-FY007193M4 | gsasuagaUfgGfAfAfuacuuaca sasa | 66 | P1usGfsuaaGuauuccaUfcU faucsasg | 69 |
| N-ER-FY007193M5 | gsasuagaUfgGfAfAfuacuuaca sasa | 66 | P1usGfsuaaGfUauuccaUfc Ufaucsasg | 70 |
| N-ER-FY007193M6 | gsasuagaUfgGfAfAfuacuuaca sasa | 66 | usGfsuaaGfuauuccaUfcUfa ucsasg | 71 |
| N-ER-FY007193M7 | gsasuagaUfgGfAfAfuacuuaca sasa | 66 | usGfsuaaGfuAfUfuccaUfc Ufaucsasg | 72 |
| N-ER-FY007193M8 | gsasuagaUfgGfAfAfuacuuaca sasa | 66 | EVPusGfsuaaGfuauuccaUf cUfaucsasg | 73 |
| N-ER-FY007193M9 | gsasuagaUfgGfAfAfuacuuaca sasa | 66 | EVPusGfsuaaGfuAfUfucca UfcUfaucsasg | 74 |
| N-ER-FY007177 | AAAUCGUAUGCAGAAUA UU | 75 | AAUAUUCUGCAUACG AUUU | 76 |
| N-ER-FY007178 | GUAUGCAGAAUAUUCAA UUUAAA | 77 | UAAAUUGAAUAUUCU GCAUACGA | 78 |
| N-ER-FY007178M2 | gsusaugcAfgAfAfUfauucaauu uasasa | 79 | P1usAfsaauUfgaauauuCfu Gfcauacsgsa | 80 |
| N-ER-FY007178M3 | gsusaugcAfgAfAfUfauucaauu uasasa | 79 | P1usAfsaauUfgAfAfuauuC fuGfcauacsgsa | 81 |
| N-ER-FY007178M4 | gsusaugcAfgAfAfUfauucaauu uasasa | 79 | P1usAfsaauUgaauauuCfuG fcauacsgsa | 82 |
| N-ER-FY007178M5 | gsusaugcAfgAfAfUfauucaauu uasasa | 79 | P1usAfsaauUfGaauauuCfu Gfcauacsgsa | 83 |
| N-ER-FY007178M6 | gsusaugcAfgAfAfUfauucaauu uasasa | 79 | usAfsaauUfgaauauuCfuGfc auacsgsa | 84 |
| N-ER-FY007178M7 | gsusaugcAfgAfAfUfauucaauu uasasa | 79 | usAfsaauUfgAfAfuauuCfu Gfcauacsgsa | 85 |
| N-ER-FY007178M8 | gsusaugcAfgAfAfUfauucaauu uasasa | 79 | EVPusAfsaauUfgaauauuCf uGfcauacsgsa | 86 |
| N-ER-FY007178M9 | gsusaugcAfgAfAfUfauucaauu uasasa | 79 | EVPusAfsaauUfgAfAfuauu CfuGfcauacsgsa | 87 |
| N-ER-FY007179 | GUAUGCAGAAUAUUCAA UU | 88 | AAUUGAAUAUUCUGC AUAC | 89 |
| N-ER-FY007179M2 | gsusaugcAfgAfAfUfauucaauu sTsT | 90 | P1asAfsuugAfauauucuGfc AfuacsTsT | 91 |
| N-ER-FY007179M3 | gsusaugcAfgAfAfUfauucaauu sTsT | 90 | P1asAfsuugAfaUfAfuucuG fcAfuacsTsT | 92 |
| N-ER-FY007179M4 | gsusaugcAfgAfAfUfauucaauu sTsT | 90 | P1asAfsuugAauauucuGfcA fuacsTsT | 93 |
| N-ER-FY007179M5 | gsusaugcAfgAfAfUfauucaauu sTsT | 90 | P1asAfsuugAfAuauucuGfc AfuacsTsT | 94 |
| N-ER-FY007179M6 | gsusaugcAfgAfAfUfauucaauu sTsT | 90 | asAfsuugAfauauucuGfcAfu acsTsT | 95 |
| N-ER-FY007179M7 | gsusaugcAfgAfAfUfauucaauu sTsT | 90 | asAfsuugAfaUfAfuucuGfc AfuacsTsT | 96 |
| N-ER-FY007179M8 | gsusaugcAfgAfAfUfauucaauu sTsT | 90 | EVPasAfsuugAfauauucuGf cAfuacsTsT | 97 |
| N-ER-FY007179M9 | gsusaugcAfgAfAfUfauucaauu sTsT | 90 | EVPasAfsuugAfaUfAfuucu GfcAfuacsTsT | 98 |
| N-ER-FY007179M10 | gsusaugcAfgAfAfUfauucaauu sTsT | 90 | asAfsuugAfauauucuGfcaua csTsT | 99 |
| N-ER-FY007179M11 | gsusaugcAfgAfAfUfauucaauu sTsT | 90 | asAfsuugAfaUfAfuucuGfc auacsTsT | 100 |
| N-ER-FY007194 | GUAUGCAGAAUAUUCAA UUUG | 101 | AAUUGAAUAUUCUGC AUACGA | 102 |
| N-ER-FY007194M2 | gsusaugcAfgAfAfUfauucaauu susg | 103 | P1asAfsuugAfauauucuGfc Afuacsgsa | 104 |
| N-ER-FY007194M3 | gsusaugcAfgAfAfUfauucaauu susg | 103 | P1asAfsuugAfaUfAfuucuG fcAfuacsgsa | 105 |
| N-ER-FY007194M4 | gsusaugcAfgAfAfUfauucaauu susg | 103 | P1asAfsuugAauauucuGfcA fuacsgsa | 106 |
| N-ER-FY007194M5 | gsusaugcAfgAfAfUfauucaauu susg | 103 | P1asAfsuugAfAuauucuGfc Afuacsgsa | 107 |
| N-ER-FY007194M6 | gsusaugcAfgAfAfUfauucaauu susg | 103 | asAfsuugAfauauucuGfcAfu acsgsa | 108 |
| N-ER-FY007194M7 | gsusaugcAfgAfAfUfauucaauu susg | 103 | asAfsuugAfaUfAfuucuGfc Afuacsgsa | 109 |
| N-ER-FY007194M8 | gsusaugcAfgAfAfUfauucaauu susg | 103 | EVPasAfsuugAfauauucuGf cAfuacsgsa | 110 |
| N-ER-FY007194M9 | gsusaugcAfgAfAfUfauucaauu susg | 103 | EVPasAfsuugAfaUfAfuucu GfcAfuacsgsa | 111 |
| N-ER-FY007180 | GCAGAAUAUUCAAUUUG AA | 112 | UUCAAAUUGAAUAUU CUGC | 113 |
| N-ER-FY007180M2 | gscsagaaUfaUfUfCfaauuugaa sTsT | 114 | P1usUfscaaAfuugaauaUfu CfugcsTsT | 115 |
| N-ER-FY007180M3 | gscsagaaUfaUfUfCfaauuugaa sTsT | 114 | P1usUfscaaAfuUfGfaauaU fuCfugcsTsT | 116 |
| N-ER-FY007180M4 | gscsagaaUfaUfUfCfaauuugaa sTsT | 114 | P1usUfscaaAuugaauaUfuC fugcsTsT | 117 |
| N-ER-FY007180M5 | gscsagaaUfaUfUfCfaauuugaa sTsT | 114 | P1usUfscaaAfUugaauaUfu CfugcsTsT | 118 |
| N-ER-FY007180M6 | gscsagaaUfaUfUfCfaauuugaa sTsT | 114 | usUfscaaAfuugaauaUfuCfu gcsTsT | 119 |
| N-ER-FY007180M7 | gscsagaaUfaUfUfCfaauuugaa sTsT | 114 | usUfscaaAfuUfGfaauaUfu CfugcsTsT | 120 |
| N-ER-FY007180M8 | gscsagaaUfaUfUfCfaauuugaa sTsT | 114 | EVPusUfscaaAfuugaaauaUf uCfugcsTsT | 121 |
| N-ER-FY007180M9 | gscsagaaUfaUfUfCfaauuugaa sTsT | 114 | EVPusUfscaaAfuUfGfaaua UfuCfugcsTsT | 122 |
| N-ER-FY007180M10 | gscsagaaUfaUfUfCfaauuugaa sTsT | 114 | usUfscaaAfuugaauaUfucug csTsT | 123 |
| N-ER-FY007180M11 | gscsagaaUfaUfUfCfaauuugaa sTsT | 114 | usUfscaaAfuUfGfaauaUfuc ugcsTsT | 124 |
| N-ER-FY007195 | GCAGAAUAUUCAAUUUG AAGC | 125 | UUCAAAUUGAAUAUU CUGCAU | 126 |
| N-ER-FY007195M2 | gscsagaaUfaUfUfCfaauuugaa sgsc | 127 | P1usUfscaaAfuugaauaUfu Cfugcsasu | 128 |
| N-ER-FY007195M3 | gscsagaaUfaUfUfCfaauuugaa sgsc | 127 | P1usUfscaaAfuUfGfaauaU fuCfugcsasu | 129 |
| N-ER-FY007195M4 | gscsagaaUfaUfUfCfaauuugaa sgsc | 127 | P1usUfscaaAuugaauaUfuC fugcsasu | 130 |
| N-ER-FY007195M5 | gscsagaaUfaUfUfCfaauuugaa sgsc | 127 | P1usUfscaaAfUugaauaUfu Cfugcsasu | 131 |
| N-ER-FY007195M6 | gscsagaaUfaUfUfCfaauuugaa sgsc | 127 | usUfscaaAfuugaauaUfuCfu gcsasu | 132 |
| N-ER-FY007195M7 | gscsagaaUfaUfUfCfaauuugaa sgsc | 127 | usUfscaaAfuUfGfaauaUfu Cfugcsasu | 133 |
| N-ER-FY007195M8 | gscsagaaUfaUfUfCfaauuugaa sgsc | 127 | EVPusUfscaaAfuugaauaUf uCfugcsasu | 134 |
| N-ER-FY007195M9 | gscsagaaUfaUfUfCfaauuugaa sgsc | 127 | EVPusUfscaaAfuUfGfaaua UfuCfugcsasu | 135 |
| N-ER-FY007181 | GAAUAUUCAAUUUGAAG CA | 136 | UGCUUCAAAUUGAAU AUUC | 137 |
| N-ER-FY007181M2 | gsasauauUfcAfAfUfuugaagca sTsT | 138 | P1usGfscuuCfaaauugaAfu AfuucsTsT | 139 |
| N-ER-FY007180M12 | gscsagaauaUfUfCfaauuugaa | 144 | EVPusUfscaaAfuugaaauaUf uCfugcsTsT | 121 |
| N-ER-FY007180M13 | gscsagaauAfUfUfcaauuugaa | 145 | EVPusUfscaaAfuugaaauaUf uCfugcsTsT | 121 |
| N-ER-FY007180M14 | gscsagaaUfaUfUfCfaauuugaa | 146 | EVPusUfsCfaAfauUfgAfau aUfuCfuGfcsTsT | 147 |
| N-ER-FY007180M15 | gscsagaaUfaUfUfCfaauuugaa | 146 | EVPusUfsCfAfAfaUfugAf auaUfucugcsTsT | 148 |
| N-ER-FY007180M16 | gscagaauAfUfUfCfaauuugaa | 149 | EVPusUfsCfAfAfaUfugAf auaUfucugcsTsT | 148 |
| N-ER-FY007180M17 | gscsagaaUfaUfUfCfaauuugaa | 146 | EVPusUfscAfaAfuugaaUfa UfuCfuGfcsTsT | 150 |
| N-ER-FY007180M18 | gscsagaaUfaUfUfCfaauuugaa | 146 | EVPusUfscaaauugaauaUfu cugcsTsT | 151 |
| N-ER-FY007180M19 | gscsagaaUfaUfudCaauuugaa | 152 | EVPusUfscAfAfAfuUfgAf aUfaUfuCfuGfcsTsT | 153 |
| N-ER-FY007180M21 | gscsagaaUfaUfuCfaauuugaa | 154 | EVPusUfscaaAfuugaaauaUf uCfugcsTsT | 121 |
| N-ER-FY007180M24 | gscsagaaUfaUfuCfaAfuUfuga a | 155 | EVPusUfscaaAfuugaaauaUf uCfugcsTsT | 121 |
| N-ER-FY007180M26 | gscsagaaUfaUfuCfaAfuuugaa | 156 | EVPusUfscaaAfuugaaauaUf uCfugcsTsT | 121 |
| N-ER-FY007180M27 | gscsAfgaaUfAfUfucaauuugaa | 157 | EVPusUfscaaAfuugaaauaUf uCfugcsTsT | 121 |
| N-ER-FY007180M28 | gscsAfgaauaUfuCfaAfuuugaa | 158 | EVPusUfscaaAfuugaaauaUf uCfugcsTsT | 121 |
| N-ER-FY007180M29 | gscsAfgaaUfaUfuCfaauuugaa | 159 | EVPusUfscaaAfuugaaauaUf uCfugcsTsT | 121 |
| N-ER-FY007180M30 | gscsagaaUfauuCfaAfuuugaa | 160 | EVPusUfscaaAfuugaaauaUf uCfugcsTsT | 121 |
| N-ER-FY007180M31 | gscsagaaUfauuCfAfAfuuugaa | 161 | EVPusUfscaaAfuugaaauaUf uCfugcsTsT | 121 |
| N-ER-FY007180M32 | gscsAfgaaUfauuCfaauuUfgaa | 162 | EVPusUfscaaAfuugaaauaUf uCfugcsTsT | 121 |
| N-ER-FY007180M34 | gscsAfgaaUfauuCfaauuUfGfa a | 163 | EVPusUfscaaAfuugaaauaUf uCfugcsTsT | 121 |
| N-ER-FY007180M35 | gscsagaaUfaUfUfCfaauuugaa | 146 | EVPusUfsCfAfAfAUfugAf auaUfucugcsTsT | 164 |
| N-ER-FY007180M37 | gscsagaaUfaUfUfCfaauuugaa | 146 | EVPusUfscaaaUfugAfauaU fucugcsTsT | 165 |
| N-ER-FY007180M40 | gscsagaaUfaUfUfCfaauuugaa | 146 | EVPusUfscaAfaUfugaauaU fucugcsTsT | 166 |
| N-ER-FY007180M44 | gscsAfgaaUfAfUfucaauuugaa | 157 | EVPusUfscaaaUfugAfauaU fucugcsTsT | 165 |
| N-ER-FY007180M45 | gscsAfgaaUfaUfuCfaauuugaa | 159 | EVPusUfscaaaUfugAfauaU fucugcsTsT | 165 |
| N-ER-FY007180M46 | gscsAfgaaUfaUfuCfaauuugaa | 159 | EVPusUfscaAfaUfugaauaU fucugcsTsT | 166 |
| N-ER-FY007180M47 | gscsAfgaaUfaUfucaauuugaa | 167 | EVPusUfscaAfaUfugaauaU fucugcsTsT | 166 |
| N-ER-FY007180M48 | gscsAfgaaUfaUfucaauuugaa | 167 | EVPusUfscaaaUfugAfauaU fucugcsTsT | 165 |
| N-ER-FY007180M49 | gscsAfgaaUfaUfuCfaauuugaa | 159 | EVPusUfscaaaUfugaaUfaU fucugcsTsT | 168 |
| N-ER-FY007180M50 | gscsAfgaaUfaUfuCfaauuugaa | 159 | EVPusUfscaaAfuugaaUfaU fucugcsTsT | 169 |
| N-ER-FY007180M51 | gscsAfgaaUfaUfuCfaauuugaa | 159 | EVPusUfsCfaaAfuugaauaU fucugcsTsT | 170 |
| N-ER-FY007180M52 | gscsAfgaaUfaUfuCfaauuugaa | 159 | EVPusUfscaaAfuUfgaauaU fucugcsTsT | 171 |
| N-ER-FY007180M53 | gscsAfgaaUfaUfuCfaauuugaa | 159 | EVPusUfscadAaTugAfaua UfucugcsTsT | 172 |
| N-ER-FY007180M54 | gscsAfgaaUfaUfuCfaauuugaa | 159 | EVPusUfscadAaUfugaaTa UfucugcsTsT | 173 |
| N-ER-FY007180M55 | gscsAfgaaUfaUfuCfaauuugaa | 159 | EVPusUfscaaaTugaaTaUfu cugcsTsT | 174 |
| N-ER-FY007180M56 | gscsAfgaaUfAfUfucaauuugaa s(invAb) | 175 | EVPusUfscaaaUfugAfauaU fucugcsTsT | 165 |
| N-ER-FY007180M57 | gscsAfgaaUfaUfuCfaauuugaa s(invAb) | 176 | EVPusUfscaaaUfugAfauaU fucugcsTsT | 165 |
| N-ER-FY007180M58 | (invAb)sgscAfgaaUfAfUfuca auuugaas(invAb) | 177 | EVPusUfscaaaUfugAfauaU fucugcsTsT | 165 |
| N-ER-FY007180M59 | gscsagaaUfaUfucaaUfuugaa | 178 | EVPusUfscaaaUfugAfauaU fucugcsTsT | 165 |
| N-ER-FY007180M60 | gscsAfgaaUfAfUfucaauuugaa | 157 | EVPusUfsCfaAfAUfugAfa UfaUfucugcsTsT | 179 |
| N-ER-FY007180M61 | gscsAfgaaUfaUfuCfaauuugaa | 159 | EVPusUfsCfaAfAUfugAfa UfaUfucugcsTsT | 179 |
| N-ER-FY007180M62 | (invAb)sgscAfgaaUfAfUfuca auuugaas(invAb) | 177 | EVPusUfsCfaAfAUfugAfa UfaUfucugcsTsT | 179 |
| N-ER-FY007180M63 | (invAb)sgscAfgaaUfaUfuCfaa uuugaas(invAb) | 180 | EVPusUfsCfaAfAUfugAfa UfaUfucugcsTsT | 179 |
| N-ER-FY007180M64 | gscsagAfaUfAfUfucaauuugaa | 181 | usUfscaaAfuugaauaUfuCfu gcsTsT | 119 |

**Table 1-1**

| siRNA ID | Ss: 5'- 3' | SEQ ID NO | As: 5'-3' | SEQ ID NO |
|---|---|---|---|---|
| siRNA 1 | GGUUCUGUGGGAUA UUAAUAA | 208 | UUAUUAAUAUC CCACAGAACC | 209 |

wherein the uppercase letters "G", "C", "A", "T", and "U" generally represent nucleotides containing guanine, cytosine, adenine, thymine, and uracil as bases, respectively; the lowercase letters a, u, c, and g represent 2'-methoxy-modified nucleotides; Af, Gf, Cf, and Uf represent 2'-fluoro-modified nucleotides; "dG", "dC", and "dA" represent 2'-deoxyribonucleotides; when "T" is at an overhang position, it represents thymine ribonucleotide (T), and when "T" is in a double-stranded region of a motif, it represents that a uracil ribonucleotide (U) at a corresponding position in the motif sequence is replaced with a thymine ribonucleotide (T); (invAb) is an inverted abasic deoxyribose residue; the lowercase letter s represents that a phosphorothioate linkage is present between two nucleotides immediately adjacent to the left and right of the letter s; P1 represents that one nucleotide immediately adjacent to the right of the P1 is a 5'-phosphate nucleotide; EVP represents that one nucleotide immediately adjacent to the right of the EVP is a 5'-(E)-vinylphosphonate nucleotide; ***A***, ***U***, ***C***, and ***G*** (underlined + bold + italic) represent GNA-modified nucleotides.

siRNA conjugates having the following sequences were synthesized by Shanghai Talen-bio Scientific Co.,Ltd:

**Table 2. siRNA conjugates and sequences thereof:**

| siRNA ID | Ss: 5'→3' | SEQ ID NO | As: 5'→3' | SEQ ID NO |
|---|---|---|---|---|
| N-ER-FY007104M2L96 | gsgsuucuGfuGfGfGfau auuaauaaL96 | 182 | P1usUfsauuAfauauccc AfcAfgaaccsasa | 4 |
| N-ER-FY007104M3L96 | gsgsuucuGfuGfGfGfau auuaauaaL96 | 182 | P1usUfsauuAfaUfAfuc ccAfcAfgaaccsasa | 5 |
| N-ER-FY007104M6L96 | gsgsuucuGfuGfGfGfau auuaauaaL96 | 182 | usUfsauuAfauaucccAfc Afgaaccsasa | 8 |
| N-ER-FY007104M7L96 | gsgsuucuGfuGfGfGfau auuaauaaL96 | 182 | usUfsauuAfaUfAfuccc AfcAfgaaccsasa | 9 |
| N-ER-FY007104M8L96 | gsgsuucuGfuGfGfGfau auuaauaaL96 | 182 | EVPusUfsauuAfauaucc cAfcAfgaaccsasa | 10 |
| N-ER-FY007104M9L96 | gsgsuucuGfuGfGfGfau auuaauaaL96 | 182 | EVPusUfsauuAfaUfAf ucccAfcAfgaaccsasa | 11 |
| N-ER-FY007127M2L96 | csasccaaGfgAfUfGfaa gagauuaaL96 | 183 | P1usUfsaauCfucuucau CfcUfuggugscsu | 15 |
| N-ER-FY007127M3L96 | csasccaaGfgAfUfGfaa gagauuaaL96 | 183 | P1usUfsaauCfuCfUfuc auCfcUfuggugscsu | 16 |
| N-ER-FY007127M6L96 | csasccaaGfgAfUfGfaa gagauuaaL96 | 183 | usUfsaauCfucuucauCfc Ufuggugscsu | 19 |
| N-ER-FY007127M7L96 | csasccaaGfgAfUfGfaa gagauuaaL96 | 183 | usUfsaauCfuCfUfucau CfcUfuggugscsu | 20 |
| N-ER-FY007127M8L96 | csasccaaGfgAfUfGfaa gagauuaaL96 | 183 | EVPusUfsaauCfucuuca uCfcUfuggugscsu | 21 |
| N-ER-FY007127M9L96 | csasccaaGfgAfUfGfaa gagauuaaL96 | 183 | EVPusUfsaauCfuCfUf ucauCfcUfuggugscsu | 22 |
| N-ER-FY007159M2L96 | gsusucacCfaAfAfAfau ccaagcaaL96 | 184 | P1usUfsgcuUfggauuuu UfgGfugaacscsc | 30 |
| N-ER-FY007159M3L96 | gsusucacCfaAfAfAfau ccaagcaaL96 | 184 | P1usUfsgcuUfgGfAfuu uuUfgGfugaacscsc | 31 |
| N-ER-FY007159M6L96 | gsusucacCfaAfAfAfau ccaagcaaL96 | 184 | usUfsgcuUfggauuuuUf gGfugaacscsc | 34 |
| N-ER-FY007159M7L96 | gsusucacCfaAfAfAfau ccaagcaaL96 | 184 | usUfsgcuUfgGfAfuuuu UfgGfugaacscsc | 35 |
| N-ER-FY007159M8L96 | gsusucacCfaAfAfAfau ccaagcaaL96 | 184 | EVPusUfsgcuUfggauu uuUfgGfugaacscsc | 36 |
| N-ER-FY007159M9L96 | gsusucacCfaAfAfAfau ccaagcaaL96 | 184 | EVPusUfsgcuUfgGfAf uuuuUfgGfugaacscsc | 37 |
| N-ER-FY007166M2L96 | gsusaagaAfgUfCfUfga uagauaL96 | 185 | P1usAfsucuAfucagacu UfcUfuacsTsT | 43 |
| N-ER-FY007166M3L96 | gsusaagaAfgUfCfUfga uagauaL96 | 185 | P1usAfsucuAfuCfAfga cuUfcUfuacsTsT | 44 |
| N-ER-FY007166M6L96 | gsusaagaAfgUfCfUfga uagauaL96 | 185 | usAfsucuAfucagacuUfc UfuacsTsT | 47 |
| N-ER-FY007166M7L96 | gsusaagaAfgUfCfUfga uagauaL96 | 185 | usAfsucuAfuCfAfgacu UfcUfuacsTsT | 48 |
| N-ER-FY007166M8L96 | gsusaagaAfgUfCfUfga uagauaL96 | 185 | EVPusAfsucuAfucagac uUfcUfuacsTsT | 49 |
| N-ER-FY007166M9L96 | gsusaagaAfgUfCfUfga uagauaL96 | 185 | EVPusAfsucuAfuCfAf gacuUfcUfuacsTsT | 50 |
| N-ER-FY007166M10L96 | gsusaagaAfgUfCfUfga uagauaL96 | 185 | usAfsucuAfucagacuUfc uuacsTsT | 51 |
| N-ER-FY007166M11L96 | gsusaagaAfgUfCfUfga uagauaL96 | 185 | usAfsucuAfuCfAfgacu UfcuuacsTsT | 52 |
| N-ER-FY007168M2L96 | gsasuagaUfgGfAfAfua cuuacaL96 | 186 | P1usGfsuaaGfuauucca UfcUfaucsTsT | 56 |
| N-ER-FY007168M3L96 | gsasuagaUfgGfAfAfua cuuacaL96 | 186 | P1usGfsuaaGfuAfUfuc caUfcUfaucsTsT | 57 |
| N-ER-FY007168M6L96 | gsasuagaUfgGfAfAfua cuuacaL96 | 186 | usGfsuaaGfuauuccaUfc UfaucsTsT | 60 |
| N-ER-FY007168M7L96 | gsasuagaUfgGfAfAfua cuuacaL96 | 186 | usGfsuaaGfuAfUfucca UfcUfaucsTsT | 61 |
| N-ER-FY007168M8L96 | gsasuagaUfgGfAfAfua cuuacaL96 | 186 | EVPusGfsuaaGfuauucc aUfcUfaucsTsT | 62 |
| N-ER-FY007168M9L96 | gsasuagaUfgGfAfAfua cuuacaL96 | 186 | EVPusGfsuaaGfuAfUf uccaUfcUfaucsTsT | 63 |
| N-ER-FY007178M2L96 | gsusaugcAfgAfAfUfau ucaauuuaL96 | 187 | P1usAfsaauUfgaauauu CfuGfcauacsgsa | 80 |
| N-ER-FY007178M3L96 | gsusaugcAfgAfAfUfau ucaauuuaL96 | 187 | P1usAfsaauUfgAfAfua uuCfuGfcauacsgsa | 81 |
| N-ER-FY007178M6L96 | gsusaugcAfgAfAfUfau ucaauuuaL96 | 187 | usAfsaauUfgaauauuCfu Gfcauacsgsa | 84 |
| N-ER-FY007178M7L96 | gsusaugcAfgAfAfUfau ucaauuuaL96 | 187 | usAfsaauUfgAfAfuauu CfuGfcauacsgsa | 85 |
| N-ER-FY007178M8L96 | gsusaugcAfgAfAfUfau ucaauuuaL96 | 187 | EVPusAfsaauUfgaauau uCfuGfcauacsgsa | 86 |
| N-ER-FY007178M9L96 | gsusaugcAfgAfAfUfau ucaauuuaL96 | 187 | EVPusAfsaauUfgAfAf uauuCfuGfcauacsgsa | 87 |
| N-ER-FY007179M2L96 | gsusaugcAfgAfAfUfau ucaauuL96 | 188 | P1asAfsuugAfauauucu GfcAfuacsTsT | 91 |
| N-ER-FY007179M3L96 | gsusaugcAfgAfAfUfau ucaauuL96 | 188 | P1asAfsuugAfaUfAfuu cuGfcAfuacsTsT | 92 |
| N-ER-FY007179M6L96 | gsusaugcAfgAfAfUfau ucaauuL96 | 188 | asAfsuugAfauauucuGf cAfuacsTsT | 95 |
| N-ER-FY007179M7L96 | gsusaugcAfgAfAfUfau ucaauuL96 | 188 | asAfsuugAfaUfAfuucu GfcAfuacsTsT | 96 |
| N-ER-FY007179M8L96 | gsusaugcAfgAfAfUfau ucaauuL96 | 188 | EVPasAfsuugAfauauuc uGfcAfuacsTsT | 97 |
| N-ER-FY007179M9L96 | gsusaugcAfgAfAfUfau ucaauuL96 | 188 | EVPasAfsuugAfaUfAf uucuGfcAfuacsTsT | 98 |
| N-ER-FY007179M10L96 | gsusaugcAfgAfAfUfau ucaauuL96 | 188 | asAfsuugAfauauucuGf cauacsTsT | 99 |
| N-ER-FY007179M11L96 | gsusaugcAfgAfAfUfau ucaauuL96 | 188 | asAfsuugAfaUfAfuucu GfcauacsTsT | 100 |
| N-ER-FY007180M2L96 | gscsagaaUfaUfUfCfaa uuugaaL96 | 189 | P1usUfscaaAfuugaaua UfuCfugcsTsT | 115 |
| N-ER-FY007180M3L96 | gscsagaaUfaUfUfCfaa uuugaaL96 | 189 | P1usUfscaaAfuUfGfaa uaUfuCfugcsTsT | 116 |
| N-ER-FY007180M6L96 | gscsagaaUfaUfUfCfaa uuugaaL96 | 189 | usUfscaaAfuugaauaUfu CfugcsTsT | 119 |
| N-ER-FY007180M7L96 | gscsagaaUfaUfUfCfaa uuugaaL96 | 189 | usUfscaaAfuUfGfaaua UfuCfugcsTsT | 120 |
| N-ER-FY007180M8L96 | gscsagaaUfaUfUfCfaa uuugaaL96 | 189 | EVPusUfscaaAfuugaau aUfuCfugcsTsT | 121 |
| N-ER-FY007180M9L96 | gscsagaaUfaUfUfCfaa uuugaaL96 | 189 | EVPusUfscaaAfuUfGf aauaUfuCfugcsTsT | 122 |
| N-ER-FY007180M10L96 | gscsagaaUfaUfUfCfaa uuugaaL96 | 189 | usUfscaaAfuugaauaUfu cugcsTsT | 123 |
| N-ER-FY007180M11L96 | gscsagaaUfaUfUfCfaa uuugaaL96 | 189 | usUfscaaAfuUfGfaaua UfucugcsTsT | 124 |
| N-ER-FY007193M6L96 | gsasuagaUfgGfAfAfua cuuacaL96 | 186 | usGfsuaaGfuauuccaUfc Ufaucsasg | 71 |
| N-ER-FY007193M7L96 | gsasuagaUfgGfAfAfua cuuacaL96 | 186 | usGfsuaaGfuAfUfucca UfcUfaucsasg | 72 |
| N-ER-FY007193M8L96 | gsasuagaUfgGfAfAfua cuuacaL96 | 186 | EVPusGfsuaaGfuauucc aUfcUfaucsasg | 73 |
| N-ER-FY007193M9L96 | gsasuagaUfgGfAfAfua cuuacaL96 | 186 | EVPusGfsuaaGfuAfUf uccaUfcUfaucsasg | 74 |
| N-ER-FY007194M6L96 | gsusaugcAfgAfAfUfau ucaauuL96 | 188 | asAfsuugAfauauucuGf cAfuacsgsa | 108 |
| N-ER-FY007194M7L96 | gsusaugcAfgAfAfUfau ucaauuL96 | 188 | asAfsuugAfaUfAfuucu GfcAfuacsgsa | 109 |
| N-ER-FY007194M8L96 | gsusaugcAfgAfAfUfau ucaauuL96 | 188 | EVPasAfsuugAfauauuc uGfcAfuacsgsa | 110 |
| N-ER-FY007194M9L96 | gsusaugcAfgAfAfUfau ucaauuL96 | 188 | EVPasAfsuugAfaUfAf uucuGfcAfuacsgsa | 111 |
| N-ER-FY007195M6L96 | gscsagaaUfaUfUfCfaa uuugaaL96 | 189 | usUfscaaAfuugaauaUfu Cfugcsasu | 132 |
| N-ER-FY007195M7L96 | gscsagaaUfaUfUfCfaa uuugaaL96 | 189 | usUfscaaAfuUfGfaaua UfuCfugcsasu | 133 |
| N-ER-FY007195M8L96 | gscsagaaUfaUfUfCfaa uuugaaL96 | 189 | EVPusUfscaaAfuugaau aUfuCfugcsasu | 134 |
| N-ER-FY007195M9L96 | gscsagaaUfaUfUfCfaa uuugaaL96 | 189 | EVPusUfscaaAfuUfGf aauaUfuCfugcsasu | 135 |
| N-ER-FY007180M13L96 | gscsagaauAfUfUfcaau uugaaL96 | 190 | EVPusUfscaaAfuugaau aUfuCfugcsTsT | 121 |
| N-ER-FY007180M15L96 | gscsagaaUfaUfUfCfaa uuugaaL96 | 189 | EVPusUfsCfAfAfaUfu gAfauaUfucugcsTsT | 148 |
| N-ER-FY007180M21L96 | gscsagaaUfaUfuCfaauu ugaaL96 | 191 | EVPusUfscaaAfuugaau aUfuCfugcsTsT | 121 |
| N-ER-FY007180M24L96 | gscsagaaUfaUfuCfaAf uUfugaaL96 | 192 | EVPusUfscaaAfuugaau aUfuCfugcsTsT | 121 |
| N-ER-FY007180M26L96 | gscsagaaUfaUfuCfaAf uuugaaL96 | 193 | EVPusUfscaaAfuugaau aUfuCfugcsTsT | 121 |
| N-ER-FY007180M27L96 | gscsAfgaaUfAfUfucaa uuugaaL96 | 194 | EVPusUfscaaAfuugaau aUfuCfugcsTsT | 121 |
| N-ER-FY007180M29L96 | gscsAfgaaUfaUfuCfaa uuugaaL96 | 195 | EVPusUfscaaAfuugaau aUfuCfugcsTsT | 121 |
| N-ER-FY007180M31L96 | gscsagaaUfauuCfAfAf uuugaaL96 | 196 | EVPusUfscaaAfuugaau aUfuCfugcsTsT | 121 |
| N-ER-FY007180M32L96 | gscsAfgaaUfauuCfaau uUfgaaL96 | 197 | EVPusUfscaaAfuugaau aUfuCfugcsTsT | 121 |
| N-ER-FY007180M34L96 | gscsAfgaaUfauuCfaau uUfGfaaL96 | 198 | EVPusUfscaaAfuugaau aUfuCfugcsTsT | 121 |
| N-ER-FY007180M35L96 | gscsagaaUfaUfUfCfaa uuugaaL96 | 189 | EVPusUfsCfAfAfAUfu gAfauaUfucugcsTsT | 164 |
| N-ER-FY007180M37L96 | gscsagaaUfaUfUfCfaa uuugaaL96 | 189 | EVPusUfscaaaUfugAfa uaUfucugcsTsT | 165 |
| N-ER-FY007180M40L96 | gscsagaaUfaUfUfCfaa uuugaaL96 | 189 | EVPusUfscaAfaUfugaa uaUfucugcsTsT | 166 |
| N-ER-FY007180M44L96 | gscsAfgaaUfAfUfucaa uuugaaL96 | 194 | EVPusUfscaaaUfugAfa uaUfucugcsTsT | 165 |
| N-ER-FY007180M45L96 | gscsAfgaaUfaUfuCfaa uuugaaL96 | 195 | EVPusUfscaaaUfugAfa uaUfucugcsTsT | 165 |
| N-ER-FY007180M46L96 | gscsAfgaaUfaUfuCfaa uuugaaL96 | 195 | EVPusUfscaAfaUfugaa uaUfucugcsTsT | 166 |
| N-ER-FY007180M47L96 | gscsAfgaaUfaUfucaau uugaaL96 | 199 | EVPusUfscaAfaUfugaa uaUfucugcsTsT | 166 |
| N-ER-FY007180M48L96 | gscsAfgaaUfaUfucaau uugaaL96 | 199 | EVPusUfscaaaUfugAfa uaUfucugcsTsT | 165 |
| N-ER-FY007180M49L96 | gscsAfgaaUfaUfuCfaa uuugaaL96 | 195 | EVPusUfscaaaUfugaa UfaUfucugcsTsT | 168 |
| N-ER-FY007180M50L96 | gscsAfgaaUfaUfuCfaa uuugaaL96 | 195 | EVPusUfscaaAfuugaa UfaUfucugcsTsT | 169 |
| N-ER-FY007180M51L96 | gscsAfgaaUfaUfuCfaa uuugaaL96 | 195 | EVPusUfsCfaaAfuugaa uaUfucugcsTsT | 170 |
| N-ER-FY007180M52L96 | gscsAfgaaUfaUfuCfaa uuugaaL96 | 195 | EVPusUfscaaAfuUfgaa uaUfucugcsTsT | 171 |
| N-ER-FY007180M53L96 | gscsAfgaaUfaUfuCfaa uuugaaL96 | 195 | EVPusUfscadAaTugAf auaUfucugcsTsT | 172 |
| N-ER-FY007180M54L96 | gscsAfgaaUfaUfuCfaa uuugaaL96 | 195 | EVPusUfscadAaUfuga aTaUfucugcsTsT | 173 |
| N-ER-FY007180M55L96 | gscsAfgaaUfaUfuCfaa uuugaaL96 | 195 | EVPusUfscaaaTugaaTa UfucugcsTsT | 174 |
| N-ER-FY007180M56L96 | gscsAfgaaUfAfUfucaa uuugaas(invAb)L96 | 200 | EVPusUfscaaaUfugAfa uaUfucugcsTsT | 165 |
| N-ER-FY007180M57L96 | gscsAfgaaUfaUfuCfaa uuugaas(invAb)L96 | 201 | EVPusUfscaaaUfugAfa uaUfucugcsTsT | 165 |
| N-ER-FY007180M58L96 | (invAb)sgscAfgaaUfAf Ufucaauuugaas(invAb) L96 | 202 | EVPusUfscaaaUfugAfa uaUfucugcsTsT | 165 |
| N-ER-FY007180M59L96 | gscsagaaUfaUfucaaUfu ugaaL96 | 203 | EVPusUfscaaaUfugAfa uaUfucugcsTsT | 165 |
| N-ER-FY007180M60L96 | gscsAfgaaUfAfUfucaa uuugaaL96 | 194 | EVPusUfsCfaAfAUfug AfaUfaUfucugcsTsT | 179 |
| N-ER-FY007180M61L96 | gscsAfgaaUfaUfuCfaa uuugaaL96 | 195 | EVPusUfsCfaAfAUfug AfaUfaUfucugcsTsT | 179 |
| N-ER-FY007180M62L96 | (invAb)sgscAfgaaUfAf Ufucaauuugaas(invAb) | 202 | EVPusUfsCfaAfAUfug AfaUfaUfucugcsTsT | 179 |
| | L96 | | | |
| N-ER-FY007180M63L96 | (invAb)sgscAfgaaUfa UfuCfaauuugaas(invA b)L96 | 204 | EVPusUfsCfaAfAUfug AfaUfaUfucugcsTsT | 179 |
| N-ER-FY007180M64L96 | gscsagAfaUfAfUfucaa uuugaaL96 | 205 | usUfscaaAfuugaauaUfu CfugcsTsT | 119 |

wherein L96 is linked to the 3' end of a sense strand or the blunt end formed at the 3' end of a sense strand in Table 1 via a phosphodiester bond, and L96 is:

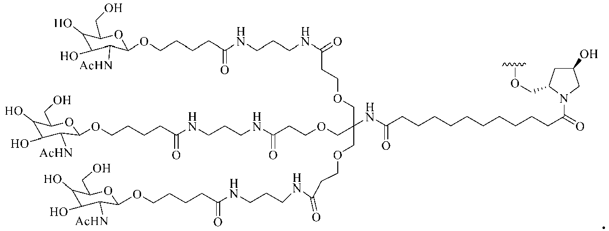

In Table 1 and Table 2, if the 5' end nucleotides of the sense strand, the modified sense strand, and the modified sense strand linked to a conjugated group are not labeled with P1 or EVP on their left sides, it indicates that the 5' end nucleotides are not linked to a 5' phosphate group or a 5' phosphate-derived group (i.e., the ribosyl groups of the 5' end nucleotides are 5' hydroxy groups), and their structures are as shown in formula X: 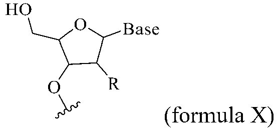 wherein Base represents a base, such as A, U, G, C, or T; R is hydroxy or hydrogen or is substituted with various groups known to those skilled in the art; for example, R may be fluorine, alkoxy, substituted alkoxy, alkyl, substituted alkyl, amino, or substituted amino.

In Table 1 and Table 2, if the 5' end nucleotides of the antisense strand and the modified antisense strand are not labeled with P1 or EVP on their left sides, it indicates that the 5' end nucleotides are not linked to a 5' phosphate group or a 5' phosphate-derived group, and their structures are also as shown in formula X.

In Table 1 and Table 2, the 3' positions of the 3' end nucleotides of the sense strand, the modified sense strand, the antisense strand, and the modified antisense strand are hydroxy groups.

### Example 2. Inhibition of HSD17B 13 Gene Expression by siRNAs and siRNA Conjugates

The Inhibition activity of the siRNAs of the present disclosure against HSD17B13 gene expression was evaluated by a dual-luciferase reporter gene vector.

### Main experimental reagents:

| Reagent | Supplier-No. |
|---|---|
| Lipofectamine^{®} RNAiMAX | Invitrogen-13778-150 |
| Opti-MEM | gibco-31985-070 |
| Fugene-HD transfection reagent | Promega-E231A |
| Dual-Glo luciferase assay system | Promega-E2940 |
| psiCHECK2-HSD17B13 plasmid | GENEWIZ Biotechnology Co., Ltd. |
| Human liver cancer cells: Huh7 cells | JCRB cell bank |

### Experimental procedures:

### Day 0: Transfecting psiCHECK2-HSD17B13 plasmid into Huh7 cells

The psiCHECK2-HSD17B13 plasmid was diluted to 10 ng/µL with Opti-MEM. Huh7 cells were taken, washed with DPBS, and digested with trypsin, and the cell density was adjusted to 1 × 10⁵ cells/mL. Mixing was performed according to a ratio of Fugene-HD transfection reagent:10 ng/µL psiCHECK2-HSD17B13 dilution = 3:100 (volume ratio), and after uniform mixing, incubation was performed at room temperature for 10 min. After the incubation, the mixture was added to the Huh7 cells according to a ratio of 1:9 (volume ratio), and the cells were then inoculated into a 96-well plate at a density of 10,000 cells/well, with 100 µL of culture solution per well. Huh7 cells were cultured overnight in an incubator at 37°C with 5% CO₂.

### Day 1: Treatment with siRNA compounds (siRNAs and siRNA conjugates)

The Lipofectamine^{®} RNAiMAX transfection reagent and Opti-MEM were uniformly mixed at a ratio of 1.5:48.5 (volume ratio) to obtain mixed solution A, and incubation was performed at room temperature for 15 min. Each of the siRNA compounds to be tested (final concentrations were 1 nM, 0.1 nM, and 0.05 nM, respectively) and the mixed solution A were mixed at 1:1, and incubation was performed at room temperature for 15 min. After the incubation, 20 µL of the obtained mixed solution was added to 100 µL of fresh Opti-MEM culture medium at a ratio of 1:5 and uniformly mixed to obtain mixed solution B. The supernatant of the Huh7 cells was discarded, and the mixed solution B described above was added to the 96-well plate at 120 µL/well. The 96-well plate was cultured in a cell incubator with CO₂ for 48 h.

### Day 2: Detecting reporter gene

The cell state was observed under a microscope, and the cell supernatant was discarded. 75 µL of Dual-Glo^{®} & Glo^{®} luciferase reagent and 75 µL of fresh 10% FBS-DMEM culture medium were added to each well, and the plate was shaken on a plate shaker for 10 min in the dark. 100 µL of the sample described above was taken and transferred into a 96-well all-white detection plate, and a luminescence value of firefly luciferase (Firefly lum) was detected with a multi-functional microplate reader. Subsequently, 50 µL of Dual-Glo^{®} Stop & Glo^{®} reagent was added to each well, the plate was shaken on the plate shaker for 10 min in the dark, and a luminescence value of Renilla luciferase (Renilla lum) was detected.

Set in the experiment were: a control group, in which RNA-free H₂O was used instead of the siRNA compounds described above, and the remaining conditions were identical to those of the experimental group; a blank group, Huh7 cells not transfected with the psiCHECK2-HSD17B13 plasmid, to which no siRNA compound was added.

### Data processing:

The ratio of the Renilla luciferase fluorescence value to the firefly luciferase fluorescence value was recorded as: α. The formula is as follows: 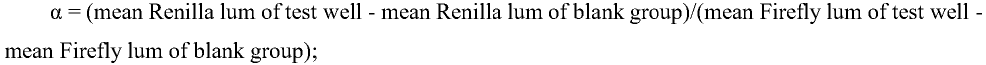 α = (mean Renilla lum of test well - mean Renilla lum of blank group)/(mean Firefly lum of test well - mean Firefly lum of blank group); an experimental group ratio was calculated according to the ratio formula described above and recorded as: α (experimental group), and a control group ratio was recorded as: α (control group).

The inhibition rates of siRNAs against HSD17B13 target gene expression were calculated according to the following formula: 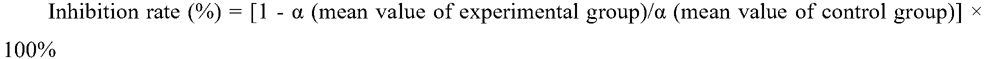 Inhibition rate (%) = [1 - α (mean value of experimental group)/α (mean value of control group)] × 100%

**Table 3. Inhibition rates of siRNAs of the present disclosure**

| siRNA ID | 1 nM 48 h (%) | 0.1 nM 48 h (%) | siRNA ID | 1 nM 48 h (%) | 0.1 nM 48 h (%) |
|---|---|---|---|---|---|
| N-ER-FY007104 | 90.88 | 86.88 | N-ER-FY007168 | 76.43 | 63.82 |
| N-ER-FY007104M2 | 88.20 | 84.86 | N-ER-FY007168M2 | 81.22 | 49.87 |
| N-ER-FY007104M3 | 85.68 | 82.59 | N-ER-FY007168M3 | 82.34 | 50.67 |
| N-ER-FY007104M4 | 79.64 | 68.77 | N-ER-FY007168M4 | 78.52 | 47.46 |
| N-ER-FY007104M5 | 78.35 | 67.49 | N-ER-FY007168M5 | 79.44 | 45.68 |
| N-ER-FY007104M6 | 86.69 | 82.68 | N-ER-FY007168M6 | 80.67 | 50.79 |
| N-ER-FY007104M7 | 89.74 | 81.15 | N-ER-FY007168M7 | 81.49 | 49.82 |
| N-ER-FY007104M8 | 91.56 | 83.79 | N-ER-FY007168M8 | 82.59 | 51.67 |
| N-ER-FY007104M9 | 88.62 | 82.44 | N-ER-FY007168M9 | 83.66 | 52.08 |
| N-ER-FY007127 | 91.91 | 86.24 | N-ER-FY007177 | 73.82 | 68.31 |
| N-ER-FY007127M2 | 87.54 | 83.71 | N-ER-FY007178 | 74.73 | 64.52 |
| N-ER-FY007127M3 | 89.59 | 84.78 | N-ER-FY007178M2 | 77.60 | 59.37 |
| N-ER-FY007127M4 | 70.66 | 62.57 | N-ER-FY007178M3 | 78.49 | 58.49 |
| N-ER-FY007127M5 | 71.62 | 63.12 | N-ER-FY007178M4 | 74.33 | 52.78 |
| N-ER-FY007127M6 | 87.55 | 82.02 | N-ER-FY007178M5 | 73.64 | 51.49 |
| N-ER-FY007127M7 | 84.03 | 81.19 | N-ER-FY007178M6 | 76.46 | 60.13 |
| N-ER-FY007127M8 | 87.64 | 83.89 | N-ER-FY007178M7 | 78.48 | 59.61 |
| N-ER-FY007127M9 | 86.11 | 82.48 | N-ER-FY007178M8 | 79.16 | 60.34 |
| N-ER-FY007128 | 78.75 | 74.99 | N-ER-FY007178M9 | 79.55 | 59.37 |
| N-ER-FY007129 | 79.39 | 74.98 | N-ER-FY007179 | 77.99 | 68.55 |
| N-ER-FY007159 | 67.34 | 56.52 | N-ER-FY007179M2 | 75.58 | 48.27 |
| N-ER-FY007159M2 | 66.35 | -- | N-ER-FY007179M3 | 76.32 | 48.01 |
| N-ER-FY007159M3 | 67.05 | 30.48 | N-ER-FY007179M4 | 73.04 | 43.22 |
| N-ER-FY007159M4 | 63.72 | -- | N-ER-FY007179M5 | 72.06 | 44.18 |
| N-ER-FY007159M5 | 64.33 | -- | N-ER-FY007179M6 | 75.52 | 49.13 |
| N-ER-FY007159M6 | 68.52 | 31.49 | N-ER-FY007179M7 | 76.23 | 49.10 |
| N-ER-FY007159M7 | 68.99 | 32.47 | N-ER-FY007179M8 | 76.59 | 49.89 |
| N-ER-FY007159M8 | 68.89 | 34.79 | N-ER-FY007179M9 | 77.19 | 50.06 |
| N-ER-FY007159M9 | 69.04 | 33.68 | N-ER-FY007180 | 91.86 | 84.05 |
| N-ER-FY007165 | 69.55 | 58.63 | N-ER-FY007180M2 | 88.20 | 81.57 |
| N-ER-FY007180M60 | 86.15 | 78.74 | N-ER-FY007180M3 | 86.37 | 82.37 |
| N-ER-FY007166M2 | 68.41 | 32.38 | N-ER-FY007180M4 | 79.61 | 69.74 |
| N-ER-FY007166M3 | 67.83 | 33.36 | N-ER-FY007180M5 | 78.27 | 66.34 |
| N-ER-FY007166M4 | 65.53 | 30.68 | N-ER-FY007180M6 | 88.67 | 81.76 |
| N-ER-FY007166M5 | 64.89 | -- | N-ER-FY007180M7 | 87.74 | 82.76 |
| N-ER-FY007166M6 | 67.98 | 33.79 | N-ER-FY007180M8 | 89.03 | 84.76 |
| N-ER-FY007166M7 | 68.52 | 34.72 | N-ER-FY007180M9 | 85.75 | 83.74 |
| N-ER-FY007166M8 | 70.54 | 33.58 | N-ER-FY007181 | 72.03 | 51.55 |
| N-ER-FY007166M9 | 69.76 | 34.17 | N-ER-FY007181M2 | 77.06 | 46.64 |
| N-ER-FY007180M12 | 80.22 | 49.69 | N-ER-FY007180M35 | 84.44 | 73.68 |
| N-ER-FY007180M13 | 83.75 | 68.77 | N-ER-FY007180M37 | 85.05 | 76.16 |
| N-ER-FY007180M14 | 83.83 | 64.79 | N-ER-FY007180M40 | 88.46 | 77.09 |
| N-ER-FY007180M15 | 82.13 | 64.39 | N-ER-FY007180M44 | 88.91 | 86.13 |
| N-ER-FY007180M16 | 84.17 | 65.93 | N-ER-FY007180M45 | 83.60 | 75.41 |
| N-ER-FY007180M17 | 82.59 | 65.57 | N-ER-FY007180M46 | 91.95 | 88.05 |
| N-ER-FY007180M18 | 80.98 | 66.53 | N-ER-FY007180M47 | 91.93 | 88.82 |
| N-ER-FY007180M19 | 87.18 | 73.12 | N-ER-FY007180M48 | 89.38 | 89.27 |
| N-ER-FY007180M21 | 85.48 | 74.16 | N-ER-FY007180M49 | 89.73 | 87.72 |
| N-ER-FY007180M24 | 87.27 | 75.06 | N-ER-FY007180M50 | 90.51 | 90.49 |
| N-ER-FY007180M26 | 88.84 | 83.89 | N-ER-FY007180M51 | 85.63 | 75.89 |
| N-ER-FY007180M27 | 86.29 | 81.72 | N-ER-FY007180M52 | 84.20 | 80.41 |
| N-ER-FY007180M28 | 84.73 | 74.00 | N-ER-FY007180M53 | 89.93 | 86.68 |
| N-ER-FY007180M29 | 87.79 | 79.96 | N-ER-FY007180M54 | 89.26 | 88.92 |
| N-ER-FY007180M30 | 87.23 | 74.95 | N-ER-FY007180M55 | 85.05 | 80.72 |
| N-ER-FY007180M31 | 86.85 | 78.94 | N-ER-FY007180M56 | 90.00 | 82.25 |
| N-ER-FY007180M32 | 86.79 | 77.68 | N-ER-FY007180M57 | 85.47 | 82.79 |
| N-ER-FY007180M34 | 84.04 | 74.84 | N-ER-FY007180M61 | 85.23 | 77.47 |
| N-ER-FY007180M58 | 86.54 | 87.11 | N-ER-FY007180M62 | 85.79 | 81.32 |
| N-ER-FY007180M59 | 87.20 | 78.27 | N-ER-FY007180M63 | 85.93 | 78.61 |

**Table 3-1**

| siRNA ID | 1 nM 48 h (%) | 0.1 nM 48 h (%) | siRNA ID | 1 nM 48 h (%) | 0.1 nM 48 h (%) |
|---|---|---|---|---|---|
| N-ER-FY007180M13L96 | 83.28 | 72.04 | N-ER-FY007180M45L96 | 87.36 | 81.95 |
| N-ER-FY007180M15L96 | 77.72 | 79.51 | N-ER-FY007180M46L96 | 84.55 | 67.57 |
| N-ER-FY007180M21L96 | 83.91 | 74.97 | N-ER-FY007180M47L96 | 82.38 | 68.17 |
| N-ER-FY007180M24L96 | 86.02 | 78.41 | N-ER-FY007180M48L96 | 83.50 | 64.71 |
| N-ER-FY007180M26L96 | 84.44 | 77.82 | N-ER-FY007180M49L96 | 82.77 | 67.15 |
| N-ER-FY007180M27L96 | 85.80 | 81.14 | N-ER-FY007180M50L96 | 86.39 | 78.34 |
| N-ER-FY007180M29L96 | 86.31 | 73.62 | N-ER-FY007180M51L96 | 81.03 | 58.83 |
| N-ER-FY007180M31L96 | 83.94 | 71.61 | N-ER-FY007180M52L96 | 82.56 | 60.66 |
| N-ER-FY007180M32L96 | 83.55 | 66.88 | N-ER-FY007180M53L96 | 85.87 | 59.23 |
| N-ER-FY007180M34L96 | 84.37 | 70.04 | N-ER-FY007180M54L96 | 84.47 | 64.48 |
| N-ER-FY007180M35L96 | 82.04 | 72.33 | N-ER-FY007180M55L96 | 80.93 | 72.57 |
| N-ER-FY007180M37L96 | 86.35 | 81.12 | N-ER-FY007180M56L96 | 82.59 | 85.65 |
| N-ER-FY007180M40L96 | 90.06 | 82.95 | N-ER-FY007180M57L96 | 82.58 | 74.10 |
| N-ER-FY007180M44L96 | 86.79 | 84.41 | N-ER-FY007180M61L96 | 88.25 | 80.50 |
| N-ER-FY007180M58L96 | 85.42 | 85.03 | N-ER-FY007180M62L96 | 87.10 | 80.03 |
| N-ER-FY007180M59L96 | 86.76 | 80.93 | N-ER-FY007180M63L96 | 86.83 | 79.46 |
| N-ER-FY007180M60L96 | 86.33 | 81.08 | | | |

**Table 3-2**

| siRNA ID | 1 nM 48 h (%) | 0.1 nM 48 h (%) | 0.05 nM 48 h (%) |
|---|---|---|---|
| N-ER-FY007180 | 89.65 | 83.40 | 78.24 |
| siRNA 2 | 80.92 | 64.69 | 53.94 |

The sequences of siRNA 2 are shown in Table 3-3:

**Table 3-3**

| siRNA ID | Ss: 5'- 3' | SEQ ID NO | As: 5'-3' | SEQ ID NO |
|---|---|---|---|---|
| siRNA 2 | GCAGAAUAUUCAAUUUGA AGU | 210 | ACUUCAAAUUGA AUAUUCUGCAU | 211 |

As can be seen from Table 3, Table 3-1, and Table 3-2, the siRNAs and siRNA conjugates of the present disclosure can significantly inhibit the expression of the HSD17B13 gene at 1 nM, 0.1 nM, and 0.05 nM.

### Example 3. Determination of Inhibition Rates of siRNA Conjugates against HSD17B13 Gene Expression

### Materials:

Primary human hepatocytes: PHH cells, provided by WuXi AppTec (Shanghai) Co., Ltd;
PHH culture medium: invitroGRO CP Meduim serum free BIOVIT, Cat. No.: S03316;
Lipo^{®}RNAiMAX transfection reagent, purchased from Invitrogen, Cat. No.: 13778-150;
RNA extraction kit: RNeasy^{®} 96 Kit, Cat No.: QIAGEN-74182;
Reverse transcription kit: FastKing RT Kit (with gDNase), Cat. No.: TIANGEN-KR116-02;
FastStart Universal Probe Master (Roche-04914058001);
TaqMan Gene Expression Assay (GAPDH, Thermo, Assay ID-Hs02786624_g1);
TaqMan Gene Expression Assay (HSD17B13, Thermo, Assay ID-Hs01068199_m1).

(1) siRNA conjugates (final concentrations of siRNA conjugates were 100 nM and 10 nM, respectively, with replicates) entered PHH cells through free uptake, and the procedures were as described below: Frozen PHH cells were taken, thawed, counted, and adjusted to 6 × 10⁵ cells/mL. Meanwhile, the siRNA conjugates were added, and the cells were seeded into a 96-well plate at a density of 54,000 cells/well, with 100 µL of culture solution added to each well. The cells were cultured in an incubator at 37°C with 5% CO₂. After 48 h, the culture medium was removed, and the cells were collected for total RNA extraction. The total RNA was extracted using the RNeasy^{®} 96 Kit according to the kit product instructions.
(2) Reverse transcription of the extracted total RNA to cDNA was performed using a Fastking RT Kit (With gDNase) according to the following steps:
a) gDNA was removed with a gDNA enzyme according to the table below;

**Table 4**

| | Volume/µL |
|---|---|
| 5× gDNA Buffer | 2 |
| Sample (RNA) | 8 |

The program was run at 42°C for 3 min, and the plate was then placed at 4°C.
b) the following reagents were added to the system obtained in step a), and reverse transcription was performed:

**Table 5**

| | Volume/µL |
|---|---|
| Fastking RT Enzyme Mix | 1 |
| FQ-RT Primer Mix | 2 |
| 10×King RT Buffer | 2 |
| RNase-Free ddH₂O | 5 |

42°C, 15 min; 95°C, 3 min.
c) The reverse transcription product obtained in step b) was stored at -20°C for real-time PCR analysis.
(3) The real-time qPCR analysis was performed:
a) The qPCR reaction mixture was prepared as shown in the table below, and throughout the operation, all reagents were placed on ice;

**Table 6**

| | Volume/µL |
|---|---|
| FastStart Universal Probe Mast | 5 |
| HSD17B13 TaqMan^{®} Gene Expression Assays(60×) | 0.17 |
| cDNA obtained in step (3) | 2 |
| RNase-Free ddH₂O | 2.83 |
| Total volume | 10 |

**Table 7**

| | Volume/µL |
|---|---|
| FastStart Universal Probe Mast | 5 |
| GAPDH gene TaqMan^{®} Gene Expression Assays(60×) | 0.17 |
| cDNA obtained in step (3) | 2 |
| Rnase-Free ddH₂O | 2.83 |
| Total volume | 10 |

b) The qPCR program was performed as described below:
95°C, 10 min;
95°C, 15 s, and 60°C, 1 min (this operation was performed for 40 cycles).

(4) Results analysis:
a) The Ct value was automatically calculated using the Quant Studio 7 software with default settings;
b) The relative expression level of the gene was calculated using the following formulas: $ΔCt = Ct \left(HSD17B13 gene\right) - Ct \left(GAPDH\right) ;$ $ΔΔCt = ΔCt \left(test sample group\right) - ΔCt \left(Mock group\right) ,$ wherein the Mock group represents a group to which no siRNA conjugate was added as compared with the test sample group; mRNA expression relative to Mock group = 2^{-ΔΔCt}; 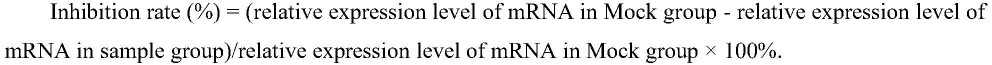 Inhibition rate (%) = (relative expression level of mRNA in Mock group - relative expression level of mRNA in sample group)/relative expression level of mRNA in Mock group × 100%.

**Table 8. Inhibition rates of siRNA conjugates against HSD17B13 gene expression**

| siRNA ID | Entering PHH through free uptake | | siRNA ID | Entering PHH through free uptake | |
|---|---|---|---|---|---|
| | 100 nM (%) | 10 nM (%) | | 100 nM (%) | 10 nM (%) |
| N-ER-FY007104M2L96 | 92.66 | 86.94 | N-ER-FY007168M2L96 | 89.23 | 87.56 |
| N-ER-FY007104M3L96 | 90.75 | 85.50 | N-ER-FY007168M3L96 | 89.62 | 85.39 |
| N-ER-FY007104M6L96 | 91.66 | 86.75 | N-ER-FY007168M6L96 | 70.18 | 68.86 |
| N-ER-FY007104M7L96 | 89.34 | 85.48 | N-ER-FY007168M7L96 | 70.82 | 69.69 |
| N-ER-FY007104M8L96 | 92.67 | 86.14 | N-ER-FY007168M8L96 | 75.16 | 70.36 |
| N-ER-FY007104M9L96 | 87.34 | 85.06 | N-ER-FY007168M9L96 | 74.97 | 71.40 |
| N-ER-FY007127M2L96 | 90.34 | 87.67 | N-ER-FY007178M2L96 | 80.36 | 79.01 |
| N-ER-FY007127M3L96 | 91.47 | 88.68 | N-ER-FY007178M3L96 | 81.46 | 80.12 |
| N-ER-FY007127M6L96 | 93.33 | 86.96 | N-ER-FY007178M6L96 | 79.86 | 77.89 |
| N-ER-FY007127M7L96 | 89.99 | 85.31 | N-ER-FY007178M7L96 | 79.50 | 78.03 |
| N-ER-FY007127M8L96 | 91.16 | 86.64 | N-ER-FY007178M8L96 | 82.75 | 78.67 |
| N-ER-FY007127M9L96 | 89.07 | 82.69 | N-ER-FY007178M9L96 | 81.92 | 78.34 |
| N-ER-FY007159M2L96 | 79.64 | 73.16 | N-ER-FY007179M2L96 | 79.46 | 75.33 |
| N-ER-FY007159M3L96 | 78.55 | 74.31 | N-ER-FY007179M3L96 | 78.97 | 75.34 |
| N-ER-FY007159M6L96 | 80.64 | 74.53 | N-ER-FY007179M6L96 | 75.12 | 72.22 |
| N-ER-FY007159M7L96 | 81.33 | 75.03 | N-ER-FY007179M7L96 | 76.37 | 73.32 |
| N-ER-FY007159M8L96 | 82.37 | 76.90 | N-ER-FY007179M8L96 | 76.47 | 73.33 |
| N-ER-FY007159M9L96 | 81.46 | 75.32 | N-ER-FY007179M9L96 | 77.75 | 74.24 |
| N-ER-FY007166M2L96 | 84.38 | 79.33 | N-ER-FY007180M2L96 | 92.67 | 88.22 |
| N-ER-FY007166M3L96 | 85.03 | 78.60 | N-ER-FY007180M3L96 | 91.43 | 88.70 |
| N-ER-FY007166M6L96 | 88.36 | 85.78 | N-ER-FY007180M6L96 | 88.92 | 85.16 |
| N-ER-FY007166M7L96 | 89.09 | 84.31 | N-ER-FY007180M7L96 | 88.30 | 86.72 |
| N-ER-FY007166M8L96 | 89.38 | 85.34 | N-ER-FY007180M8L96 | 92.79 | 89.01 |
| N-ER-FY007166M9L96 | 90.10 | 85.46 | N-ER-FY007180M9L96 | 90.46 | 89.57 |

As can be seen from Table 8, the siRNA conjugates of the present disclosure exhibited very high HSD17B13 gene inhibition activity. In a case where the siRNA conjugates enter PHH through free uptake, the inhibition rate was not less than about 70% at 100 nM and not less than about 65% at 10 nM.

### Example 4. In Vivo Silencing Effects of siRNA Conjugates in Mice Expressing Human HSD17B13 (hHSD17B13) Gene

### (1) Construction of mouse model overexpressing hHSD17B13 gene by AAV

C57BL/6 mice aged 6-8 weeks (purchased from Beijing Vital River Laboratory Animal Technology Co., Ltd.) entered the facility. After 3-5 days of adaptive feeding, a single tail vein injection of the adeno-associated virus (AAV) carrying the hHSD17B13 gene (pAAV[Exp]-CBh>{HSD17B13 CDS(ns)}:T2A:SEAP:WPRE, the virus was provided by VectorBuilder (Guangzhou) Co., Ltd.) was administered to establish the target gene overexpression model, with the administration volume being 100 µL (2 × 10¹³ vg)/mouse, followed by feeding with common feed.

### (2) Investigation of in vivo silencing efficacy of siRNA conjugates in hHSD17B13 mouse model

14 days after AAV virus injection, the mice were grouped (6 mice per group) and subcutaneously administered a single 3 mg/kg dose of the siRNA conjugate of the present disclosure. The expression level of the SEAP protein (i.e., the expression level of the hHSD17B13 protein) was measured on Day 7, Day 14, Day 21, Day 28, Day 35, and Day 42 after the administration to obtain the inhibition rates of the siRNA conjugates against hHSD17B13.

**Table 9. Inhibition rates of siRNA conjugates against hHSD17B13**

| siRNA ID | Inhibition rate (%) against hHSD17B13 | | | | | |
|---|---|---|---|---|---|---|
| | D7 | D14 | D21 | D28 | D35 | D42 |
| N-ER-FY007104M6L96 | 90.21 | 85.89 | 87.52 | 81.45 | 79.79 | 75.81 |
| N-ER-FY007104M8L96 | 90.15 | 89.29 | 85.90 | 83.34 | 83.20 | 77.44 |
| N-ER-FY007127M6L96 | 89.94 | 84.01 | 86.65 | 80.44 | 80.95 | 74.81 |
| N-ER-FY007159M6L96 | 66.07 | 77.06 | 62.60 | 63.29 | 63.08 | -- |
| N-ER-FY007166M6L96 | 69.03 | 79.59 | 67.99 | 73.07 | -- | -- |
| N-ER-FY007168M2L96 | 55.14 | 63.27 | 56.48 | 50.78 | -- | -- |
| N-ER-FY007178M6L96 | 65.32 | 69.43 | 53.54 | 42.83 | -- | -- |
| N-ER-FY007179M6L96 | 72.15 | 78.48 | 71.03 | 68.90 | 67.67 | -- |
| N-ER-FY007180M6L96 | 91.43 | 87.92 | 89.03 | 81.43 | 79.11 | 68.16 |
| N-ER-FY007180M8L96 | 90.17 | 90.53 | 84.04 | 84.15 | 79.92 | 77.98 |
| N-ER-FY007180M26L96 | 79.75 | 89.34 | 85.74 | 83.15 | 76.87 | -- |
| N-ER-FY007180M35L96 | 72.99 | 81.54 | 73.37 | 64.89 | 65.79 | -- |
| N-ER-FY007180M44L96 | 90.34 | 86.84 | 84.67 | 77.03 | 71.84 | 72.50 |
| N-ER-FY007180M56L96 | 89.14 | 87.67 | 85.86 | 88.16 | 83.95 | 83.41 |
| N-ER-FY007180M58L96 | 91.34 | 87.40 | 85.66 | 87.03 | 83.65 | 82.70 |
| N-ER-FY007180M62L96 | 75.39 | 74.56 | 79.12 | 83.18 | 77.55 | 80.26 |
| ARO-HSD | 49.05 | 66.08 | 70.54 | 62.01 | 63.94 | 62.58 |

| | | | | | | |
|---|---|---|---|---|---|---|
| Note: ARO-HSD (WO2023196941) is a clinical drug developed by Arrowhead Pharmaceuticals, INC., which serves as a positive reference, and a sequence thereof is shown in Table 10. | | | | | | |

**Table 10**

| siRNA ID | Ss: 5'→3' | SEQ ID NO | As: 5'→3' | SEQ ID NO |
|---|---|---|---|---|
| ARO-HSD | (NAG37)s(invAb)scguaagaaGfuC fuGfauagaugas(invAb) | 206 | usCfsasUfcUfaucagAf cUfuCfuUfaCfsg | 207 |

Structural formula of (NAG37)s:

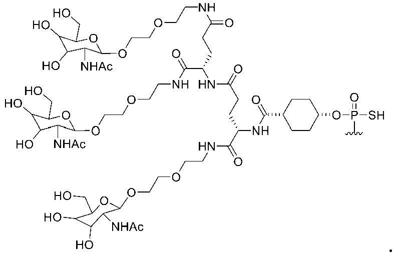

As can be seen from Table 9, the siRNA conjugates of the present disclosure exhibited relatively high inhibition activity against the hHSD17B13 gene *in vivo* and were able to reduce the hHSD17B13 protein level for a long duration.

### Example 5. Repeat-Dose Toxicity Test of N-ER-FY007180M8L96 Administered via Subcutaneous Injection in SD Rats

A total of 4 groups were set in this test. Main test groups each had 15 female and 15 male SD rats (from Guoke Safe (Hebei) Pharmaceutical Technology Co., Ltd.), and the rats were administered via subcutaneous injection with a vehicle control (0.9% sodium chloride injection), or 30 mg/kg, 100 mg/kg, or 300 mg/kg of N-ER-FY007180M8L96, once every two weeks, for a total of 3 times, with an administration volume of 5 mL/kg. On D30, a scheduled necropsy at the end of the administration period was performed on 10 female and 10 male animals in each group, and on D84, a scheduled necropsy was performed on the remaining animals in convalescence. A satellite group was set (the administration dose and administration mode were identical to those of the animals in the main test group), with 5 female and 5 male rats in each group used for blood collection for cytokine study. In addition, a TK group was set (the administration mode and dose were identical to those of the animals in the main test group); a vehicle control group had 4 female and 4 male rats, and test sample groups each had 8 female and 8 male rats used for blood collection for the TK study.

Toxicity indicators of this test included: death/moribundity, general observation, body weight, food consumption, ophthalmic examination, clinical pathology indicators (hematology and coagulation indicators, serum biochemical indicators, immune function indicators, urine indicators, and bone marrow smears), gross necropsy, organ weight, histopathological examination, and tissue distribution. Blood samples of the animals in the satellite group were collected during the acclimation period, and at 4 h and 24 h after the administration on D1 and D29, respectively, for cytokine analysis. Blood samples of the animals in the TK group were collected before the administration and at 0.25 h, 0.5 h, 1 h, 2 h, 4 h, 6 h, 8 h, and 24 h after the administration on D1 and D29, respectively, for toxicokinetic analysis.

During the test, all animals survived to the scheduled necropsy, and no obvious abnormalities related to the test samples were observed in general observation, body weight, food consumption, ophthalmic examination, cytokines, clinical pathology indicators (hematology indicators, coagulation indicators, serum biochemical indicators, immune function indicators, urine indicators, and bone marrow smears), and gross necropsy of all the animals. Therefore, a No Observed Adverse Effect Level (NOAEL) of N-ER-FY007180M8L96 administered via subcutaneous injection in SD rats was 300 mg/kg.

### Example 6. Efficacy Experiment of siRNA Conjugates in Cynomolgus Monkeys with NASH Model

1. 12 male cynomolgus monkeys (body weight > 8 kg) aged 10 years or more with a NASH model (hepatic histopathology scores required to meet steatosis ≥ 2, inflammatory response ≥ 1, ballooning ≥ 1, 2 ≥ hepatic fibrosis score ≥ 1) (provided by Kunming Biomed International Co., Ltd.) were divided into 4 groups, entered the housing facility for feeding, and underwent MRI liver fat scanning at 1 week before administration (D-7).

### 2. Administration observation period

The animals underwent a 12-week administration observation period. The day of administration was defined as Day 0 (D0). The specific administration design is summarized in Table 11.

**Table 11**

| Compound | Administration route | Dose (mg/kg) | Concentration (mg/mL) | Administration volume (mL/kg) | Administration frequency |
|---|---|---|---|---|---|
| Normal saline | | - | - | 0.5 | |
| N-ER-FY007104M8L96 | Subcutaneous administration | 10 | 20 | 0.5 | Once every four weeks, 4 doses in total |
| N-ER-FY007180M8L96 | | 10 | 20 | 0.5 | |
| ARO-HSD | | 10 | 20 | 0.5 | |

The injection site was the lateral scapular/neck area, where the skin was loose. The hair around the injection site was shaved clean, the skin was disinfected with iodine and alcohol, and then subcutaneous administration was performed. On D56 and D112 after administration, liver biopsy was performed for mRNA expression analysis, and a liver mRNA change rate (%) was calculated according to the following formulas: $ΔCt = Ct \left(HSD17B13 gene\right) - Ct \left(GAPDH\right) ;$ $ΔΔCt = ΔCt \left(after administration\right) - ΔCt \left(before administration\right) ;$ mRNA expression relative to pre-administration = 2^{-ΔΔCt}; 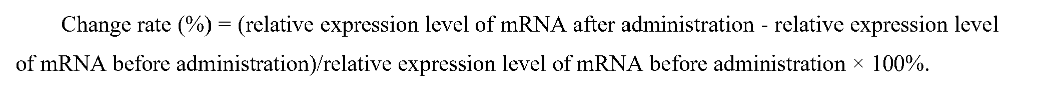 Change rate (%) = (relative expression level of mRNA after administration - relative expression level of mRNA before administration)/relative expression level of mRNA before administration × 100%.

The results are shown in Table 12.

**Table 12**

| | Group | Liver mRNA change rate (%) | |
|---|---|---|---|
| | | D56 | D112 |
| | Normal saline | 41.12 | 59.40 |
| N-ER-FY007104M8L96 | | -83.39 | -85.96 |
| N-ER-FY007180M8L96 | | -92.18 | -95.06 |
| ARO-HSD | | -81.70 | -81.76 |

The siRNA conjugates N-ER-FY007104M8L96 and N-ER-FY007180M8L96 of the present disclosure were able to significantly reduce the expression level of HSD17B13 mRNA in the liver of cynomolgus monkeys *in vivo*, and were superior to ARO-HSD, a drug under clinical development.

### Example 7. In Vitro Stability Experiment of Rat Liver Homogenate

### 1. Experimental reagents and consumables

| Instrument/Reagent name | Brand | Cat. No./Model/CAS No. |
|---|---|---|
| Thermostatic shaking metal bath | DLAB | HCM100-PRO |
| Rat liver | / | / |
| 1 M MgCl₂ | Beyotime | ST269 |
| Ammonium bicarbonate | Tianjin Guangfu Fine Chemical | GB663-78 |
| Sodium dihydrogen phosphate | Tianjin Guangfu Fine Chemical | GB/T1267-1999 |
| Disodium hydrogen phosphate | Tianjin Guangfu Fine Chemical | 10039-32-4 |
| Acetonitrile | Honeywell | AH015-4HC |
| Clarity OTX Lysis-loading Buffer | Agela-Phenomenex | AL0-8579 |
| Methanol | Honeywell | AH230-4HC |
| pH meter | Mettler-Toledo | PE20 |
| Clarity OTX 96-well plate | Agela-Phenomenex | 100mg |
| Solid-phase extraction apparatus (positive pressure) | Agela-Phenomenex | SPE96-196-F0011 |
| Vacuum concentrator | Hunan Kecheng | ZL3-2K |
| 0.5 M EDTA | Beyotime | ST066 |

### 2. Experimental procedures

### 2.1. Preparation of liver homogenate

### 2.1.1. Preparation of grinding solution

**Table 13**

| Reagent name | Preparation method |
|---|---|
| 5 mM EDTA solution | 1 mL of 0.5 M EDTA was taken into a 100 mL volumetric flask, and the volume was adjusted to the scale mark with purified water, followed by shaking uniformly. |
| 100 mM magnesium chloride solution | 1 mL of 1 M magnesium chloride was taken into a 10 mL volumetric flask, and the volume was adjusted to the scale mark with purified water, followed by shaking uniformly. |
| Grinding solution | 1 mL of EDTA solution + 1 mL of magnesium chloride solution + 98 mL of purified water, with pH adjusted to 6.0. |

### 2.1.2. Tissue homogenization

A rat liver tissue and the grinding solution were prepared according to 100 mg:5 mL (a concentration of 20 mg/mL), and grinding beads were then added. The mixture was placed into a homogenizer, and homogenization parameters were set as follows.

**Table 14**

| Running speed | 60 Hz |
|---|---|
| Running time | 30 s |
| Pause time | 15 s |
| Number of runs | 4 times |
| Running temperature | -20°C |

### 2.2. Sample preparation

A siRNA conjugate sample was prepared into a 1 mg/mL solution with enzyme-free water for later use. An internal standard sample was prepared to a concentration of 0.125 mg/mL with enzyme-free water.

### 2.3. Sample incubation

(1) 250 µL of the prepared liver homogenate was added to a 2 mL enzyme-free tube;
(2) 50 µL of a nucleic acid sample was added on the basis of step 1;
(3) A biological sample with a system of 300 µL was vortexed and left to stand for 5 min;
(4) The mixture was divided into 2 tubes, with 100 µL per tube;
(5) System incubation time: 48 h.

### 2.4. Biological sample processing

Each 100 µL of the biological sample system was vortexed and uniformly mixed, and 300 µL of a clarity OTX lysis buffer was added. The mixture was vortexed and left to stand for 30 min, and 100 µL of an internal standard solution was added. The mixture was vortexed, left to stand for 5 min, and centrifuged for 1 min for later use (a total volume of the sample was about 500 µL).

### 2.5. Solid-phase extraction:

### (1) Preparation of solid-phase extraction reagents

Activating agent: 200 mL of methanol was taken into a mobile phase bottle and labeled as activating agent;
Equilibration solution: a 1 M phosphate buffer solution [877 mL of sodium dihydrogen phosphate (1.56 g/L) + 123 mL of disodium hydrogen phosphate (3.58 g/L)] was prepared and diluted 100-fold, with pH adjusted to 5.5 using phosphoric acid, and labeled as equilibration solution;
Rinsing solution: 500 mL of the equilibration solution was taken into a 1 L mobile phase bottle, and 500 mL of acetonitrile was added, with pH adjusted to 5.5 using phosphoric acid. The mixture was mixed uniformly and labeled as rinsing solution;
Eluent: 7.9 g of ammonium bicarbonate was weighed into a 1 L mobile phase bottle, and 1 L of water was added. 500 mL of the ammonium bicarbonate solution was taken into a 1 L mobile phase bottle, and 500 mL of acetonitrile was added, with pH adjusted to 9 using sodium hydroxide. The mixture was mixed uniformly and labeled as eluent;

### (2) Extraction steps were as follows:

**Table 15**

| Step | Process |
|---|---|
| Activation | 1 mL of methanol, 10 min |
| Equilibration | 2 × 1 mL of 10 mM phosphate (pH = 5.5), 10 min for the first time, 10 min for the second time |
| Sample loading | 400 µL (4/5 of the total volume) |
| Rinsing | 2 × 1 mL of 10 mM phosphate (pH = 5.5):50% acetonitrile, 20 min for the first time, 20 min for the second time |
| Elution | 2 × 0.75 mL of 100 mM ammonium bicarbonate (pH = 9):50% acetonitrile, 20 min for the first time, 20 min for the second time |

### 2.6. Post-treatment

The eluent was taken (taken in two times, 600 µL for each, 1200 µL in total) and placed in a 2 mL EP tube, followed by vacuum concentration for 6 h at a rotation speed of 1800 rpm; 100 µL of a mobile phase (initial ratio) was added to the concentrated sample for reconstitution, followed by low-speed centrifugation for 2 min, and 10 µL of the supernatant was taken and injected into a high-resolution mass spectrometer. The antisense strand proportion of the siRNA conjugates of the present disclosure was semi-quantitatively detected by the LC-MS/MS method, and the metabolic results after *in vitro* incubation in the rat liver homogenate for 48 h are shown in Table 16 below.

**Table 16. Remaining percentage of antisense strand of siRNA conjugates**

| Sample ID | AS% (remaining rate) |
|---|---|
| N-ER-FY007180M8L96 | 94.11 |
| N-ER-FY007180M11L96 | 73.97 |
| N-ER-FY007180M44L96 | 90.14 |
| N-ER-FY007180M56L96 | 92.26 |
| N-ER-FY007180M58L96 | 90.70 |
| N-ER-FY007180M62L96 | 91.59 |

Wherein a larger remaining amount of AS indicates better stability of a drug and better long-term efficacy of the drug, and the siRNA conjugates of the present disclosure have good stability.

## Claims

1. A siRNA capable of inhibiting HSD17B13 gene expression, comprising a sense strand and an antisense strand, wherein each nucleotide in the siRNA is independently a modified or unmodified nucleotide; the sense strand comprises a nucleotide sequence I, the antisense strand comprises a nucleotide sequence II, and the nucleotide sequence I is at least partially reversely complementary to the nucleotide sequence II to form a double-stranded region, wherein the nucleotide sequence I and the nucleotide sequence II are selected from the following sequences:
(1) the nucleotide sequence I comprising the nucleotide sequence as shown in SEQ ID NO: 25, and the nucleotide sequence II comprising the nucleotide sequence as shown in SEQ ID NO: 26;
(2) the nucleotide sequence I comprising the nucleotide sequence as shown in SEQ ID NO: 40, and the nucleotide sequence II comprising the nucleotide sequence as shown in SEQ ID NO: 41;
(3) the nucleotide sequence I comprising the nucleotide sequence as shown in SEQ ID NO: 53, and the nucleotide sequence II comprising the nucleotide sequence as shown in SEQ ID NO: 54;
(4) the nucleotide sequence I comprising the nucleotide sequence as shown in SEQ ID NO: 140, and the nucleotide sequence II comprising the nucleotide sequence as shown in SEQ ID NO: 141:
5'-GUAUGCAGAAUAUU-3' (SEQ ID NO: 140)
5'-AAUAUUCUGCAUAC-3' (SEQ ID NO: 141);
(5) the nucleotide sequence I comprising the nucleotide sequence as shown in SEQ ID NO: 142, and the nucleotide sequence II comprising the nucleotide sequence as shown in SEQ ID NO: 143:
5'-GAAUAUUCAAUUUGAA-3' (SEQ ID NO: 142)
5'-UUCAAAUUGAAUAUUC-3' (SEQ ID NO: 143);
(6) the nucleotide sequence I comprising the nucleotide sequence as shown in SEQ ID NO: 1, and the nucleotide sequence II comprising the nucleotide sequence as shown in SEQ ID NO: 2; and
(7) the nucleotide sequence I comprising the nucleotide sequence as shown in SEQ ID NO: 27, and the nucleotide sequence II comprising the nucleotide sequence as shown in SEQ ID NO: 28.

2. The siRNA according to claim 1, wherein the nucleotide sequence I is substantially reversely complementary, virtually reversely complementary, or completely reversely complementary to the nucleotide sequence II; the substantially reversely complementary means that there are no more than 3 base mismatches between the two nucleotide sequences; the virtually reversely complementary means that there is no more than 1 base mismatch between the two nucleotide sequences; the completely reversely complementary means that there is no mismatch between the two nucleotide sequences.

3. The siRNA according to claim 1 or 2, wherein the sense strand further comprises a nucleotide sequence III, and the antisense strand further comprises a nucleotide sequence IV, wherein the nucleotide sequence III and the nucleotide sequence IV each independently have a length of 0-7 nucleotides; the nucleotide sequence III is linked to the 5' end of the nucleotide sequence I, the nucleotide sequence IV is linked to the 3' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences; and/or, the nucleotide sequence III is linked to the 3' end of the nucleotide sequence I, the nucleotide sequence IV is linked to the 5' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences.

4. The siRNA according to any one of claims 1-3, comprising a sense strand and an antisense strand, wherein each nucleotide in the siRNA is independently a modified or unmodified nucleotide; the sense strand comprises nucleotide sequences I and III, the antisense strand comprises nucleotide sequences II and IV, and nucleotide sequences I and III are at least partially reversely complementary to nucleotide sequences II and IV to form a double-stranded region, wherein nucleotide sequences I and III and nucleotide sequences II and IV are selected from the following sequences:
(1) the nucleotide sequences I and III comprising or consisting of the nucleotide sequence as shown in SEQ ID NO: 112, and the nucleotide sequences II and IV comprising or consisting of the nucleotide sequence as shown in SEQ ID NO: 113; and
(2) the nucleotide sequences I and III comprising or consisting of the nucleotide sequence as shown in SEQ ID NO: 208, and the nucleotide sequences II and IV comprising or consisting of the nucleotide sequence as shown in SEQ ID NO: 209.

5. The siRNA according to any one of claims 1-4, wherein the sense strand further comprises a nucleotide sequence V, and/or the antisense strand further comprises a nucleotide sequence VI, wherein the nucleotide sequence V and the nucleotide sequence VI each has a length of 0-3 nucleotides; the nucleotide sequence V is linked to the 3' end of the sense strand to form a 3' overhang of the sense strand, and/or the nucleotide sequence VI is linked to the 3' end of the antisense strand to form a 3' overhang of the antisense strand; preferably, the nucleotide sequence V or the nucleotide sequence VI has a length of 2 nucleotides; more preferably, the nucleotide sequence V or the nucleotide sequence VI is two consecutive thymine deoxyribonucleotides or two consecutive uracil ribonucleotides;
or, the nucleotide sequence V is identical to or different from a nucleotide at a corresponding position of the target mRNA, or the nucleotide sequence VI is mismatched or complementary to a nucleotide at a corresponding position of the target mRNA.

6. The siRNA according to any one of claims 1-5, wherein the double-stranded region has a length of 15-30 nucleotide pairs; preferably, the double-stranded region has a length of 17-23 nucleotide pairs; more preferably, the double-stranded region has a length of 19-21 nucleotide pairs.

7. The siRNA according to any one of claims 1-6, wherein the sense strand or the antisense strand has 15-30 nucleotides; preferably, the sense strand or the antisense strand has 19-25 nucleotides; more preferably, the sense strand or the antisense strand has 19-23 nucleotides.

8. The siRNA according to any one of claims 1-7, wherein at least one nucleotide in the sense strand or the antisense strand is a modified nucleotide, and/or at least one phosphoester group is a phosphoester group with a modification group; preferably, the phosphoester group with a modification group is a phosphorothioate group formed by substituting one oxygen atom in a phosphodiester bond of the phosphoester group with a sulfur atom; and/or, the siRNA comprises a sense strand without a 3' overhang nucleotide.

9. The siRNA according to any one of claims 1-8, wherein the 5' end nucleotide of the antisense strand is linked to a 5' phosphate group or a 5' phosphate-derived group, or the 5' end nucleotide of the antisense strand is not linked to a 5' phosphate group or a 5' phosphate-derived group.

10. The siRNA according to any one of claims 1-9, wherein the 3' end nucleotide of the sense strand is linked to an inverted abasic deoxyribose residue, and/or the 5' end nucleotide of the sense strand is linked to an inverted abasic deoxyribose residue.

11. The siRNA according to any one of claims 1-10, wherein the modified nucleotide is selected from a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxyribonucleotide, a 2'-amino-modified nucleotide, a 2'-substituted amino-modified nucleotide, a nucleotide analog, and a combination of any two or more thereof; preferably, the modified nucleotide is selected from a 2'-fluoro-modified nucleotide, a 2'-methoxy-modified nucleotide, a 2'-O-CH₂-CH₂-O-CH₃-modified nucleotide, a 2'-O-CH₂-CH=CH₂-modified nucleotide, a 2'-CH₂-CH₂-CH=CH₂-modified nucleotide, a 2'-deoxyribonucleotide, a nucleotide analog, and a combination of any two or more thereof.

12. The siRNA according to any one of claims 1-11, wherein each nucleotide in the sense strand and the antisense strand is independently a 2'-fluoro-modified nucleotide or a non-fluoro-modified nucleotide;
preferably, in the sense strand, in the 5' to 3' direction, the 2'-fluoro-modified nucleotides are located at positions 7, 9, 10, and 11, and the remaining positions are the non-fluoro-modified nucleotides; or, the 2'-fluoro-modified nucleotides are located at positions 9, 10, and 11, and the remaining positions are the non-fluoro-modified nucleotides; or, the 2'-fluoro-modified nucleotides are located at positions 8, 9, and 10, and the remaining positions are the non-fluoro-modified nucleotides; or, the 2'-fluoro-modified nucleotides are located at positions 8, 9, 10, and 11, and the remaining positions are the non-fluoro-modified nucleotides; or, the 2'-fluoro-modified nucleotides are located at positions 7 and 9, and the remaining positions are the non-fluoro-modified nucleotides; or, the 2'-fluoro-modified nucleotides are located at positions 7, 9, and 11, and the remaining positions are the non-fluoro-modified nucleotides; or, the 2'-fluoro-modified nucleotides are located at positions 7, 9, 11, 13, and 15, and the remaining positions are the non-fluoro-modified nucleotides; or, the 2'-fluoro-modified nucleotides are located at positions 7, 9, 11, and 13, and the remaining positions are the non-fluoro-modified nucleotides; or, the 2'-fluoro-modified nucleotides are located at positions 3, 7, 8, and 9, and the remaining positions are the non-fluoro-modified nucleotides; or, the 2'-fluoro-modified nucleotides are located at positions 3, 9, 11, and 13, and the remaining positions are the non-fluoro-modified nucleotides; or, the 2'-fluoro-modified nucleotides are located at positions 3, 7, 9, and 11, and the remaining positions are the non-fluoro-modified nucleotides; or, the 2'-fluoro-modified nucleotides are located at positions 7, 11, and 13, and the remaining positions are the non-fluoro-modified nucleotides; or, the 2'-fluoro-modified nucleotides are located at positions 7, 11, 12, and 13, and the remaining positions are the non-fluoro-modified nucleotides; or, the 2'-fluoro-modified nucleotides are located at positions 3, 7, 11, and 16, and the remaining positions are the non-fluoro-modified nucleotides; or, the 2'-fluoro-modified nucleotides are located at positions 3, 7, 11, 16, and 17, and the remaining positions are the non-fluoro-modified nucleotides; or, the 2'-fluoro-modified nucleotides are located at positions 3, 7, and 9, and the remaining positions are the non-fluoro-modified nucleotides; or, the 2'-fluoro-modified nucleotides are located at positions 7, 9, and 14, and the remaining positions are the non-fluoro-modified nucleotides; or, the 2'-fluoro-modified nucleotides are located at positions 5, 7, 8, and 9, and the remaining positions are the non-fluoro-modified nucleotides; and/or
in the antisense strand, in the 5' to 3' direction, the 2'-fluoro-modified nucleotides are located at positions 2, 6, 14, and 16, and the remaining positions are the non-fluoro-modified nucleotides; or, the 2'-fluoro-modified nucleotides are located at positions 2, 6, 8, 9, 14, and 16, and the remaining positions are the non-fluoro-modified nucleotides; or, the 2'-fluoro-modified nucleotides are located at positions 2, 14, and 16, and the remaining positions are the non-fluoro-modified nucleotides; or, the 2'-fluoro-modified nucleotides are located at positions 2, 6, and 14, and the remaining positions are the non-fluoro-modified nucleotides; or, the 2'-fluoro-modified nucleotides are located at positions 2, 6, 8, 9, and 14, and the remaining positions are the non-fluoro-modified nucleotides; or, the 2'-fluoro-modified nucleotides are located at positions 2, 3, 5, 8, 10, 14, 16, and 18, and the remaining positions are the non-fluoro-modified nucleotides; or, the 2'-fluoro-modified nucleotides are located at positions 2, 3, 4, 5, 7, 10, and 14, and the remaining positions are the non-fluoro-modified nucleotides; or, the 2'-fluoro-modified nucleotides are located at positions 2, 4, 6, 12, 14, 16, and 18, and the remaining positions are the non-fluoro-modified nucleotides; or, the 2'-fluoro-modified nucleotides are located at positions 2 and 14, and the remaining positions are the non-fluoro-modified nucleotides; or, the 2'-fluoro-modified nucleotides are located at positions 2, 4, 5, 6, 8, 10, 12, 14, 16, and 18, and the remaining positions are the non-fluoro-modified nucleotides; or, the 2'-fluoro-modified nucleotides are located at positions 2, 7, 10, and 14, and the remaining positions are the non-fluoro-modified nucleotides; or, the 2'-fluoro-modified nucleotides are located at positions 2, 5, 7, and 14, and the remaining positions are the non-fluoro-modified nucleotides; or, the 2'-fluoro-modified nucleotides are located at positions 2, 7, 12, and 14, and the remaining positions are the non-fluoro-modified nucleotides; or, the 2'-fluoro-modified nucleotides are located at positions 2, 6, 12, and 14, and the remaining positions are the non-fluoro-modified nucleotides; or, the 2'-fluoro-modified nucleotides are located at positions 2, 3, 6, and 14, and the remaining positions are the non-fluoro-modified nucleotides; or, the 2'-fluoro-modified nucleotides are located at positions 2, 6, 8, and 14, and the remaining positions are the non-fluoro-modified nucleotides; or, the 2'-fluoro-modified nucleotides are located at positions 2, 10, and 14, and the remaining positions are the non-fluoro-modified nucleotides; or, the 2'-fluoro-modified nucleotides are located at positions 2, 7, and 14, and the remaining positions are the non-fluoro-modified nucleotides; or, the 2'-fluoro-modified nucleotides are located at positions 2, 3, 5, 7, 10, 12, and 14, and the remaining positions are the non-fluoro-modified nucleotides.

13. The siRNA according to claim 12, wherein each of the non-fluoro-modified nucleotides is independently selected from one of a nucleotide formed by substituting hydroxy at the 2' position of a ribosyl group of the nucleotide with a non-fluorine group and a nucleotide analog;
the nucleotide analog is selected from one of pseudouracil, isonucleotide, LNA, ENA, cET BNA, UNA, and GNA.

14. The siRNA according to any one of claims 1-13, wherein each nucleotide in the sense strand and the antisense strand is independently a 2'-fluoro-modified nucleotide, a 2'-methoxy-modified nucleotide, a 2'-deoxyribonucleotide, a GNA-modified nucleotide, or a combination of any two or more thereof;
preferably, in the sense strand, in the 5' to 3' direction, the 2'-fluoro-modified nucleotides are located at positions 7, 9, 10, and 11, and the remaining positions are the 2'-methoxy-modified nucleotides; or, the 2'-fluoro-modified nucleotides are located at positions 9, 10, and 11, and the remaining positions are the 2'-methoxy-modified nucleotides; or, the 2'-fluoro-modified nucleotides are located at positions 8, 9, and 10, and the remaining positions are the 2'-methoxy-modified nucleotides; or, the 2'-fluoro-modified nucleotides are located at positions 8, 9, 10, and 11, and the remaining positions are the 2'-methoxy-modified nucleotides; or, the 2'-fluoro-modified nucleotides are located at positions 7 and 9, the 2'-deoxyribonucleotide is located at position 11 of the sense strand, and the remaining positions are the 2'-methoxy-modified nucleotides; or, the 2'-fluoro-modified nucleotides are located at positions 7, 9, and 11, and the remaining positions are the 2'-methoxy-modified nucleotides; or, the 2'-fluoro-modified nucleotides are located at positions 7, 9, 11, 13, and 15, and the remaining positions are the 2'-methoxy-modified nucleotides; or, the 2'-fluoro-modified nucleotides are located at positions 7, 9, 11, and 13, and the remaining positions are the 2'-methoxy-modified nucleotides; or, the 2'-fluoro-modified nucleotides are located at positions 3, 7, 8, and 9, and the remaining positions are the 2'-methoxy-modified nucleotides; or, the 2'-fluoro-modified nucleotides are located at positions 3, 9, 11, and 13, and the remaining positions are the 2'-methoxy-modified nucleotides; or, the 2'-fluoro-modified nucleotides are located at positions 3, 7, 9, and 11, and the remaining positions are the 2'-methoxy-modified nucleotides; or, the 2'-fluoro-modified nucleotides are located at positions 7, 11, and 13, and the remaining positions are the 2'-methoxy-modified nucleotides; or, the 2'-fluoro-modified nucleotides are located at positions 7, 11, 12, and 13, and the remaining positions are the 2'-methoxy-modified nucleotides; or, the 2'-fluoro-modified nucleotides are located at positions 3, 7, 11, and 16, and the remaining positions are the 2'-methoxy-modified nucleotides; or, the 2'-fluoro-modified nucleotides are located at positions 3, 7, 11, 16, and 17, and the remaining positions are the 2'-methoxy-modified nucleotides; or, the 2'-fluoro-modified nucleotides are located at positions 3, 7, and 9, and the remaining positions are the 2'-methoxy-modified nucleotides; or, the 2'-fluoro-modified nucleotides are located at positions 7, 9, and 14, and the remaining positions are the 2'-methoxy-modified nucleotides; or, the 2'-fluoro-modified nucleotides are located at positions 5, 7, 8, and 9, and the remaining positions are the 2'-methoxy-modified nucleotides; and/or
in the antisense strand, in the 5' to 3' direction, the 2'-fluoro-modified nucleotides are located at positions 2, 6, 14, and 16, and the remaining positions are the 2'-methoxy-modified nucleotides; or, the 2'-fluoro-modified nucleotides are located at positions 2, 6, 8, 9, 14, and 16, and the remaining positions are the 2'-methoxy-modified nucleotides; or, the 2'-fluoro-modified nucleotides are located at positions 2, 14, and 16, the GNA-modified nucleotide is located at position 6 of the antisense strand, and the remaining positions are the 2'-methoxy-modified nucleotides; or, the 2'-fluoro-modified nucleotides are located at positions 2, 6, 14, and 16, the GNA-modified nucleotide is located at position 7 of the antisense strand, and the remaining positions are the 2'-methoxy-modified nucleotides; or, the 2'-fluoro-modified nucleotides are located at positions 2, 6, and 14, and the remaining positions are the 2'-methoxy-modified nucleotides; or, the 2'-fluoro-modified nucleotides are located at positions 2, 6, 8, 9, and 14, and the remaining positions are the 2'-methoxy-modified nucleotides; or, the 2'-fluoro-modified nucleotides are located at positions 2, 3, 5, 8, 10, 14, 16, and 18, and the remaining positions are the 2'-methoxy-modified nucleotides; or, the 2'-fluoro-modified nucleotides are located at positions 2, 3, 4, 5, 7, 10, and 14, and the remaining positions are the 2'-methoxy-modified nucleotides; or, the 2'-fluoro-modified nucleotides are located at positions 2, 4, 6, 12, 14, 16, and 18, and the remaining positions are the 2'-methoxy-modified nucleotides; or, the 2'-fluoro-modified nucleotides are located at positions 2 and 14, and the remaining positions are the 2'-methoxy-modified nucleotides; or, the 2'-fluoro-modified nucleotides are located at positions 2, 4, 5, 6, 8, 10, 12, 14, 16, and 18, and the remaining positions are the 2'-methoxy-modified nucleotides; or, the 2'-fluoro-modified nucleotides are located at positions 2, 3, 4, 5, 7, 10, and 14, the GNA-modified nucleotide is located at position 6 of the antisense strand, and the remaining positions are the 2'-methoxy-modified nucleotides; or, the 2'-fluoro-modified nucleotides are located at positions 2, 7, 10, and 14, and the remaining positions are the 2'-methoxy-modified nucleotides; or, the 2'-fluoro-modified nucleotides are located at positions 2, 5, 7, and 14, and the remaining positions are the 2'-methoxy-modified nucleotides; or, the 2'-fluoro-modified nucleotides are located at positions 2, 7, 12, and 14, and the remaining positions are the 2'-methoxy-modified nucleotides; or, the 2'-fluoro-modified nucleotides are located at positions 2, 6, 12, and 14, and the remaining positions are the 2'-methoxy-modified nucleotides; or, the 2'-fluoro-modified nucleotides are located at positions 2, 3, 6, and 14, and the remaining positions are the 2'-methoxy-modified nucleotides; or, the 2'-fluoro-modified nucleotides are located at positions 2, 6, 8, and 14, and the remaining positions are the 2'-methoxy-modified nucleotides; or, the 2'-fluoro-modified nucleotides are located at positions 2, 10, and 14, the nucleotide at position 7 is replaced with a deoxyribonucleotide, the 2'-deoxyribonucleotide is located at position 5 of the antisense strand, and the remaining positions are the 2'-methoxy-modified nucleotides; or, the 2'-fluoro-modified nucleotides are located at positions 2, 7, and 14, the nucleotide at position 12 is replaced with a deoxyribonucleotide, the 2'-deoxyribonucleotide is located at position 5 of the antisense strand, and the remaining positions are the 2'-methoxy-modified nucleotides; or, the 2'-fluoro-modified nucleotides are located at positions 2 and 14, the nucleotides at positions 7 and 12 are replaced with deoxyribonucleotides, and the remaining positions are the 2'-methoxy-modified nucleotides; or, the 2'-fluoro-modified nucleotides are located at positions 2, 3, 5, 7, 10, 12, and 14, the GNA-modified nucleotide is located at position 6 of the antisense strand, and the remaining positions are the 2'-methoxy-modified nucleotides.

15. The siRNA according to any one of claims 1-14, wherein in the direction from the 5' end to the 3' end,
(1) the sense strand comprises phosphorothioate groups located at the positions shown below:
a position between the first nucleotide and the second nucleotide from the 5' end of the sense strand; and
a position between the second nucleotide and the third nucleotide from the 5' end of the sense strand; and
a position between the first nucleotide and the second nucleotide from the 3' end of the sense strand; and
a position between the second nucleotide and the third nucleotide from the 3' end of the sense strand;
or,
(2) the sense strand comprises phosphorothioate groups located at the positions shown below:
a position between the first nucleotide and the second nucleotide from the 5' end of the sense strand; and
a position between the second nucleotide and the third nucleotide from the 5' end of the sense strand;
or,
(3) the sense strand comprises phosphorothioate groups located at the positions shown below:
a position between the first nucleotide and the second nucleotide from the 5' end of the sense strand;
or,
(4) the sense strand comprises phosphorothioate groups located at the positions shown below:
a position between the first nucleotide and the second nucleotide from the 5' end of the sense strand; and
a position between the second nucleotide and the third nucleotide from the 5' end of the sense strand; and
a position between the inverted abasic deoxyribose residue and the first nucleotide from the 3' end of the sense strand;
or,
(5) the sense strand comprises phosphorothioate groups located at the positions shown below:
a position between the inverted abasic deoxyribose residue and the first nucleotide from the 5' end of the sense strand; and
a position between the first nucleotide and the second nucleotide from the 5' end of the sense strand; and
a position between the inverted abasic deoxyribose residue and the first nucleotide from the 3' end of the sense strand.

16. The siRNA according to any one of claims 1-15, wherein in the direction from the 5' end to the 3' end, the antisense strand comprises phosphorothioate groups located at the positions shown below:
a position between the first nucleotide and the second nucleotide from the 5' end of the antisense strand; and
a position between the second nucleotide and the third nucleotide from the 5' end of the antisense strand; and
a position between the first nucleotide and the second nucleotide from the 3' end of the antisense strand; and
a position between the second nucleotide and the third nucleotide from the 3' end of the antisense strand.

17. The siRNA according to any one of claims 1-16, wherein each nucleotide in the sense strand and the antisense strand is independently a 2'-fluoro-modified nucleotide, a 2'-methoxy-modified nucleotide, a 2'-deoxyribonucleotide, a GNA-modified nucleotide, or a combination of any two or more thereof;
preferably, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, and the remaining positions are the 2'-methoxy-modified nucleotides; the 5' end nucleotide of the antisense strand is linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 14, and 16 of the antisense strand, the GNA-modified nucleotide is located at position 6 of the antisense strand, and the remaining positions are the 2'-methoxy-modified nucleotides; the 5' end nucleotide of the antisense strand is linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the GNA-modified nucleotide is located at position 7 of the antisense strand, and the remaining positions are the 2'-methoxy-modified nucleotides; the 5' end nucleotide of the antisense strand is linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is not linked to a 5' phosphate group or a 5' phosphate-derived group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, and the remaining positions are the 2'-methoxy-modified nucleotides; the 5' end nucleotide of the antisense strand is not linked to a 5' phosphate group or a 5' phosphate-derived group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, and 14 of the antisense strand, and the remaining positions are the 2'-methoxy-modified nucleotides; the 5' end nucleotide of the antisense strand is not linked to a 5' phosphate group or a 5' phosphate-derived group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, and 14 of the antisense strand, and the remaining positions are the 2'-methoxy-modified nucleotides; the 5' end nucleotide of the antisense strand is not linked to a 5' phosphate group or a 5' phosphate-derived group;
or, in the 5' to 3' direction, the 2'-fluoro-modified nucleotides are located at positions 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 8, 9, and 10 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 5, 8, 10, 14, 16, and 18 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 8, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 4, 6, 12, 14, 16, and 18 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2 and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7 and 9 of the sense strand, the 2'-deoxyribonucleotide is located at position 11 of the sense strand, and the remaining positions are the 2'-methoxy-modified nucleotides; in the 5' to 3' direction, the 2'-fluoro-modified nucleotides are located at positions 2, 4, 5, 6, 8, 10, 12, 14, 16, and 18 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 11, 13, and 15 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 8, and 9 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 11, 12, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 11, and 16 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 11, 16, and 17 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, position 6 is the GNA-modified nucleotide, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 5, 7, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 8, and 9 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 5, 7, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, and 9 of the sense strand, and the remaining positions are the 2'-methoxy-modified nucleotides; in the 5' to 3' direction, the 2'-fluoro-modified nucleotides are located at positions 2, 5, 7, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, and 9 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 7, 12, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 6, 12, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 3, 6, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 6, 8, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 10, and 14 of the antisense strand, the nucleotide at position 7 is replaced with a thymine nucleotide, the 2'-deoxyribonucleotide is located at position 5 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 7, and 14 of the antisense strand, the nucleotide at position 12 is replaced with a thymine nucleotide, the 2'-deoxyribonucleotide is located at position 5 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2 and 14 of the antisense strand, the nucleotides at positions 7 and 12 are replaced with thymine nucleotides, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 7, 9, and 14 of the sense strand, and the remaining positions are the 2'-methoxy-modified nucleotides; in the 5' to 3' direction, the 2'-fluoro-modified nucleotides are located at positions 2, 7, 10, and 14 of the antisense strand, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 8, and 9 of the sense strand, and the remaining positions are the 2'-methoxy-modified nucleotides; in the 5' to 3' direction, the 2'-fluoro-modified nucleotides are located at positions 2, 3, 5, 7, 10, 12, and 14 of the antisense strand, position 6 is the GNA-modified nucleotide, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 3, 5, 7, 10, 12, and 14 of the antisense strand, position 6 is the GNA-modified nucleotide, and the remaining positions are the 2'-methoxy-modified nucleotides; the 5' end nucleotide of the antisense strand is linked to a 5'-(E)-vinylphosphonate group;
or, in the 5' to 3' direction, the 2'-fluoro-modified nucleotides are located at positions 5, 7, 8, and 9 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides.

18. The siRNA according to any one of claims 1-17, wherein the siRNA is selected from siRNAs in Table 1; preferably, the siRNA is selected from N-ER-FY007180, N-ER-FY007180M8, N-ER-FY007180M44, N-ER-FY007180M56, N-ER-FY007180M58, N-ER-FY007180M62, N-ER-FY007180M2, N-ER-FY007180M3, N-ER-FY007180M4, N-ER-FY007180M5, N-ER-FY007180M6, N-ER-FY007180M7, N-ER-FY007180M9, N-ER-FY007180M10, N-ER-FY007180M11, N-ER-FY007180M12, N-ER-FY007180M13, N-ER-FY007180M14, N-ER-FY007180M15, N-ER-FY007180M16, N-ER-FY007180M17, N-ER-FY007180M18, N-ER-FY007180M19, N-ER-FY007180M21, N-ER-FY007180M24, N-ER-FY007180M26, N-ER-FY007180M27, N-ER-FY007180M28, N-ER-FY007180M29, N-ER-FY007180M30, N-ER-FY007180M31, N-ER-FY007180M32, N-ER-FY007180M34, N-ER-FY007180M35, N-ER-FY007180M37, N-ER-FY007180M40, N-ER-FY007180M45, N-ER-FY007180M46, N-ER-FY007180M47, N-ER-FY007180M48, N-ER-FY007180M49, N-ER-FY007180M50, N-ER-FY007180M51, N-ER-FY007180M52, N-ER-FY007180M53, N-ER-FY007180M54, N-ER-FY007180M55, N-ER-FY007180M57, N-ER-FY007180M59, N-ER-FY007180M60, N-ER-FY007180M61, N-ER-FY007180M63, and N-ER-FY007180M64.

19. A siRNA conjugate or a prodrug thereof, wherein the siRNA conjugate comprises the siRNA according to any one of claims 1-18 and a conjugated group conjugated to the siRNA.

20. The siRNA conjugate or the prodrug thereof according to claim 19, wherein the conjugated group comprises a pharmaceutically acceptable targeting group and a linker, and the siRNA, the linker, and the targeting group are sequentially linked covalently or non-covalently;
preferably, in the siRNA conjugate, the sense strand and the antisense strand of the siRNA are complementary to form a double-stranded region of the siRNA conjugate, a blunt end is formed at the 3' end of the sense strand, and the 3' end of the antisense strand has 1-3 protruding nucleotides extending out of the double-stranded region;
or,
in the siRNA conjugate, the sense strand and the antisense strand of the siRNA are complementary to form a double-stranded region of the siRNA conjugate, a blunt end is formed at the 3' end of the sense strand, and a blunt end is formed at the 3' end of the antisense strand.

21. The siRNA conjugate or the prodrug thereof according to claim 19 or 20, wherein the conjugated group is selected from:

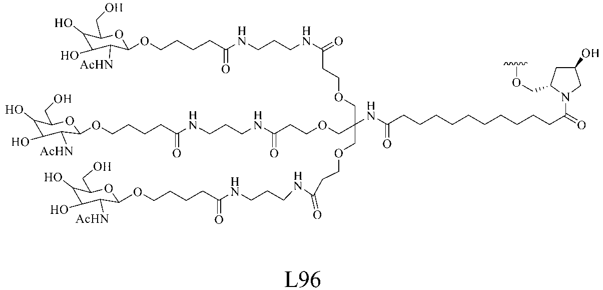

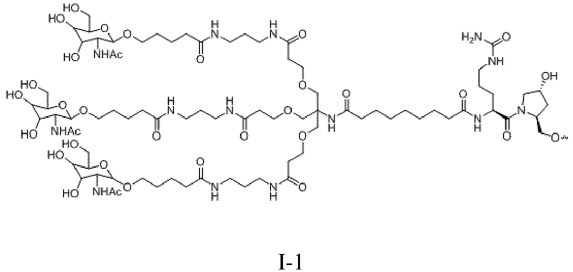

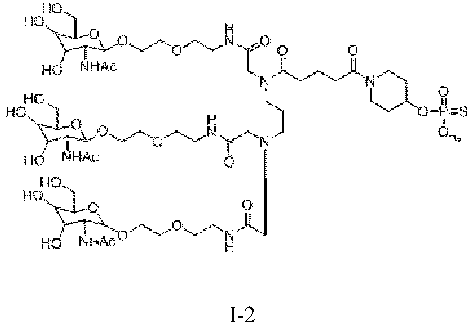

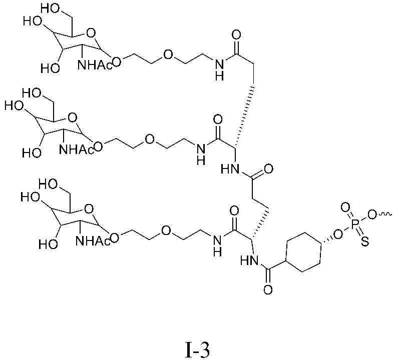

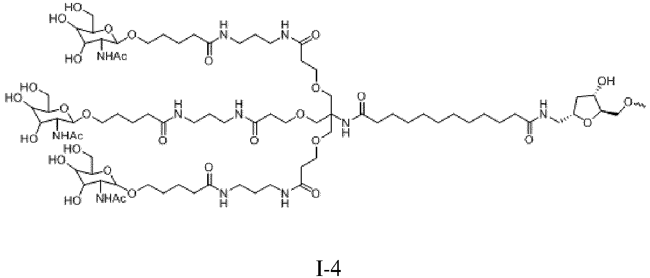

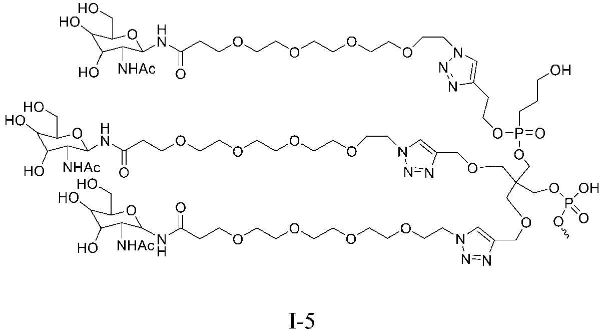

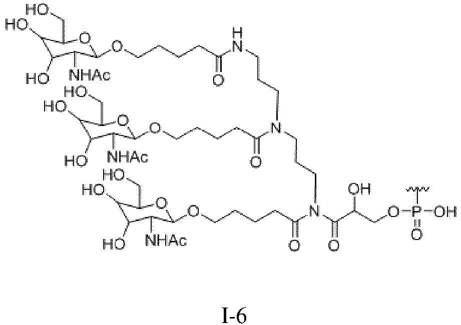

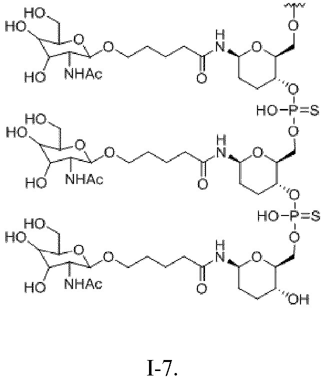

22. The siRNA conjugate or the prodrug thereof according to any one of claims 19-21, wherein the siRNA conjugate is a siRNA conjugate selected from Table 2; preferably, the siRNA conjugate is selected from N-ER-FY007180M8L96, N-ER-FY007180M44L96, N-ER-FY007180M56L96, N-ER-FY007180M58L96, N-ER-FY007180M62L96, N-ER-FY007180M2L96, N-ER-FY007180M3L96, N-ER-FY007180M6L96, N-ER-FY007180M7L96, N-ER-FY007180M9L96, N-ER-FY007180M10L96, N-ER-FY007180M11L96, N-ER-FY007180M13L96, N-ER-FY007180M15L96, N-ER-FY007180M21L96, N-ER-FY007180M24L96, N-ER-FY007180M26L96, N-ER-FY007180M27L96, N-ER-FY007180M29L96, N-ER-FY007180M31L96, N-ER-FY007180M32L96, N-ER-FY007180M34L96, N-ER-FY007180M35L96, N-ER-FY007180M37L96, N-ER-FY007180M40L96, N-ER-FY007180M45L96, N-ER-FY007180M46L96, N-ER-FY007180M47L96, N-ER-FY007180M48L96, N-ER-FY007180M49L96, N-ER-FY007180M50L96, N-ER-FY007180M51L96, N-ER-FY007180M52L96, N-ER-FY007180M53L96, N-ER-FY007180M54L96, N-ER-FY007180M55L96, N-ER-FY007180M57L96, N-ER-FY007180M59L96, N-ER-FY007180M60L96, N-ER-FY007180M61L96, N-ER-FY007180M63L96, and N-ER-FY007180M64L96.

23. A pharmaceutical composition, comprising the siRNA according to any one of claims 1-18, or the siRNA conjugate or the prodrug thereof according to any one of claims 19-22, and a pharmaceutically acceptable carrier.

24. A kit, comprising the siRNA according to any one of claims 1-18, or the siRNA conjugate or the prodrug thereof according to any one of claims 19-22, or the pharmaceutical composition according to claim 21.

25. Use of the siRNA according to any one of claims 1-18, or the siRNA conjugate or the prodrug thereof according to any one of claims 19-22, or the pharmaceutical composition according to claim 23 in preparing a medicament for inhibiting HSD17B13 gene expression.

26. Use of the siRNA according to any one of claims 1-18, or the siRNA conjugate or the prodrug thereof according to any one of claims 19-22, or the pharmaceutical composition according to claim 23 in preparing a medicament for preventing and/or treating a disease associated with HSD17B13 gene overexpression.

27. The use according to claim 26, wherein the disease is selected from non-alcoholic fatty liver disease, non-alcoholic steatohepatitis, cirrhosis, alcoholic hepatitis, hepatic fibrosis, and liver cancer.

28. A method for inhibiting HSD17B13 gene expression, comprising contacting a therapeutically effective amount of the siRNA according to any one of claims 1-18, or the siRNA conjugate or the prodrug thereof according to any one of claims 19-22, or the pharmaceutical composition according to claim 23 with a cell expressing HSD17B13, or administering to a subject in need a therapeutically effective amount of the siRNA according to any one of claims 1-18, or the siRNA conjugate or the prodrug thereof according to any one of claims 19-22, or the pharmaceutical composition according to claim 23.

29. A method for treating and/or preventing a disease associated with HSD17B13 gene overexpression, comprising administering to a subject in need a therapeutically effective amount of the siRNA according to any one of claims 1-18, or the siRNA conjugate or the prodrug thereof according to any one of claims 19-22, or the pharmaceutical composition according to claim 23.

30. The siRNA according to any one of claims 1-18, or the siRNA conjugate or the prodrug thereof according to any one of claims 19-22, or the pharmaceutical composition according to claim 23 for use as a medicament.
